# FASCICULE DE BREVET EUROPEEN

(11) **EP 3 011 019 B1**
(45) Date de publication et mention de la délivrance du brevet: **07.08.2019**
(21) Numéro de dépôt: 14738375.6
(22) Date de dépôt: 17.06.2014
(51) Int. Cl.: C12N 9/10

(54) **POLYPEPTIDE AYANT LA CAPACITE DE FORMER DES BRANCHEMENTS D'UNITES GLUCOSYLE EN ALPHA-1,3 SUR UN ACCEPTEUR**
POLYPEPTID MIT FÄHIGKEIT ZUR BILDUNG VON ALPHA-1,3-GLUCOSYLEINHEITSVERZWEIGUNGEN AUF EINEM AKZEPTOR
POLYPEPTIDE HAVING THE CAPACITY TO FORM ALPHA-1,3 GLUCOSYL UNIT BRANCHINGS ON AN ACCEPTOR

(30) Priorité: 17.06.2013 FR 1301402
(43) Date de publication de la demande: 27.04.2016
(73) Titulaire: Institut National de la Recherche Agronomique (INRA), 75338 Paris Cedex 07 (FR); Institut National des Sciences Appliquées de Toulouse, 31400 Toulouse (FR); Centre National de la Recherche Scientifique (C.N.R.S.), 75016 Paris (FR)
(72) Inventeur: REMAUD-SIMEON, Magali, F-31520 Ramonville (FR); VUILLEMIN, Marlène, F-31400 Toulouse (FR); MOULIS, Claire, F31390 Vieillevigne (FR); MONSAN, Pierre, F-31700 Mondonville (FR); MOREL, Sandrine, F-31320 Auzeville-Tolosane (FR)
(74) Mandataire: Barbot, Willy
(86) Numéro de dépôt international: PCT/EP2014/001644
(87) Numéro de publication internationale: WO 2014/202208

(56) Documents cités:
- HEE-KYOUNG KANG ET AL: "Directed evolution of a dextransucrase for increased constitutive activity and the synthesis of a highly branched dextran", JOURNAL OF MOLECULAR CATALYSIS. B, ENZYMATIC, ELSEVIER, AMSTERDAM, NL, vol. 26, no. 3-6, 1 décembre 2003 (2003-12-01), pages 167-176, XP002720574, ISSN: 1381-1177, DOI: 10.1016/J.MOLCATB.2003.05.006
- KIM H ET AL: "CLONING AND SEQUENCING OF THE ALPHA-1 6 DEXTRANSUCRASE GENE FROM LEUCONOSTOC MENSENTEROIDES B-742CB", JOURNAL OF MICROBIOLOGY AND BIOTECHNOLOGY, KOREAN SOCIETY FOR APPLIED MICROBIOLOGY, SEOUL, KR, vol. 10, no. 4, 1 août 2000 (2000-08-01), pages 559-563, XP008057614, ISSN: 1017-7825
- KANG H K ET AL: "Bioengineering of Leuconostoc mesenteroides Glucansucrases That Gives Selected Bond Formation for Glucan Synthesis and/or Acceptor-Product Synthesis", JOURNAL OF AGRICULTURAL AND FOOD CHEMISTRY, AMERICAN CHEMICAL SOCIETY, US, vol. 59, no. 8, 27 avril 2011 (2011-04-27) , pages 4148-4155, XP002720575, ISSN: 0021-8561, DOI: 10.1021/JF104629G [extrait le 2011-03-10]
- CHUNG C-H ET AL: "GLUCOOLIGOSACCHARIDES FROM LEUCONOSTOC MESENTEROIDES B-742 (ATCC 13146): A POTENTIAL PREBIOTIC", JOURNAL OF INDUSTRIAL MICROBIOLOGY AND BIOTECHNOLOGY, BASINGSTOKE, GB, vol. 29, no. 4, 1 octobre 2002 (2002-10-01), pages 196-199, XP008024426, ISSN: 1367-5435, DOI: 10.1038/SJ.JIM.7000269
- HANS LEEMHUIS ET AL: "Glucansucrases: Three-dimensional structures, reactions, mechanism, [alpha]-glucan analysis and their implications in biotechnology and food applications", JOURNAL OF BIOTECHNOLOGY, vol. 163, no. 2, 1 janvier 2013 (2013-01-01), pages 250-272, XP055102852, ISSN: 0168-1656, DOI: 10.1016/j.jbiotec.2012.06.037

### SEQUENCE LISTING

<110> INRA Magali, Remaud-Simeon Marlène, Vuillemin claire, Moulis Sandrine, Morel Pierre, Monsan
<120> POLYPEPTIDE AYANT LA CAPACITE DE FORMER DES BRANCHEMENTS D'UNITES GLUCOSYLES EN ALPHA-1,3 SUR UN ACCEPTEUR
<130> INR-B-0018-PCT1
<150> FR13/01402
   <151> 2013-06-17
<160> 24
<170> PatentIn version 3.5
<210> 1
   <211> 7
   <212> PRT
   <213> Artificial sequence
<220>
   <223> enzymatic domain
<220>
   <221> MISC_FEATURE
   <222> (3)..(3)
   <223> X= F or Y
<400> 1
   Ala Asp Xaa Val Ala Asn Gln
   1               5
<210> 2
   <211> 11
   <212> PRT
   <213> Artificial Sequence
<220>
   <223> enzymatic domain
<220>
   <221> MISC_FEATURE
   <222> (2)..(2)
   <223> X= M or I
<400> 2
   Ser Xaa Arg Ile Asp Ala Ile Ser Phe Val Asp
   1               5                   10
<210> 3
   <211> 11
   <212> PRT
   <213> Artificial sequence
<220>
   <223> enzymatic domain
<220>
   <221> MISC_FEATURE
   <222> (2)..(2)
   <223> X= I or v
<220>
   <221> MISC_FEATURE
   <222> (8)..(8)
   <223> X= P or S
<220>
   <221> MISC_FEATURE
   <222> (9)..(9)
   <223> X= K or A
<220>
   <221> MISC_FEATURE
   <222> (10)..(10)
   <223> X= G or D
<220>
   <221> MISC_FEATURE
   <222> (11)..(11)
   <223> X= E or Q
<400> 3
   His Xaa Ser Ile Val Glu Ala Xaa Xaa Xaa Xaa
   1               5                   10
<210> 4
   <211> 15
   <212> PRT
   <213> Artificial sequence
<220>
   <223> enzymatic domain
<220>
   <221> MISC_FEATURE
   <222> (12)..(12)
   <223> X= T or A
<220>
   <221> MISC_FEATURE
   <222> (14)..(14)
   <223> X= I or S
<220>
   <221> MISC_FEATURE
   <222> (15)..(15)
   <223> X= H or N
<400> 4
   Ile Val His Ala His Asp Lys Asp Ile Gln Asp Xaa Val Xaa Xaa
   1               5                   10                  15
<210> 5
   <211> 635
   <212> PRT
   <213> Artificial sequence
<220>
   <223> structural domain
<400> 5
   Asn Pro Val Val Gln Ala Glu Gln Leu Asn Trp Leu Tyr Tyr Leu Met
   1 5 10 15
   Asn Phe Gly Gln Ile Thr Ala Asn Asp Ser Asn Ala Asn Phe Asp Ser
   20 25 30
   Met Arg Ile Asp Ala Ile Ser Phe Val Asp Pro Gln Ile Ala Lys Lys
   35 40 45
   Ala Tyr Asp Leu Leu Asp Lys Met Tyr Gly Leu Thr Asp Asn Glu Ala
   50 55 60
   Val Ala Asn Gln His Ile Ser Ile Val Glu Ala Pro Lys Gly Glu Thr
   65 70 75 80
   Pro Ile Thr Val Glu Lys Gln Ser Ala Leu Val Glu Ser Asn Trp Arg
   85 90 95
   Asp Arg Met Lys Gln Ser Leu Ser Lys Asn Ala Thr Leu Asp Lys Leu
   100 105 110
   Asp Pro Asp Pro Ala Ile Asn Ser Leu Glu Lys Leu Val Ala Asp Asp
   115 120 125
   Leu Val Asn Arg Ser Gln Ser Ser Asp Lys Asp Ser Ser Thr Ile Pro
   130 135 140
   Asn Tyr Ser Ile Val His Ala His Asp Lys Asp Ile Gln Asp Thr Val
   145 150 155 160
   Ile His Ile Met Lys Ile Val Asn Asn Asn Pro Asn Ile Ser Met Ser
   165 170 175
   Asp Phe Thr Met Gln Gln Leu Gln Asn Gly Leu Lys Ala Phe Tyr Glu
   180 185 190
   Asp Gln His Gln Ser Val Lys Lys Tyr Asn Gln Tyr Asn Ile Pro Ser
   195 200 205
   Ala Tyr Ala Leu Leu Leu Thr Asn Lys Asp Thr Val Pro Arg Val Phe
   210 215 220
   Tyr Gly Asp Met Tyr Gln Asp Tyr Gly Asp Asp Leu Asp Gly Gly Gln
   225 230 235 240
   Tyr Met Ala Thr Lys Ser Ile Tyr Tyr Asn Ala Ile Glu Gln Met Met
   245 250 255
   Lys Ala Arg Leu Lys Tyr Val Ala Gly Gly Gln Ile Met Ala Val Thr
   260 265 270
   Lys Ile Lys Asn Asp Gly Ile Asn Lys Asp Gly Thr Asn Lys Ser Gly
   275 280 285
   Glu Val Leu Thr Ser Val Arg Phe Gly Lys Asp Ile Met Asp Ala Gln
   290 295 300
   Gly Gln Gly Thr Ala Glu Ser Arg Asn Gln Gly Ile Gly Val Ile Val
   305 310 315 320
   Ser Asn Ser Ser Gly Leu Glu Leu Lys Asn Ser Asp Ser Ile Thr Leu
   325 330 335
   His Met Gly Ile Ala His Lys Asn Gln Ala Tyr Arg Ala Leu Met Leu
   340 345 350
   Thr Asn Asp Lys Gly Ile Val Asn Tyr Asp Gln Asp Asn Asn Ala Pro
   355 360 365
   Ile Ala Trp Thr Asn Asp His Gly Asp Leu Ile Phe Thr Asn Gln Met
   370 375 380
   Ile Asn Gly Gln Ser Asp Thr Ala Val Lys Gly Tyr Leu Asn Pro Glu
   385 390 395 400
   Val Ala Gly Tyr Leu Ala Val Trp Val Pro Val Gly Ala Asn Asp Asn
   405 410 415
   Gln Asp Ala Arg Thr Val Thr Thr Asn Gln Lys Asn Thr Asp Gly Lys
   420 425 430
   Val Leu His Thr Asn Ala Ala Leu Asp Ser Lys Leu Met Tyr Glu Gly
   435 440 445
   Phe Ser Asn Phe Gln Lys Met Pro Thr Arg Gly Asn Gln Tyr Ala Asn
   450 455 460
   Val Val Ile Thr Lys Asn Ile Asp Leu Phe Lys Ser Trp Gly Ile Thr
   465 470 475 480
   Asp Phe Glu Leu Ala Pro Gln Tyr Arg Ser Ser Asp Gly Lys Asp Ile
   485 490 495
   Thr Asp Arg Phe Leu Asp Ser Ile Val Gln Asn Gly Tyr Gly Leu Ser
   500 505 510
   Asp Arg Tyr Asp Leu Gly Phe Lys Thr Pro Thr Lys Tyr Gly Thr Asp
   515 520 525
   Gln Asp Leu Arg Lys Ala Ile Glu Arg Leu His Gln Ala Gly Met Ser
   530 535 540
   Val Met Ala Asp Phe Val Ala Asn Gln Ile Tyr Gly Leu His Ala Asp
   545 550 555 560
   Lys Glu Val Val Ser Ala Gln His Val Asn Ile Asn Gly Asp Thr Lys
   565 570 575
   Leu Val Val Asp Pro Arg Tyr Gly Thr Gln Met Thr Val Val Asn Ser
   580 585 590
   Val Gly Gly Gly Asp Tyr Gln Ala Lys Tyr Gly Gly Glu Tyr Leu Asp
   595 600 605
   Thr Ile Ser Lys Leu Tyr Pro Gly Leu Leu Leu Asp Ser Asn Gly Gln
   610 615 620
   Lys Ile Asp Leu Ser Thr Lys Ile Lys Glu Trp
   625 630 635
<210> 6
   <211> 699
   <212> PRT
   <213> Artificial Sequence
<220>
   <223> structural domain
<400> 6
   Asn Val Asp Ser Glu Tyr Pro Gly Gly Trp Phe Gln Gly Gly Tyr Leu
   1 5 10 15
   Ala Tyr Gln Asn Ser Asp Leu Thr Pro Tyr Ala Asn Thr Asn Pro Asp
   20 25 30
   Tyr Arg Thr His Asn Gly Leu Glu Phe Leu Leu Ala Asn Asp Val Asp
   35 40 45
   Asn Ser Asn Pro Val Val Gln Ala Glu Gln Leu Asn Trp Leu Tyr Tyr
   50 55 60
   Leu Met Asn Phe Gly Gln Ile Thr Ala Asn Asp Ser Asn Ala Asn Phe
   65 70 75 80
   Asp Ser Met Arg Ile Asp Ala Ile Ser Phe Val Asp Pro Gln Ile Ala
   85 90 95
   Lys Lys Ala Tyr Asp Leu Leu Asp Lys Met Tyr Gly Leu Thr Asp Asn
   100 105 110
   Glu Ala Val Ala Asn Gln His Ile Ser Ile Val Glu Ala Pro Lys Gly
   115 120 125
   Glu Thr Pro Ile Thr Val Glu Lys Gln Ser Ala Leu Val Glu Ser Asn
   130 135 140
   Trp Arg Asp Arg Met Lys Gln Ser Leu Ser Lys Asn Ala Thr Leu Asp
   145 150 155 160
   Lys Leu Asp Pro Asp Pro Ala Ile Asn Ser Leu Glu Lys Leu Val Ala
   165 170 175
   Asp Asp Leu Val Asn Arg Ser Gln Ser Ser Asp Lys Asp Ser Ser Thr
   180 185 190
   Ile Pro Asn Tyr Ser Ile Val His Ala His Asp Lys Asp Ile Gln Asp
   195 200 205
   Thr Val Ile His Ile Met Lys Ile Val Asn Asn Asn Pro Asn Ile Ser
   210 215 220
   Met Ser Asp Phe Thr Met Gln Gln Leu Gln Asn Gly Leu Lys Ala Phe
   225 230 235 240
   Tyr Glu Asp Gln His Gln Ser Val Lys Lys Tyr Asn Gln Tyr Asn Ile
   245 250 255
   Pro Ser Ala Tyr Ala Leu Leu Leu Thr Asn Lys Asp Thr Val Pro Arg
   260 265 270
   Val Phe Tyr Gly Asp Met Tyr Gln Asp Tyr Gly Asp Asp Leu Asp Gly
   275 280 285
   Gly Gln Tyr Met Ala Thr Lys Ser Ile Tyr Tyr Asn Ala Ile Glu Gln
   290 295 300
   Met Met Lys Ala Arg Leu Lys Tyr Val Ala Gly Gly Gln Ile Met Ala
   305 310 315 320
   Val Thr Lys Ile Lys Asn Asp Gly Ile Asn Lys Asp Gly Thr Asn Lys
   325 330 335
   Ser Gly Glu Val Leu Thr Ser Val Arg Phe Gly Lys Asp Ile Met Asp
   340 345 350
   Ala Gln Gly Gln Gly Thr Ala Glu Ser Arg Asn Gln Gly Ile Gly Val
   355 360 365
   Ile Val Ser Asn Ser Ser Gly Leu Glu Leu Lys Asn Ser Asp Ser Ile
   370 375 380
   Thr Leu His Met Gly Ile Ala His Lys Asn Gln Ala Tyr Arg Ala Leu
   385 390 395 400
   Met Leu Thr Asn Asp Lys Gly Ile Val Asn Tyr Asp Gln Asp Asn Asn
   405 410 415
   Ala Pro Ile Ala Trp Thr Asn Asp His Gly Asp Leu Ile Phe Thr Asn
   420 425 430
   Gln Met Ile Asn Gly Gln Ser Asp Thr Ala Val Lys Gly Tyr Leu Asn
   435 440 445
   Pro Glu Val Ala Gly Tyr Leu Ala Val Trp Val Pro Val Gly Ala Asn
   450 455 460
   Asp Asn Gln Asp Ala Arg Thr Val Thr Thr Asn Gln Lys Asn Thr Asp
   465 470 475 480
   Gly Lys Val Leu His Thr Asn Ala Ala Leu Asp Ser Lys Leu Met Tyr
   485 490 495
   Glu Gly Phe Ser Asn Phe Gln Lys Met Pro Thr Arg Gly Asn Gln Tyr
   500 505 510
   Ala Asn Val Val Ile Thr Lys Asn Ile Asp Leu Phe Lys Ser Trp Gly
   515 520 525
   Ile Thr Asp Phe Glu Leu Ala Pro Gln Tyr Arg Ser Ser Asp Gly Lys
   530 535 540
   Asp Ile Thr Asp Arg Phe Leu Asp Ser Ile Val Gln Asn Gly Tyr Gly
   545 550 555 560
   Leu Ser Asp Arg Tyr Asp Leu Gly Phe Lys Thr Pro Thr Lys Tyr Gly
   565 570 575
   Thr Asp Gln Asp Leu Arg Lys Ala Ile Glu Arg Leu His Gln Ala Gly
   580 585 590
   Met Ser Val Met Ala Asp Phe Val Ala Asn Gln Ile Tyr Gly Leu His
   595 600 605
   Ala Asp Lys Glu Val Val Ser Ala Gln His Val Asn Ile Asn Gly Asp
   610 615 620
   Thr Lys Leu Val Val Asp Pro Arg Tyr Gly Thr Gln Met Thr Val Val
   625 630 635 640
   Asn Ser Val Gly Gly Gly Asp Tyr Gln Ala Lys Tyr Gly Gly Glu Tyr
   645 650 655
   Leu Asp Thr Ile Ser Lys Leu Tyr Pro Gly Leu Leu Leu Asp Ser Asn
   660 665 670
   Gly Gln Lys Ile Asp Leu Ser Thr Lys Ile Lys Glu Trp Ser Ala Lys
   675 680 685
   Tyr Leu Asn Gly Ser Asn Ile Pro Gln Val Gly
   690 695
<210> 7
   <211> 911
   <212> PRT
   <213> Artificial sequence
<220>
   <223> structural domain
<400> 7
   Val Lys Asp Val Tyr Ser Gln His Asn Ala Val Asn Asn Leu Ser Ala
   1               5                   10                  15
   Asn Asn Phe Lys Asn Leu Val Asp Gly Phe Leu Thr Ala Glu Thr Trp
               20                  25                  30
   Tyr Arg Pro Ala Gln Ile Leu Ser His Gly Thr Asp Trp Val Ala Ser
           35                  40                  45
   Thr Asp Lys Asp Phe Arg Pro Leu Ile Thr Val Trp Trp Pro Asn Lys
       50                  55                  60
   Asp Ile Gln Val Asn Tyr Leu Lys Leu Met Gln Gln Ile Gly Ile Leu
   65                  70                  75                  80
   Asp Asn Ser Val Val Phe Asp Thr Asn Asn Asp Gln Leu Val Leu Asn
                   85                  90                  95
   Lys Gly Ala Glu Ser Ala Gln Ile Gly Ile Glu Lys Lys Val Ser Glu
               100                 105                 110
   Thr Gly Asn Thr Asp Trp Leu Asn Glu Leu Leu Phe Ala Pro Asn Gly
           115                 120                 125
   Asn Gln Pro Ser Phe Ile Lys Gln Gln Tyr Leu Trp Asn Val Asp Ser
       130                 135                 140
   Glu Tyr Pro Gly Gly Trp Phe Gln Gly Gly Tyr Leu Ala Tyr Gln Asn
   145                 150                 155                 160
   Ser Asp Leu Thr Pro Tyr Ala Asn Thr Asn Pro Asp Tyr Arg Thr His
                   165                 170                 175
   Asn Gly Leu Glu Phe Leu Leu Ala Asn Asp Val Asp Asn Ser Asn Pro
               180                 185                 190
   Val Val Gln Ala Glu Gln Leu Asn Trp Leu Tyr Tyr Leu Met Asn Phe
           195                 200                 205
   Gly Gln Ile Thr Ala Asn Asp Ser Asn Ala Asn Phe Asp Ser Met Arg
       210                 215                 220
   Ile Asp Ala Ile Ser Phe Val Asp Pro Gln Ile Ala Lys Lys Ala Tyr
   225                 230                 235                 240
   Asp Leu Leu Asp Lys Met Tyr Gly Leu Thr Asp Asn Glu Ala Val Ala
                   245                 250                 255
   Asn Gln His Ile Ser Ile Val Glu Ala Pro Lys Gly Glu Thr Pro Ile
               260                 265                 270
   Thr Val Glu Lys Gln Ser Ala Leu Val Glu Ser Asn Trp Arg Asp Arg
           275                 280                 285
   Met Lys Gln Ser Leu Ser Lys Asn Ala Thr Leu Asp Lys Leu Asp Pro
       290                 295                 300
   Asp Pro Ala Ile Asn Ser Leu Glu Lys Leu Val Ala Asp Asp Leu Val
   305                 310                 315                 320
   Asn Arg Ser Gln Ser Ser Asp Lys Asp Ser Ser Thr Ile Pro Asn Tyr
                   325                 330                 335
   Ser Ile Val His Ala His Asp Lys Asp Ile Gln Asp Thr Val Ile His
               340                 345                 350
   Ile Met Lys Ile Val Asn Asn Asn Pro Asn Ile Ser Met Ser Asp Phe
           355                 360                 365
   Thr Met Gln Gln Leu Gln Asn Gly Leu Lys Ala Phe Tyr Glu Asp Gln
       370                 375                 380
   His Gln Ser Val Lys Lys Tyr Asn Gln Tyr Asn Ile Pro Ser Ala Tyr
   385                 390                 395                 400
   Ala Leu Leu Leu Thr Asn Lys Asp Thr Val Pro Arg Val Phe Tyr Gly
                   405                 410                 415
   Asp Met Tyr Gln Asp Tyr Gly Asp Asp Leu Asp Gly Gly Gln Tyr Met
               420                 425                 430
   Ala Thr Lys Ser Ile Tyr Tyr Asn Ala Ile Glu Gln Met Met Lys Ala
           435                 440                 445
   Arg Leu Lys Tyr Val Ala Gly Gly Gln Ile Met Ala Val Thr Lys Ile
       450                 455                 460
   Lys Asn Asp Gly Ile Asn Lys Asp Gly Thr Asn Lys Ser Gly Glu Val
   465                 470                 475                 480
   Leu Thr Ser Val Arg Phe Gly Lys Asp Ile Met Asp Ala Gln Gly Gln
                   485                 490                 495
   Gly Thr Ala Glu Ser Arg Asn Gln Gly Ile Gly Val Ile Val Ser Asn
               500                 505                 510
   Ser Ser Gly Leu Glu Leu Lys Asn Ser Asp Ser Ile Thr Leu His Met
           515                 520                 525
   Gly Ile Ala His Lys Asn Gln Ala Tyr Arg Ala Leu Met Leu Thr Asn
       530                 535                 540
   Asp Lys Gly Ile Val Asn Tyr Asp Gln Asp Asn Asn Ala Pro Ile Ala
   545                 550                 555                 560
   Trp Thr Asn Asp His Gly Asp Leu Ile Phe Thr Asn Gln Met Ile Asn
                   565                 570                 575
   Gly Gln Ser Asp Thr Ala Val Lys Gly Tyr Leu Asn Pro Glu Val Ala
               580                 585                 590
   Gly Tyr Leu Ala Val Trp Val Pro Val Gly Ala Asn Asp Asn Gln Asp
           595                 600                 605
   Ala Arg Thr Val Thr Thr Asn Gln Lys Asn Thr Asp Gly Lys Val Leu
       610                 615                 620
   His Thr Asn Ala Ala Leu Asp Ser Lys Leu Met Tyr Glu Gly Phe Ser
   625                 630                 635                 640
   Asn Phe Gln Lys Met Pro Thr Arg Gly Asn Gln Tyr Ala Asn Val Val
                   645                 650                 655
   Ile Thr Lys Asn Ile Asp Leu Phe Lys Ser Trp Gly Ile Thr Asp Phe
               660                 665                 670
   Glu Leu Ala Pro Gln Tyr Arg Ser Ser Asp Gly Lys Asp Ile Thr Asp
           675                 680                 685
   Arg Phe Leu Asp Ser Ile Val Gln Asn Gly Tyr Gly Leu Ser Asp Arg
       690                 695                 700
   Tyr Asp Leu Gly Phe Lys Thr Pro Thr Lys Tyr Gly Thr Asp Gln Asp
   705                 710                 715                 720
   Leu Arg Lys Ala Ile Glu Arg Leu His Gln Ala Gly Met Ser Val Met
                   725                 730                 735
   Ala Asp Phe Val Ala Asn Gln Ile Tyr Gly Leu His Ala Asp Lys Glu
               740                 745                 750
   Val Val Ser Ala Gln His Val Asn Ile Asn Gly Asp Thr Lys Leu Val
           755                 760                 765
   Val Asp Pro Arg Tyr Gly Thr Gln Met Thr Val Val Asn Ser Val Gly
       770                 775                 780
   Gly Gly Asp Tyr Gln Ala Lys Tyr Gly Gly Glu Tyr Leu Asp Thr Ile
   785                 790                 795                 800
   Ser Lys Leu Tyr Pro Gly Leu Leu Leu Asp Ser Asn Gly Gln Lys Ile
                   805                 810                 815
   Asp Leu Ser Thr Lys Ile Lys Glu Trp Ser Ala Lys Tyr Leu Asn Gly
               820                 825                 830
   Ser Asn Ile Pro Gln Val Gly Met Gly Tyr Val Leu Lys Asp Trp Asn
           835                 840                 845
   Asn Gly Gln Tyr Phe His Ile Leu Asp Lys Glu Gly Gln Tyr Ser Leu
       850                 855                 860
   Pro Thr Gln Leu Val Ser Asn Asp Pro Glu Thr Gln Ile Gly Glu Ser
   865                 870                 875                 880
   Val Asn Tyr Lys Tyr Phe Ile Gly Asn Ser Asp Ala Thr Tyr Asn Met
                   885                 890                 895
   Tyr His Asn Leu Pro Asn Thr Val Ser Leu Ile Asn Ser Gln Glu
               900                 905                 910
<210> 8
   <211> 1204
   <212> PRT
   <213> Artificial Sequence
<220>
   <223> structural domain
<400> 8
   Leu Asn Tyr Phe Asp Leu Ala Thr Gly Ile Gln Leu Lys Gly Gln Ala
   1               5                   10                  15
   Lys Asn Ile Asp Gly Ile Gly Tyr Tyr Phe Asp Gln Asn Asn Gly Asn
               20                  25                  30
   Gly Glu Tyr Arg Tyr Ser Leu Thr Gly Pro Val Val Lys Asp Val Tyr
           35                  40                  45
   Ser Gln His Asn Ala Val Asn Asn Leu Ser Ala Asn Asn Phe Lys Asn
   50 55 60
   Leu Val Asp Gly Phe Leu Thr Ala Glu Thr Trp Tyr Arg Pro Ala Gln
   65 70 75 80
   Ile Leu Ser His Gly Thr Asp Trp Val Ala Ser Thr Asp Lys Asp Phe
   85 90 95
   Arg Pro Leu Ile Thr Val Trp Trp Pro Asn Lys Asp Ile Gln Val Asn
   100 105 110
   Tyr Leu Lys Leu Met Gln Gln Ile Gly Ile Leu Asp Asn Ser Val Val
   115 120 125
   Phe Asp Thr Asn Asn Asp Gln Leu Val Leu Asn Lys Gly Ala Glu Ser
   130 135 140
   Ala Gln Ile Gly Ile Glu Lys Lys Val Ser Glu Thr Gly Asn Thr Asp
   145 150 155 160
   Trp Leu Asn Glu Leu Leu Phe Ala Pro Asn Gly Asn Gln Pro Ser Phe
   165 170 175
   Ile Lys Gln Gln Tyr Leu Trp Asn Val Asp Ser Glu Tyr Pro Gly Gly
   180 185 190
   Trp Phe Gln Gly Gly Tyr Leu Ala Tyr Gln Asn Ser Asp Leu Thr Pro
   195 200 205
   Tyr Ala Asn Thr Asn Pro Asp Tyr Arg Thr His Asn Gly Leu Glu Phe
   210 215 220
   Leu Leu Ala Asn Asp Val Asp Asn Ser Asn Pro Val Val Gln Ala Glu
   225 230 235 240
   Gln Leu Asn Trp Leu Tyr Tyr Leu Met Asn Phe Gly Gln Ile Thr Ala
   245 250 255
   Asn Asp Ser Asn Ala Asn Phe Asp Ser Met Arg Ile Asp Ala Ile Ser
   260 265 270
   Phe Val Asp Pro Gln Ile Ala Lys Lys Ala Tyr Asp Leu Leu Asp Lys
   275 280 285
   Met Tyr Gly Leu Thr Asp Asn Glu Ala Val Ala Asn Gln His Ile Ser
   290 295 300
   Ile Val Glu Ala Pro Lys Gly Glu Thr Pro Ile Thr Val Glu Lys Gln
   305 310 315 320
   Ser Ala Leu Val Glu Ser Asn Trp Arg Asp Arg Met Lys Gln Ser Leu
   325 330 335
   Ser Lys Asn Ala Thr Leu Asp Lys Leu Asp Pro Asp Pro Ala Ile Asn
   340 345 350
   Ser Leu Glu Lys Leu Val Ala Asp Asp Leu Val Asn Arg Ser Gln Ser
   355 360 365
   Ser Asp Lys Asp Ser Ser Thr Ile Pro Asn Tyr Ser Ile Val His Ala
   370 375 380
   His Asp Lys Asp Ile Gln Asp Thr Val Ile His Ile Met Lys Ile Val
   385 390 395 400
   Asn Asn Asn Pro Asn Ile Ser Met Ser Asp Phe Thr Met Gln Gln Leu
   405 410 415
   Gln Asn Gly Leu Lys Ala Phe Tyr Glu Asp Gln His Gln Ser Val Lys
   420 425 430
   Lys Tyr Asn Gln Tyr Asn Ile Pro Ser Ala Tyr Ala Leu Leu Leu Thr
   435 440 445
   Asn Lys Asp Thr Val Pro Arg Val Phe Tyr Gly Asp Met Tyr Gln Asp
   450 455 460
   Tyr Gly Asp Asp Leu Asp Gly Gly Gln Tyr Met Ala Thr Lys Ser Ile
   465 470 475 480
   Tyr Tyr Asn Ala Ile Glu Gln Met Met Lys Ala Arg Leu Lys Tyr Val
   485 490 495
   Ala Gly Gly Gln Ile Met Ala Val Thr Lys Ile Lys Asn Asp Gly Ile
   500 505 510
   Asn Lys Asp Gly Thr Asn Lys Ser Gly Glu Val Leu Thr Ser Val Arg
   515 520 525
   Phe Gly Lys Asp Ile Met Asp Ala Gln Gly Gln Gly Thr Ala Glu Ser
   530 535 540
   Arg Asn Gln Gly Ile Gly Val Ile Val Ser Asn Ser Ser Gly Leu Glu
   545 550 555 560
   Leu Lys Asn Ser Asp Ser Ile Thr Leu His Met Gly Ile Ala His Lys
   565 570 575
   Asn Gln Ala Tyr Arg Ala Leu Met Leu Thr Asn Asp Lys Gly Ile Val
   580 585 590
   Asn Tyr Asp Gln Asp Asn Asn Ala Pro Ile Ala Trp Thr Asn Asp His
                   595                 600                 605
   Gly Asp Leu Ile Phe Thr Asn Gln Met Ile Asn Gly Gln Ser Asp Thr
       610                 615                 620
   Ala Val Lys Gly Tyr Leu Asn Pro Glu Val Ala Gly Tyr Leu Ala Val
   625                 630                 635                 640
   Trp Val Pro Val Gly Ala Asn Asp Asn Gln Asp Ala Arg Thr Val Thr
                   645                 650                 655
   Thr Asn Gln Lys Asn Thr Asp Gly Lys Val Leu His Thr Asn Ala Ala
               660                 665                 670
   Leu Asp Ser Lys Leu Met Tyr Glu Gly Phe Ser Asn Phe Gln Lys Met
           675                 680                 685
   Pro Thr Arg Gly Asn Gln Tyr Ala Asn Val Val Ile Thr Lys Asn Ile
       690                 695                 700
   Asp Leu Phe Lys Ser Trp Gly Ile Thr Asp Phe Glu Leu Ala Pro Gln
   705                 710                 715                 720
   Tyr Arg Ser Ser Asp Gly Lys Asp Ile Thr Asp Arg Phe Leu Asp Ser
                   725                 730                 735
   Ile Val Gln Asn Gly Tyr Gly Leu Ser Asp Arg Tyr Asp Leu Gly Phe
               740                 745                 750
   Lys Thr Pro Thr Lys Tyr Gly Thr Asp Gln Asp Leu Arg Lys Ala Ile
           755                 760                 765
   Glu Arg Leu His Gln Ala Gly Met Ser Val Met Ala Asp Phe Val Ala
       770                 775                 780
   Asn Gln Ile Tyr Gly Leu His Ala Asp Lys Glu Val Val Ser Ala Gln
   785                 790                 795                 800
   His Val Asn Ile Asn Gly Asp Thr Lys Leu Val Val Asp Pro Arg Tyr
                   805                 810                 815
   Gly Thr Gln Met Thr Val Val Asn Ser Val Gly Gly Gly Asp Tyr Gln
               820                 825                 830
   Ala Lys Tyr Gly Gly Glu Tyr Leu Asp Thr Ile Ser Lys Leu Tyr Pro
           835                 840                 845
   Gly Leu Leu Leu Asp Ser Asn Gly Gln Lys Ile Asp Leu Ser Thr Lys
       850                 855                 860
   Ile Lys Glu Trp Ser Ala Lys Tyr Leu Asn Gly Ser Asn Ile Pro Gln
   865 870 875 880
   Val Gly Met Gly Tyr Val Leu Lys Asp Trp Asn Asn Gly Gln Tyr Phe
   885 890 895
   His Ile Leu Asp Lys Glu Gly Gln Tyr Ser Leu Pro Thr Gln Leu Val
   900 905 910
   Ser Asn Asp Pro Glu Thr Gln Ile Gly Glu Ser Val Asn Tyr Lys Tyr
   915 920 925
   Phe Ile Gly Asn Ser Asp Ala Thr Tyr Asn Met Tyr His Asn Leu Pro
   930 935 940
   Asn Thr Val Ser Leu Ile Asn Ser Gln Glu Gly Gln Ile Lys Thr Gln
   945 950 955 960
   Gln Ser Gly Val Thr Ser Asp Tyr Glu Gly Gln Gln Val Gln Val Thr
   965 970 975
   Arg Gln Tyr Thr Asp Ser Lys Gly Val Ser Trp Asn Leu Ile Thr Phe
   980 985 990
   Ala Gly Gly Asp Leu Gln Gly Gln Lys Leu Trp Val Asp Ser Arg Ala
   995 1000 1005
   Leu Thr Met Thr Pro Phe Lys Thr Met Asn Gln Ile Ser Phe Ile
   1010 1015 1020
   Ser Tyr Ala Asn Arg Asn Asp Gly Leu Phe Leu Asn Ala Pro Tyr
   1025 1030 1035
   Gln Val Lys Gly Tyr Gln Leu Ala Gly Met Ser Asn Gln Tyr Lys
   1040 1045 1050
   Gly Gln Gln Val Thr Ile Ala Gly Val Ala Asn Val Ser Gly Lys
   1055 1060 1065
   Asp Trp Ser Leu Ile Ser Phe Asn Gly Thr Gln Tyr Trp Ile Asp
   1070 1075 1080
   Ser Gln Ala Leu Asn Thr Asn Phe Thr His Asp Met Asn Gln Lys
   1085 1090 1095
   Val Phe Val Asn Thr Thr Ser Asn Leu Asp Gly Leu Phe Leu Asn
   1100 1105 1110
   Ala Pro Tyr Arg Gln Pro Gly Tyr Lys Leu Ala Gly Leu Ala Lys
   1115 1120 1125
   Asn Tyr Asn Asn Gln Thr Val Thr Val Ser Gln Gln Tyr Phe Asp
   1130 1135 1140
   Asp Gln Gly Thr Val Trp Ser Glu Val Val Leu Gly Gly Gln Thr
   1145 1150 1155
   Val Trp Val Asp Asn His Ala Leu Ala Gln Met Gln Val Ser Asp
   1160 1165 1170
   Thr Ser Gln Gln Leu Tyr Val Asn Ser Asn Gly Arg Asn Asp Gly
   1175 1180 1185
   Leu Phe Leu Asn Ala Pro Tyr Arg Gly Gln Gly Ser Gln Leu Ile
   1190 1195 1200
   Gly
<210> 9
   <211> 1888
   <212> PRT
   <213> Leuconostoc citreum
<400> 9
   Met Glu Met Lys Glu Thr Ile Thr Arg Lys Lys Leu Tyr Lys Ser Gly
   1 5 10 15
   Lys Ser Trp Val Ala Ala Ala Thr Ala Phe Ala Val Met Gly Val Ser
   20 25 30
   Ala Val Thr Thr Val Ser Ala Asp Thr Gln Thr Pro Val Gly Thr Thr
   35 40 45
   Gln Ser Gln Gln Asp Leu Thr Gly Gln Thr Gly Gln Asp Lys Pro Thr
   50 55 60
   Thr Lys Glu Val Ile Asp Lys Lys Glu Pro Val Pro Gln Val Ser Ala
   65 70 75 80
   Gln Asn Val Gly Asp Leu Ser Ala Asp Ala Lys Thr Pro Lys Ala Asp
   85 90 95
   Asp Lys Gln Asp Thr Gln Pro Thr Asn Ala Gln Leu Pro Asp Gln Gly
   100 105 110
   Asn Lys Gln Thr Asn Ser Asn Ser Asp Lys Gly Val Lys Glu Ser Thr
   115 120 125
   Thr Ala Pro Val Lys Thr Thr Asp Val Pro Ser Lys Ser Val Ala Pro
   130 135 140
   Glu Thr Asn Thr Ser Ile Asn Gly Gly Gln Tyr Val Glu Lys Asp Gly
   145 150 155 160
   Gln Phe Val Tyr Ile Asp Gln Ser Gly Lys Gln Val Ser Gly Leu Gln
   165 170 175
   Asn Ile Glu Gly His Thr Gln Tyr Phe Asp Pro Lys Thr Gly Tyr Gln
   180 185 190
   Thr Lys Gly Glu Leu Lys Asn Ile Asp Asp Asn Ala Tyr Tyr Phe Asp
   195 200 205
   Lys Asn Ser Gly Asn Gly Arg Thr Phe Thr Lys Ile Ser Asn Gly Ser
   210 215 220
   Tyr Ser Glu Lys Asp Gly Met Trp Gln Tyr Val Asp Ser His Asp Lys
   225 230 235 240
   Gln Pro Val Lys Gly Leu Tyr Asp Val Glu Gly Asn Leu Gln Tyr Phe
   245 250 255
   Asp Leu Ser Thr Gly Asn Gln Ala Lys His Gln Ile Arg Ser Val Asp
   260 265 270
   Gly Val Thr Tyr Tyr Phe Asp Ala Asp Ser Gly Asn Ala Thr Ala Phe
   275 280 285
   Lys Ala Val Thr Asn Gly Arg Tyr Ala Glu Gln Thr Thr Lys Asp Lys
   290 295 300
   Asp Gly Asn Glu Thr Ser Tyr Trp Ala Tyr Leu Asp Asn Gln Gly Asn
   305 310 315 320
   Ala Ile Lys Gly Leu Asn Asp Val Asn Gly Glu Ile Gln Tyr Phe Asp
   325 330 335
   Glu His Thr Gly Glu Gln Leu Lys Gly His Thr Ala Thr Val Asp Gly
   340 345 350
   Thr Thr Tyr Tyr Phe Glu Gly Asn Lys Gly Asn Leu Val Ser Val Val
   355 360 365
   Asn Thr Ala Pro Thr Gly Gln Tyr Lys Ile Asn Gly Asp Asn Val Tyr
   370 375 380
   Tyr Leu Asp Asn Asn Asn Glu Ala Ile Lys Gly Leu Tyr Gly Ile Asn
   385 390 395 400
   Gly Asn Leu Asn Tyr Phe Asp Leu Ala Thr Gly Ile Gln Leu Lys Gly
   405 410 415
   Gln Ala Lys Asn Ile Asp Gly Ile Gly Tyr Tyr Phe Asp Gln Asn Asn
   420 425 430
   Gly Asn Gly Glu Tyr Arg Tyr Ser Leu Thr Gly Pro Val Val Lys Asp
           435                 440                 445
   Val Tyr Ser Gln His Asn Ala Val Asn Asn Leu Ser Ala Asn Asn Phe
       450                 455                 460
   Lys Asn Leu Val Asp Gly Phe Leu Thr Ala Glu Thr Trp Tyr Arg Pro
   465                 470                 475                 480
   Ala Gln Ile Leu Ser His Gly Thr Asp Trp Val Ala Ser Thr Asp Lys
                   485                 490                 495
   Asp Phe Arg Pro Leu Ile Thr Val Trp Trp Pro Asn Lys Asp Ile Gln
               500                 505                 510
   Val Asn Tyr Leu Lys Leu Met Gln Gln Ile Gly Ile Leu Asp Asn Ser
           515                 520                 525
   Val Val Phe Asp Thr Asn Asn Asp Gln Leu Val Leu Asn Lys Gly Ala
       530                 535                 540
   Glu Ser Ala Gln Ile Gly Ile Glu Lys Lys Val Ser Glu Thr Gly Asn
   545                 550                 555                 560
   Thr Asp Trp Leu Asn Glu Leu Leu Phe Ala Pro Asn Gly Asn Gln Pro
                   565                 570                 575
   Ser Phe Ile Lys Gln Gln Tyr Leu Trp Asn Val Asp Ser Glu Tyr Pro
               580                 585                 590
   Gly Gly Trp Phe Gln Gly Gly Tyr Leu Ala Tyr Gln Asn Ser Asp Leu
           595                 600                 605
   Thr Pro Tyr Ala Asn Thr Asn Pro Asp Tyr Arg Thr His Asn Gly Leu
       610                 615                 620
   Glu Phe Leu Leu Ala Asn Asp Val Asp Asn Ser Asn Pro Val Val Gln
   625                 630                 635                 640
   Ala Glu Gln Leu Asn Trp Leu Tyr Tyr Leu Met Asn Phe Gly Gln Ile
                   645                 650                 655
   Thr Ala Asn Asp Ser Asn Ala Asn Phe Asp Ser Met Arg Ile Asp Ala
               660                 665                 670
   Ile Ser Phe Val Asp Pro Gln Ile Ala Lys Lys Ala Tyr Asp Leu Leu
           675                 680                 685
   Asp Lys Met Tyr Gly Leu Thr Asp Asn Glu Ala Val Ala Asn Gln His
       690                 695                 700
   Ile Ser Ile Val Glu Ala Pro Lys Gly Glu Thr Pro Ile Thr Val Glu
   705 710 715 720
   Lys Gln Ser Ala Leu Val Glu Ser Asn Trp Arg Asp Arg Met Lys Gln
   725 730 735
   Ser Leu Ser Lys Asn Ala Thr Leu Asp Lys Leu Asp Pro Asp Pro Ala
   740 745 750
   Ile Asn Ser Leu Glu Lys Leu Val Ala Asp Asp Leu Val Asn Arg Ser
   755 760 765
   Gln Ser Ser Asp Lys Asp Ser Ser Thr Ile Pro Asn Tyr Ser Ile Val
   770 775 780
   His Ala His Asp Lys Asp Ile Gln Asp Thr Val Ile His Ile Met Lys
   785 790 795 800
   Ile Val Asn Asn Asn Pro Asn Ile Ser Met Ser Asp Phe Thr Met Gln
   805 810 815
   Gln Leu Gln Asn Gly Leu Lys Ala Phe Tyr Glu Asp Gln His Gln Ser
   820 825 830
   Val Lys Lys Tyr Asn Gln Tyr Asn Ile Pro Ser Ala Tyr Ala Leu Leu
   835 840 845
   Leu Thr Asn Lys Asp Thr Val Pro Arg Val Phe Tyr Gly Asp Met Tyr
   850 855 860
   Gln Asp Tyr Gly Asp Asp Leu Asp Gly Gly Gln Tyr Met Ala Thr Lys
   865 870 875 880
   Ser Ile Tyr Tyr Asn Ala Ile Glu Gln Met Met Lys Ala Arg Leu Lys
   885 890 895
   Tyr Val Ala Gly Gly Gln Ile Met Ala Val Thr Lys Ile Lys Asn Asp
   900 905 910
   Gly Ile Asn Lys Asp Gly Thr Asn Lys Ser Gly Glu Val Leu Thr Ser
   915 920 925
   Val Arg Phe Gly Lys Asp Ile Met Asp Ala Gln Gly Gln Gly Thr Ala
   930 935 940
   Glu Ser Arg Asn Gln Gly Ile Gly Val Ile Val Ser Asn Ser Ser Gly
   945 950 955 960
   Leu Glu Leu Lys Asn Ser Asp Ser Ile Thr Leu His Met Gly Ile Ala
   965 970 975
   His Lys Asn Gln Ala Tyr Arg Ala Leu Met Leu Thr Asn Asp Lys Gly
   980 985 990
   Ile Val Asn Tyr Asp Gln Asp Asn Asn Ala Pro Ile Ala Trp Thr Asn
   995 1000 1005
   Asp His Gly Asp Leu Ile Phe Thr Asn Gln Met Ile Asn Gly Gln
   1010 1015 1020
   Ser Asp Thr Ala Val Lys Gly Tyr Leu Asn Pro Glu Val Ala Gly
   1025 1030 1035
   Tyr Leu Ala Val Trp Val Pro Val Gly Ala Asn Asp Asn Gln Asp
   1040 1045 1050
   Ala Arg Thr Val Thr Thr Asn Gln Lys Asn Thr Asp Gly Lys Val
   1055 1060 1065
   Leu His Thr Asn Ala Ala Leu Asp Ser Lys Leu Met Tyr Glu Gly
   1070 1075 1080
   Phe Ser Asn Phe Gln Lys Met Pro Thr Arg Gly Asn Gln Tyr Ala
   1085 1090 1095
   Asn Val Val Ile Thr Lys Asn Ile Asp Leu Phe Lys Ser Trp Gly
   1100 1105 1110
   Ile Thr Asp Phe Glu Leu Ala Pro Gln Tyr Arg Ser Ser Asp Gly
   1115 1120 1125
   Lys Asp Ile Thr Asp Arg Phe Leu Asp Ser Ile Val Gln Asn Gly
   1130 1135 1140
   Tyr Gly Leu Ser Asp Arg Tyr Asp Leu Gly Phe Lys Thr Pro Thr
   1145 1150 1155
   Lys Tyr Gly Thr Asp Gln Asp Leu Arg Lys Ala Ile Glu Arg Leu
   1160 1165 1170
   His Gln Ala Gly Met Ser Val Met Ala Asp Phe Val Ala Asn Gln
   1175 1180 1185
   Ile Tyr Gly Leu His Ala Asp Lys Glu Val Val Ser Ala Gln His
   1190 1195 1200
   Val Asn Ile Asn Gly Asp Thr Lys Leu Val Val Asp Pro Arg Tyr
   1205 1210 1215
   Gly Thr Gln Met Thr Val Val Asn Ser Val Gly Gly Gly Asp Tyr
   1220 1225 1230
   Gln Ala Lys Tyr Gly Gly Glu Tyr Leu Asp Thr Ile Ser Lys Leu
   1235 1240 1245
   Tyr Pro Gly Leu Leu Leu Asp Ser Asn Gly Gln Lys Ile Asp Leu
   1250 1255 1260
   Ser Thr Lys Ile Lys Glu Trp Ser Ala Lys Tyr Leu Asn Gly Ser
   1265 1270 1275
   Asn Ile Pro Gln Val Gly Met Gly Tyr Val Leu Lys Asp Trp Asn
   1280 1285 1290
   Asn Gly Gln Tyr Phe His Ile Leu Asp Lys Glu Gly Gln Tyr Ser
   1295 1300 1305
   Leu Pro Thr Gln Leu Val Ser Asn Asp Pro Glu Thr Gln Ile Gly
   1310 1315 1320
   Glu Ser Val Asn Tyr Lys Tyr Phe Ile Gly Asn Ser Asp Ala Thr
   1325 1330 1335
   Tyr Asn Met Tyr His Asn Leu Pro Asn Thr Val Ser Leu Ile Asn
   1340 1345 1350
   Ser Gln Glu Gly Gln Ile Lys Thr Gln Gln Ser Gly Val Thr Ser
   1355 1360 1365
   Asp Tyr Glu Gly Gln Gln Val Gln Val Thr Arg Gln Tyr Thr Asp
   1370 1375 1380
   Ser Lys Gly Val Ser Trp Asn Leu Ile Thr Phe Ala Gly Gly Asp
   1385 1390 1395
   Leu Gln Gly Gln Lys Leu Trp Val Asp Ser Arg Ala Leu Thr Met
   1400 1405 1410
   Thr Pro Phe Lys Thr Met Asn Gln Ile Ser Phe Ile Ser Tyr Ala
   1415 1420 1425
   Asn Arg Asn Asp Gly Leu Phe Leu Asn Ala Pro Tyr Gln Val Lys
   1430 1435 1440
   Gly Tyr Gln Leu Ala Gly Met Ser Asn Gln Tyr Lys Gly Gln Gln
   1445 1450 1455
   Val Thr Ile Ala Gly Val Ala Asn Val Ser Gly Lys Asp Trp Ser
   1460 1465 1470
   Leu Ile Ser Phe Asn Gly Thr Gln Tyr Trp Ile Asp Ser Gln Ala
   1475 1480 1485
   Leu Asn Thr Asn Phe Thr His Asp Met Asn Gln Lys Val Phe Val
   1490 1495 1500
   Asn Thr Thr Ser Asn Leu Asp Gly Leu Phe Leu Asn Ala Pro Tyr
   1505 1510 1515
   Arg Gln Pro Gly Tyr Lys Leu Ala Gly Leu Ala Lys Asn Tyr Asn
   1520 1525 1530
   Asn Gln Thr Val Thr Val Ser Gln Gln Tyr Phe Asp Asp Gln Gly
   1535 1540 1545
   Thr Val Trp Ser Glu Val Val Leu Gly Gly Gln Thr Val Trp Val
   1550 1555 1560
   Asp Asn His Ala Leu Ala Gln Met Gln Val Ser Asp Thr Ser Gln
   1565 1570 1575
   Gln Leu Tyr Val Asn Ser Asn Gly Arg Asn Asp Gly Leu Phe Leu
   1580 1585 1590
   Asn Ala Pro Tyr Arg Gly Gln Gly Ser Gln Leu Ile Gly Met Thr
   1595 1600 1605
   Ala Asp Tyr Asn Gly Gln His Val Gln Val Thr Lys Gln Gly Gln
   1610 1615 1620
   Asp Ala Tyr Gly Ala Gln Trp Arg Leu Ile Thr Leu Asn Asn Gln
   1625 1630 1635
   Gln Val Trp Val Asp Ser Arg Ala Leu Ser Thr Thr Ile Val Gln
   1640 1645 1650
   Ala Met Asn Asp Asp Met Tyr Val Asn Ser Asn Gln Arg Thr Asp
   1655 1660 1665
   Gly Leu Trp Leu Asn Ala Pro Tyr Thr Met Ser Gly Ala Lys Trp
   1670 1675 1680
   Ala Gly Asp Thr Arg Ser Ala Asn Gly Arg Tyr Val His Ile Ser
   1685 1690 1695
   Lys Ala Tyr Ser Asn Glu Val Gly Asn Thr Tyr Tyr Leu Thr Asn
   1700 1705 1710
   Leu Asn Gly Gln Ser Thr Trp Ile Asp Lys Arg Ala Phe Thr Ala
   1715 1720 1725
   Thr Phe Asp Gln Val Val Ala Leu Asn Ala Thr Ile Val Ala Arg
   1730 1735 1740
   Gln Arg Pro Asp Gly Met Phe Lys Thr Ala Pro Tyr Gly Glu Ala
   1745 1750 1755
   Gly Ala Gln Phe Val Asp Tyr Val Thr Asn Tyr Asn Gln Gln Thr
   1760 1765 1770
   Val Pro Val Thr Lys Gln His Ser Asp Ala Gln Gly Asn Gln Trp
   1775 1780 1785
   Tyr Leu Ala Thr Val Asn Gly Thr Gln Tyr Trp Ile Asp Gln Arg
   1790 1795 1800
   Ser Phe Ser Pro Val Val Thr Lys Val Val Asp Tyr Gln Ala Lys
   1805 1810 1815
   Ile Val Pro Arg Thr Thr Arg Asp Gly Val Phe Ser Gly Ala Pro
   1820 1825 1830
   Tyr Gly Glu Val Asn Ala Lys Leu Val Asn Met Ala Thr Ala Tyr
   1835 1840 1845
   Gln Asn Gln Val Val His Ala Thr Gly Glu Tyr Thr Asn Ala Ser
   1850 1855 1860
   Gly Ile Thr Trp Ser Gln Phe Ala Leu Ser Gly Gln Glu Asp Lys
   1865 1870 1875
   Leu Trp Ile Asp Lys Arg Ala Leu Gln Ala
   1880 1885
<210> 10
   <211> 5667
   <212> DNA
   <213> Leuconostoc citreum
<400> 10
   atggaaatga aagaaacaat cactcgaaaa aagctgtaca agtcaggtaa aagctgggtt 60
   gcggctgcta cagcatttgc cgttatgggg gtatctgcgg taacaactgt cagtgccgat 120
   acacaaacgc cggttggtac aacacagagc caacaggatt tgactggtca gacagggcaa 180
   gacaagccaa caacgaaaga agttatcgac aaaaaggaac cggttcctca agtatcagca 240
   caaaacgttg gtgacttgtc agcagatgca aagactccaa aagctgatga taagcaagat 300
   acgcagccaa caaatgcaca gttacctgat caaggtaaca agcaaacgaa tagtaacagt 360
   gataagggag taaaggagtc aacaacagct cctgttaaaa cgactgatgt accaagcaag 420
   tcagtcgcac cagaaaccaa tactagtatt aatggtggcc aatatgtaga aaaagatggc 480
   caatttgttt atattgatca atctggtaag caggtaagtg gattacaaaa tattgaaggt 540
   catacgcaat attttgatcc gaaaacaggt tatcaaacta aaggtgaatt aaagaatatt 600
   gatgataatg cttattattt tgataaaaat agtggcaatg gtcgtacatt tacaaaaatt      660
   agtaatggta gctattctga aaaagatggc atgtggcagt atgttgatag ccatgacaag      720
   caaccagtaa agggtctata tgatgttgaa gggaatttac agtattttga cctgtcaaca      780
   ggtaatcagg ctaaacatca aatacgttca gttgatggtg tcacttatta ttttgacgca      840
   gacagtggta acgctacggc atttaaagcg gttaccaatg gccgttatgc tgagcagaca      900
   acgaaagata aagatggcaa tgagacaagt tattgggctt atcttgataa tcaggggaat      960
   gctatcaaag gtctaaatga cgttaatggc gaaatacaat attttgatga acatactgga     1020
   gaacaactaa aaggccatac agctacggtt gatgggacaa cgtactattt tgaaggcaat     1080
   aaaggtaatc tcgtcagtgt tgttaacaca gcaccaacag gtcaatataa aattaacgga     1140
   gacaatgttt attatcttga caacaataat gaagcaataa agggattata tggcatcaat     1200
   ggcaatctga attactttga tttagcaacg gggatacaac tcaagggcca agcaaaaaat     1260
   attgatggta ttggttatta ttttgatcaa aataatggca atggtgagta taggtacagt     1320
   ttaacaggtc cagtggttaa agacgtttat tctcaacaca atgctgtgaa taatttgagc     1380
   gcaaataatt ttaagaatct tgtggatggt tttttaacag cagagacctg gtatcgtcca     1440
   gcacaaattt tgtctcatgg tacagactgg gtagcctcaa ctgataaaga tttcagacca     1500
   cttattacag tctggtggcc aaacaaggat attcaggtca actatctaaa gttaatgcaa     1560
   caaatcggta tactagataa ctcagtagta tttgatacaa ataatgatca actagtgtta     1620
   aataaaggtg ctgagagcgc acaaattggc atcgaaaaaa aggttagtga gacaggcaat     1680
   acagattggt taaatgagtt gctttttgct cctaacggaa accaaccatc gtttatcaaa     1740
   caacaatatc tttggaatgt tgattctgaa tatcctggtg gttggtttca gggaggttat     1800
   ctagcttatc aaaatagtga tttaacacca tatgctaata caaatcctga ttatcgaaca     1860
   cataatgggt tagagttctt actagccaat gatgttgaca actccaatcc agtcgtacag     1920
   gctgagcaac tgaactggct atattatttg atgaattttg gccaaattac agcaaatgat     1980
   tcaaatgcca attttgatag tatgagaatt gatgcaattt catttgttga tccacaaatt     2040
   gctaaaaaag cttatgacct gttagataaa atgtatggat taactgacaa tgaggcagtt     2100
   gccaatcaac atatttcaat tgttgaagct ccaaaggggg aaacgccaat taccgttgaa     2160
   aagcagagtg ccctagttga atcgaactgg cgtgatagga tgaagcaatc attatcaaaa     2220
   aatgccactc tagataagct agatcctgac cctgctatca attctttgga aaagcttgtc     2280
   gcagatgatt tagtaaaccg ttcccaaagt tcagataaag acagttcaac gataccaaac     2340
   tactcgatag ttcatgcaca tgataaagac attcaagaca ctgttattca tatcatgaaa     2400
   atagttaata acaatccaaa catatctatg agtgacttca caatgcagca attgcaaaat     2460
   gggttgaaag cattttacga agatcaacac cagtctgtga aaaaatataa ccaatacaat     2520
   attcctagtg catatgcttt gttgttaacc aataaagata ccgtaccacg agttttttat     2580
   ggtgacatgt accaagacta tggtgatgat ttagatggtg gtcagtatat ggctacaaaa     2640
   tcaatttatt ataatgccat tgagcaaatg atgaaggcgc gtttgaagta cgttgctggt 2700
   ggtcaaataa tggccgtgac aaaaataaaa aatgatggta tcaacaaaga tggtaccaat 2760
   aagtcaggtg aggttcttac aagcgttcga tttggaaaag atatcatgga cgcacagggc 2820
   cagggcacag ctgagagtag aaatcagggc attggtgtca tcgtatccaa tagtagcggt 2880
   cttgagttaa agaatagtga cagtatcacc ttgcatatgg ggattgcaca taaaaatcaa 2940
   gcataccgag cattaatgct taccaatgat aaagggattg ttaactacga tcaagataat 3000
   aatgctccga ttgcttggac taatgaccac ggtgatttaa ttttcacgaa tcaaatgatt 3060
   aacggtcaaa gtgatacggc agttaagggt tatcttaatc ctgaagtcgc aggctactta 3120
   gccgtttggg taccagttgg cgccaatgac aaccaagatg cgagaactgt gacaacgaat 3180
   caaaaaaata ctgatggaaa ggtgttgcac acgaatgctg cgcttgattc taaattaatg 3240
   tatgaagggt tctccaattt ccagaaaatg ccgacacgtg gtaatcagta cgctaatgtg 3300
   gttattacta aaaatattga tttatttaaa tcatggggaa ttactgattt tgaattagca 3360
   cctcaatatc gttcaagcga cggaaaagat attaccgacc gttttcttga ctcaattgtt 3420
   caaaatggtt acggattgag cgatcgctat gacctgggat ttaagacacc cactaagtat 3480
   ggcacggacc aagacttgcg aaaagcaatt gaaagattac accaggctgg tatgtcagta 3540
   atggcagatt ttgtagccaa tcaaatttat ggcctacatg ctgataaaga agttgtgtcg 3600
   gctcagcatg tgaatattaa tggtgataca aagttagtag tagatccacg ctacggcaca 3660
   caaatgactg ttgttaattc cgttggtggt ggtgattatc aagctaaata tggtggtgag 3720
   tacttagata ctataagtaa gctttaccct gggttactct tagatagtaa tgggcaaaaa 3780
   atagatttgt ctacaaaaat taaagaatgg tcagcaaaat atctaaacgg gagcaacatt 3840
   cctcaagtgg gtatgggtta tgtcttaaaa gattggaaca atggccagta cttccacatt 3900
   cttgataaag aagggcaata tagcctacca acacaactcg tttctaatga tccagaaaca 3960
   caaataggtg agagtgtaaa ttataaatac tttattggta actctgatgc aacttataat 4020
   atgtatcata atctgcctaa taccgttagc cttattaatt ctcaagaagg tcagattaag 4080
   acacaacagt cgggtgtaac atctgattac gaagggcaac aagtgcaagt cacgcgtcaa 4140
   tacactgaca gtaagggtgt gagttggaac ttaattacct ttgctggtgg tgatttacaa 4200
   ggacaaaagc tttgggtgga tagtcgtgcg ttaactatga caccatttaa aacgatgaat 4260
   caaataagct tcattagtta tgctaaccgc aatgatgggt tgttcttgaa tgcgccatac 4320
   caagtcaagg ggtatcaatt agctgggatg tccaaccaat acaagggcca acaagtgacc 4380
   attgccgggg tggcgaacgt ttctggtaaa gactggagtc tgattagttt taatgggaca 4440
   cagtactgga ttgatagtca ggcattgaat accaatttca cacatgacat gaaccaaaag 4500
   gtctttgtca atacaactag taatcttgat gggttattct taaatgcgcc ataccgtcaa 4560
   ccaggttata agttagccgg tttggctaaa aattacaaca accaaacggt taccgttagt 4620
   caacagtact ttgatgatca aggcacggtc tggagtgagg ttgttcttgg gggtcagacg 4680
   gtctgggttg ataaccatgc attggcacag atgcaagtca gtgatacaag ccaacagctc 4740
   tatgtgaata gcaatggtcg taatgatggg ttattcttga atgcgccata tcgtggtcaa 4800
   gggtcacaac tcataggcat gacggcagat tataatgggc aacatgtaca agtgaccaag 4860
   caagggcaag atgcctacgg tgcacaatgg cgtcttatta cgctaaataa tcaacaggtc 4920
   tgggttgata gtcgcgcttt gagcacaaca atcgtgcaag ccatgaatga tgatatgtat 4980
   gtgaatagca accaacggac agatggtttg tggttaaacg caccttatac gatgagtggg 5040
   gctaaatggg ctggtgatac gcgttcagct aatgggcgct atgtccatat ttcaaaagct 5100
   tattcaaacg aagtcggcaa cacatattac ttgacgaatt tgaatggtca aagcacatgg 5160
   attgacaagc gggcgtttac tgcgaccttt gaccaggtgg tggcattaaa tgcaacgatt 5220
   gtggcacgcc aacgaccaga tgggatgttt aagacagcac catatggtga agcgggggcg 5280
   cagtttgtcg attatgtgac aaactataac cagcaaaccg tgccagtaac aaagcaacat 5340
   tcagatgctc agggtaatca atggtactta gcgacagtga atgggacaca atactggatt 5400
   gatcaacggt cattttcacc agtagtaacg aaggtggttg attatcaagc taagattgtg 5460
   ccacggacaa cacgtgatgg tgtgtttagt ggcgcaccct atggggaagt gaatgctaag 5520
   ctagttaaca tggcaactgc gtatcaaaat caagttgtcc atgcgacagg agaatatacg 5580
   aatgcttcag ggatcacatg gagtcagttc gcgttaagtg ggcaagaaga caagctatgg 5640
   attgataagc gtgctttgca agcttaa 5667
<210> 11
   <211> 3822
   <212> DNA
   <213> Artificial sequence
<220>
   <223> delta PS delta C-1313
<400> 11
   gatacacaaa cgccggttgg tacaacacag agccaacagg atttgactgg tcagacaggg      60
   caagacaagc caacaacgaa agaagttatc gacaaaaagg aaccggttcc tcaagtatca     120
   gcacaaaacg ttggtgactt gtcagcagat gcaaagactc caaaagctga tgataagcaa     180
   gatacgcagc caacaaatgc acagttacct gatcaaggta acaagcaaac gaatagtaac     240
   agtgataagg gagtaaagga gtcaacaaca gctcctgtta aaacgactga tgtaccaagc     300
   aagtcagtcg caccagaaac caatactagt attaatggtg gccaatatgt agaaaaagat     360
   ggccaatttg tttatattga tcaatctggt aagcaggtaa gtggattaca aaatattgaa     420
   ggtcatacgc aatattttga tccgaaaaca ggttatcaaa ctaaaggtga attaaagaat     480
   attgatgata atgcttatta ttttgataaa aatagtggca atggtcgtac atttacaaaa     540
   attagtaatg gtagctattc tgaaaaagat ggcatgtggc agtatgttga tagccatgac     600
   aagcaaccag taaagggtct atatgatgtt gaagggaatt tacagtattt tgacctgtca     660
   acaggtaatc aggctaaaca tcaaatacgt tcagttgatg gtgtcactta ttattttgac     720
   gcagacagtg gtaacgctac ggcatttaaa gcggttacca atggccgtta tgctgagcag 780
   acaacgaaag ataaagatgg caatgagaca agttattggg cttatcttga taatcagggg 840
   aatgctatca aaggtctaaa tgacgttaat ggcgaaatac aatattttga tgaacatact 900
   ggagaacaac taaaaggcca tacagctacg gttgatggga caacgtacta ttttgaaggc 960
   aataaaggta atctcgtcag tgttgttaac acagcaccaa caggtcaata taaaattaac 1020
   ggagacaatg tttattatct tgacaacaat aatgaagcaa taaagggatt atatggcatc 1080
   aatggcaatc tgaattactt tgatttagca acggggatac aactcaaggg ccaagcaaaa 1140
   aatattgatg gtattggtta ttattttgat caaaataatg gcaatggtga gtataggtac 1200
   agtttaacag gtccagtggt taaagacgtt tattctcaac acaatgctgt gaataatttg 1260
   agcgcaaata attttaagaa tcttgtggat ggttttttaa cagcagagac ctggtatcgt 1320
   ccagcacaaa ttttgtctca tggtacagac tgggtagcct caactgataa agatttcaga 1380
   ccacttatta cagtctggtg gccaaacaag gatattcagg tcaactatct aaagttaatg 1440
   caacaaatcg gtatactaga taactcagta gtatttgata caaataatga tcaactagtg 1500
   ttaaataaag gtgctgagag cgcacaaatt ggcatcgaaa aaaaggttag tgagacaggc 1560
   aatacagatt ggttaaatga gttgcttttt gctcctaacg gaaaccaacc atcgtttatc 1620
   aaacaacaat atctttggaa tgttgattct gaatatcctg gtggttggtt tcagggaggt 1680
   tatctagctt atcaaaatag tgatttaaca ccatatgcta atacaaatcc tgattatcga 1740
   acacataatg ggttagagtt cttactagcc aatgatgttg acaactccaa tccagtcgta 1800
   caggctgagc aactgaactg gctatattat ttgatgaatt ttggccaaat tacagcaaat 1860
   gattcaaatg ccaattttga tagtatgaga attgatgcaa tttcatttgt tgatccacaa 1920
   attgctaaaa aagcttatga cctgttagat aaaatgtatg gattaactga caatgaggca 1980
   gttgccaatc aacatatttc aattgttgaa gctccaaagg gggaaacgcc aattaccgtt 2040
   gaaaagcaga gtgccctagt tgaatcgaac tggcgtgata ggatgaagca atcattatca 2100
   aaaaatgcca ctctagataa gctagatcct gaccctgcta tcaattcttt ggaaaagctt 2160
   gtcgcagatg atttagtaaa ccgttcccaa agttcagata aagacagttc aacgatacca 2220
   aactactcga tagttcatgc acatgataaa gacattcaag acactgttat tcatatcatg 2280
   aaaatagtta ataacaatcc aaacatatct atgagtgact tcacaatgca gcaattgcaa 2340
   aatgggttga aagcatttta cgaagatcaa caccagtctg tgaaaaaata taaccaatac 2400
   aatattccta gtgcatatgc tttgttgtta accaataaag ataccgtacc acgagttttt 2460
   tatggtgaca tgtaccaaga ctatggtgat gatttagatg gtggtcagta tatggctaca 2520
   aaatcaattt attataatgc cattgagcaa atgatgaagg cgcgtttgaa gtacgttgct 2580
   ggtggtcaaa taatggccgt gacaaaaata aaaaatgatg gtatcaacaa agatggtacc 2640
   aataagtcag gtgaggttct tacaagcgtt cgatttggaa aagatatcat ggacgcacag 2700
   ggccagggca cagctgagag tagaaatcag ggcattggtg tcatcgtatc caatagtagc 2760
   ggtcttgagt taaagaatag tgacagtatc accttgcata tggggattgc acataaaaat      2820
   caagcatacc gagcattaat gcttaccaat gataaaggga ttgttaacta cgatcaagat      2880
   aataatgctc cgattgcttg gactaatgac cacggtgatt taattttcac gaatcaaatg      2940
   attaacggtc aaagtgatac ggcagttaag ggttatctta atcctgaagt cgcaggctac      3000
   ttagccgttt gggtaccagt tggcgccaat gacaaccaag atgcgagaac tgtgacaacg      3060
   aatcaaaaaa atactgatgg aaaggtgttg cacacgaatg ctgcgcttga ttctaaatta      3120
   atgtatgaag ggttctccaa tttccagaaa atgccgacac gtggtaatca gtacgctaat      3180
   gtggttatta ctaaaaatat tgatttattt aaatcatggg gaattactga ttttgaatta      3240
   gcacctcaat atcgttcaag cgacggaaaa gatattaccg accgttttct tgactcaatt      3300
   gttcaaaatg gttacggatt gagcgatcgc tatgacctgg gatttaagac acccactaag      3360
   tatggcacgg accaagactt gcgaaaagca attgaaagat tacaccaggc tggtatgtca      3420
   gtaatggcag attttgtagc caatcaaatt tatggcctac atgctgataa agaagttgtg      3480
   tcggctcagc atgtgaatat taatggtgat acaaagttag tagtagatcc acgctacggc      3540
   acacaaatga ctgttgttaa ttccgttggt ggtggtgatt atcaagctaa atatggtggt      3600
   gagtacttag atactataag taagctttac cctgggttac tcttagatag taatgggcaa      3660
   aaaatagatt tgtctacaaa aattaaagaa tggtcagcaa aatatctaaa cgggagcaac      3720
   attcctcaag tgggtatggg ttatgtctta aaagattgga acaatggcca gtacttccac      3780
   attcttgata aagaagggca atatagccta ccaacacaac tc                         3822
<210> 12
   <211> 1274
   <212> PRT
   <213> Artificial Sequence
<220>
   <223> Delta PS, delta C-1313
<400> 12
   Asp Thr Gln Thr Pro Val Gly Thr Thr Gln Ser Gln Gln Asp Leu Thr
   1               5                   10                  15
   Gly Gln Thr Gly Gln Asp Lys Pro Thr Thr Lys Glu Val Ile Asp Lys
               20                  25                  30
   Lys Glu Pro Val Pro Gln Val Ser Ala Gln Asn Val Gly Asp Leu Ser
           35                  40                  45
   Ala Asp Ala Lys Thr Pro Lys Ala Asp Asp Lys Gln Asp Thr Gln Pro
       50                  55                  60
   Thr Asn Ala Gln Leu Pro Asp Gln Gly Asn Lys Gln Thr Asn Ser Asn
   65                  70                  75                  80
   Ser Asp Lys Gly Val Lys Glu Ser Thr Thr Ala Pro Val Lys Thr Thr
   85 90 95
   Asp Val Pro Ser Lys Ser Val Ala Pro Glu Thr Asn Thr Ser Ile Asn
   100 105 110
   Gly Gly Gln Tyr Val Glu Lys Asp Gly Gln Phe Val Tyr Ile Asp Gln
   115 120 125
   Ser Gly Lys Gln Val Ser Gly Leu Gln Asn Ile Glu Gly His Thr Gln
   130 135 140
   Tyr Phe Asp Pro Lys Thr Gly Tyr Gln Thr Lys Gly Glu Leu Lys Asn
   145 150 155 160
   Ile Asp Asp Asn Ala Tyr Tyr Phe Asp Lys Asn Ser Gly Asn Gly Arg
   165 170 175
   Thr Phe Thr Lys Ile Ser Asn Gly Ser Tyr Ser Glu Lys Asp Gly Met
   180 185 190
   Trp Gln Tyr Val Asp Ser His Asp Lys Gln Pro Val Lys Gly Leu Tyr
   195 200 205
   Asp Val Glu Gly Asn Leu Gln Tyr Phe Asp Leu Ser Thr Gly Asn Gln
   210 215 220
   Ala Lys His Gln Ile Arg Ser Val Asp Gly Val Thr Tyr Tyr Phe Asp
   225 230 235 240
   Ala Asp Ser Gly Asn Ala Thr Ala Phe Lys Ala Val Thr Asn Gly Arg
   245 250 255
   Tyr Ala Glu Gln Thr Thr Lys Asp Lys Asp Gly Asn Glu Thr Ser Tyr
   260 265 270
   Trp Ala Tyr Leu Asp Asn Gln Gly Asn Ala Ile Lys Gly Leu Asn Asp
   275 280 285
   Val Asn Gly Glu Ile Gln Tyr Phe Asp Glu His Thr Gly Glu Gln Leu
   290 295 300
   Lys Gly His Thr Ala Thr Val Asp Gly Thr Thr Tyr Tyr Phe Glu Gly
   305 310 315 320
   Asn Lys Gly Asn Leu Val Ser Val Val Asn Thr Ala Pro Thr Gly Gln
   325 330 335
   Tyr Lys Ile Asn Gly Asp Asn Val Tyr Tyr Leu Asp Asn Asn Asn Glu
   340 345 350
   Ala Ile Lys Gly Leu Tyr Gly Ile Asn Gly Asn Leu Asn Tyr Phe Asp
   355 360 365
   Leu Ala Thr Gly Ile Gln Leu Lys Gly Gln Ala Lys Asn Ile Asp Gly
   370 375 380
   Ile Gly Tyr Tyr Phe Asp Gln Asn Asn Gly Asn Gly Glu Tyr Arg Tyr
   385 390 395 400
   Ser Leu Thr Gly Pro Val Val Lys Asp Val Tyr Ser Gln His Asn Ala
   405 410 415
   Val Asn Asn Leu Ser Ala Asn Asn Phe Lys Asn Leu Val Asp Gly Phe
   420 425 430
   Leu Thr Ala Glu Thr Trp Tyr Arg Pro Ala Gln Ile Leu Ser His Gly
   435 440 445
   Thr Asp Trp Val Ala Ser Thr Asp Lys Asp Phe Arg Pro Leu Ile Thr
   450 455 460
   Val Trp Trp Pro Asn Lys Asp Ile Gln Val Asn Tyr Leu Lys Leu Met
   465 470 475 480
   Gln Gln Ile Gly Ile Leu Asp Asn Ser Val Val Phe Asp Thr Asn Asn
   485 490 495
   Asp Gln Leu Val Leu Asn Lys Gly Ala Glu Ser Ala Gln Ile Gly Ile
   500 505 510
   Glu Lys Lys Val Ser Glu Thr Gly Asn Thr Asp Trp Leu Asn Glu Leu
   515 520 525
   Leu Phe Ala Pro Asn Gly Asn Gln Pro Ser Phe Ile Lys Gln Gln Tyr
   530 535 540
   Leu Trp Asn Val Asp Ser Glu Tyr Pro Gly Gly Trp Phe Gln Gly Gly
   545 550 555 560
   Tyr Leu Ala Tyr Gln Asn Ser Asp Leu Thr Pro Tyr Ala Asn Thr Asn
   565 570 575
   Pro Asp Tyr Arg Thr His Asn Gly Leu Glu Phe Leu Leu Ala Asn Asp
   580 585 590
   Val Asp Asn Ser Asn Pro Val Val Gln Ala Glu Gln Leu Asn Trp Leu
   595 600 605
   Tyr Tyr Leu Met Asn Phe Gly Gln Ile Thr Ala Asn Asp Ser Asn Ala
   610 615 620
   Asn Phe Asp Ser Met Arg Ile Asp Ala Ile Ser Phe Val Asp Pro Gln
   625 630 635 640
   Ile Ala Lys Lys Ala Tyr Asp Leu Leu Asp Lys Met Tyr Gly Leu Thr
   645 650 655
   Asp Asn Glu Ala Val Ala Asn Gln His Ile Ser Ile Val Glu Ala Pro
   660 665 670
   Lys Gly Glu Thr Pro Ile Thr Val Glu Lys Gln Ser Ala Leu Val Glu
   675 680 685
   Ser Asn Trp Arg Asp Arg Met Lys Gln Ser Leu Ser Lys Asn Ala Thr
   690 695 700
   Leu Asp Lys Leu Asp Pro Asp Pro Ala Ile Asn Ser Leu Glu Lys Leu
   705 710 715 720
   Val Ala Asp Asp Leu Val Asn Arg Ser Gln Ser Ser Asp Lys Asp Ser
   725 730 735
   Ser Thr Ile Pro Asn Tyr Ser Ile Val His Ala His Asp Lys Asp Ile
   740 745 750
   Gln Asp Thr Val Ile His Ile Met Lys Ile Val Asn Asn Asn Pro Asn
   755 760 765
   Ile Ser Met Ser Asp Phe Thr Met Gln Gln Leu Gln Asn Gly Leu Lys
   770 775 780
   Ala Phe Tyr Glu Asp Gln His Gln Ser Val Lys Lys Tyr Asn Gln Tyr
   785 790 795 800
   Asn Ile Pro Ser Ala Tyr Ala Leu Leu Leu Thr Asn Lys Asp Thr Val
   805 810 815
   Pro Arg Val Phe Tyr Gly Asp Met Tyr Gln Asp Tyr Gly Asp Asp Leu
   820 825 830
   Asp Gly Gly Gln Tyr Met Ala Thr Lys Ser Ile Tyr Tyr Asn Ala Ile
   835 840 845
   Glu Gln Met Met Lys Ala Arg Leu Lys Tyr Val Ala Gly Gly Gln Ile
   850 855 860
   Met Ala Val Thr Lys Ile Lys Asn Asp Gly Ile Asn Lys Asp Gly Thr
   865 870 875 880
   Asn Lys Ser Gly Glu Val Leu Thr Ser Val Arg Phe Gly Lys Asp Ile
   885 890 895
   Met Asp Ala Gln Gly Gln Gly Thr Ala Glu Ser Arg Asn Gln Gly Ile
   900 905 910
   Gly Val Ile Val Ser Asn Ser Ser Gly Leu Glu Leu Lys Asn Ser Asp
   915 920 925
   Ser Ile Thr Leu His Met Gly Ile Ala His Lys Asn Gln Ala Tyr Arg
   930 935 940
   Ala Leu Met Leu Thr Asn Asp Lys Gly Ile Val Asn Tyr Asp Gln Asp
   945 950 955 960
   Asn Asn Ala Pro Ile Ala Trp Thr Asn Asp His Gly Asp Leu Ile Phe
   965 970 975
   Thr Asn Gln Met Ile Asn Gly Gln Ser Asp Thr Ala Val Lys Gly Tyr
   980 985 990
   Leu Asn Pro Glu Val Ala Gly Tyr Leu Ala Val Trp Val Pro Val Gly
   995 1000 1005
   Ala Asn Asp Asn Gln Asp Ala Arg Thr Val Thr Thr Asn Gln Lys
   1010 1015 1020
   Asn Thr Asp Gly Lys Val Leu His Thr Asn Ala Ala Leu Asp Ser
   1025 1030 1035
   Lys Leu Met Tyr Glu Gly Phe Ser Asn Phe Gln Lys Met Pro Thr
   1040 1045 1050
   Arg Gly Asn Gln Tyr Ala Asn Val Val Ile Thr Lys Asn Ile Asp
   1055 1060 1065
   Leu Phe Lys Ser Trp Gly Ile Thr Asp Phe Glu Leu Ala Pro Gln
   1070 1075 1080
   Tyr Arg Ser Ser Asp Gly Lys Asp Ile Thr Asp Arg Phe Leu Asp
   1085 1090 1095
   Ser Ile Val Gln Asn Gly Tyr Gly Leu Ser Asp Arg Tyr Asp Leu
   1100 1105 1110
   Gly Phe Lys Thr Pro Thr Lys Tyr Gly Thr Asp Gln Asp Leu Arg
   1115 1120 1125
   Lys Ala Ile Glu Arg Leu His Gln Ala Gly Met Ser Val Met Ala
   1130 1135 1140
   Asp Phe Val Ala Asn Gln Ile Tyr Gly Leu His Ala Asp Lys Glu
   1145 1150 1155
   Val Val Ser Ala Gln His Val Asn Ile Asn Gly Asp Thr Lys Leu
   1160 1165 1170
   Val Val Asp Pro Arg Tyr Gly Thr Gln Met Thr Val Val Asn Ser
   1175 1180 1185
   Val Gly Gly Gly Asp Tyr Gln Ala Lys Tyr Gly Gly Glu Tyr Leu
   1190 1195 1200
   Asp Thr Ile Ser Lys Leu Tyr Pro Gly Leu Leu Leu Asp Ser Asn
   1205 1210 1215
   Gly Gln Lys Ile Asp Leu Ser Thr Lys Ile Lys Glu Trp Ser Ala
   1220 1225 1230
   Lys Tyr Leu Asn Gly Ser Asn Ile Pro Gln Val Gly Met Gly Tyr
   1235 1240 1245
   Val Leu Lys Asp Trp Asn Asn Gly Gln Tyr Phe His Ile Leu Asp
   1250 1255 1260
   Lys Glu Gly Gln Tyr Ser Leu Pro Thr Gln Leu
   1265 1270
<210> 13
   <211> 4244
   <212> DNA
   <213> Leuconostoc citreum
<400> 13
   atggaaatga aagaaacaat cactcgaaaa aagctgtaca agtcaggtaa aagctgggtt      60
   gcggctgcta cagcatttgc cgttatgggg gtatctgcgg taacaactgt cagtgccgat     120
   acacaaacgc cggttggtac aacacagagc caacagaatt tgactggtca gacagggcaa     180
   gacaagccaa caacgaaaga agttatcgac aaaaaggaac cggttcccca ggtatcagca     240
   caaaatgctg gtgacttgtc agcagatgca aagactccaa aagctgatga taagcaagat     300
   acgcagccaa caaatgcaca gttacctgat caaggtaaca agcaaacgaa tagtaacagt     360
   gataagggag taaaggagtc aacaacagct cctgttaaaa cgactgatgt accaagcaag     420
   tcagtcacac cagaaacaaa tactagtatt aatggtggac aatatgtaga aaaagatggc     480
   caatttgttt atattgatca atctggtaag caggtaagtg gattacaaaa tattgaaggt     540
   catacgcaat attttgatcc gaaaacaggt tatcaaacta aaggtgaatt aaagaatatt     600
   gatgataatg cttattattt tgataaaaat agtggcaatg gtcgtacatt tacaaaaatt     660
   agtaatggta gctattctga aaaagatggc atgtggcagt atgttgatag ccatgacaag     720
   caaccagtaa agggtctata tgatgttgaa gggaatttac agtattttga cctgtcaaca     780
   ggtaatcagg ctaaacatca aatacgttca gttgatggtg tcacttatta tttcgacgca     840
   gacagtggta acgctacggc atttaaagcg gttaccaatg gccgttatgc tgagcagaca     900
   acgaaagata aagatggcaa tgagacaagt tattgggctt atcttgataa tcaggggaat     960
   gctgtcaaag gtctaaatga cgtcaatggt gaaatacagt actttgatga aatcgctggc 1020
   gcacagctaa aaggtcacac agctacggtt gatggtgtca cttactattt tgaaagcaat 1080
   aaaggaaatc tcgtaagtgt tgttaacgca gcgccgacag gacaatataa aatagatggt 1140
   gataaagtat actatcttga taatcaaaat caaccattaa agggattgta tagtatcaat 1200
   ggcaatctga attactttga tttagccacg gggatacaag tcaaaggtca ggcagaaaac 1260
   atcaatggta ttggttatta ttttgatcaa aataatggca atggtgagta taggtacagt 1320
   ttaacaggtc cagtggttaa agacgtttat tctcaacaca atgctgtgaa taatttgagc 1380
   gcaaataatt ttaggaatct tgtggatggt ttcttaacag cagagacctg gtatcgtcca 1440
   gcacaaattt tgtctcaggg taaagactgg gtagcctcaa ctgataaaga tttcagacca 1500
   cttattacag tctggtggcc aaacaaggat attcaggtca actatctaaa gttaatgcaa 1560
   caaatcggta tagtagataa ctcagtagta tttgatacaa ataatgatca actagtgtta 1620
   aataaaggtg ctgagagcgc acaaattggc atcgaaaaaa aggttagcga gacaggcaat 1680
   acagattggt taaatgagtt gctttttgct cctaacggaa accaaccatc gtttatcaaa 1740
   caacaatatc tttggaatgt tgattctgaa tatcctggtg gttggtttca gggaggttat 1800
   ctatcttatc aaaatagtga tttaacacca tatgctaata caaatcctga ttatcgaaca 1860
   cataatgggt tagagttctt actagccaat gatgttgaca actccaatcc agtcgtacag 1920
   gctgagcaac tgaactggct atattatttg atgaattttg gccaaattac agcaaatgat 1980
   tcaaatgcca attttgatag tatgagaatt gatgcaattt catttgttga tccacaaatt 2040
   gctaaaaaag cttatgacct gttagataaa atgtatggat taactgacaa tgaggcagtt 2100
   gccaatcaac atatttcaat tgttgaagct ccaaaggggg aaacgccaat taccgttgaa 2160
   aagcagagtg ccctagttga atcgaactgg cgtgatagga tgaagcaatc attatcaaaa 2220
   aatgccactc tagataagct agatcctgac cctgctatca attctttgga aaagcttgtc 2280
   gcagatgatt tagtaaaccg ttcccaaagt tcagataaag acagttcaac gataccaaac 2340
   tactcgatag ttcatgcaca tgataaagac attcaagaca ctgttattca tatcatgaaa 2400
   atagttaata acaatccaaa catatctatg agtgacttca caatgcagca attgcaaaat 2460
   gggttgaaag cattttacga agatcaacac cagtctgtga aaaaatataa ccaatacaat 2520
   attcctagtg catatgcttt gttgttaacc aataaagata ccgtaccacg agttttttat 2580
   ggtgacatgt accaagacta tggtgatgat ttagatggtg gtcagtatat ggctacaaaa 2640
   tcaatttatt ataatgccat tgagcaaatg atgaaggcgc gtttgaagta cgttgctggt 2700
   ggtcaaataa tggccgtgac aaaaataaaa aatgatggta tcaacaaaga tggtaccaat 2760
   aagtcaggtg aggttcttac aagcgttcga tttggaaaag atatcatgga cgcacagggc 2820
   cagggcacag ctgagagtag aaatcagggc attggtgtca tcgtatccaa tagtagcggt 2880
   cttgagttaa agaatagtga cagtatcacc ttgcatatgg ggattgcaca taaaaatcaa 2940
   gcataccgag cattaatgct taccaatgat aaagggattg ttaactacga tcaagataat 3000
   aatgctccga ttgcttggac taatgaccac ggtgatttaa ttttcacgaa tcaaatgatt 3060
   aacggtcaaa gtgatacggc agttaagggt tatcttaatc ctgaagtcgc aggctactta 3120
   gccgtttggg taccagttgg cgccaatgac aaccaagatg cgagaactgt gacaacgaat 3180
   caaaaaaata ctgatggaaa ggtgttgcac acgaatgctg cgcttgattc taaattaatg 3240
   tatgaagggt tctccaattt ccagaaaatg ccgacacgtg gtaatcagta cgctaatgtg 3300
   attattgcta aaaatattga tttatttaaa tcatggggaa ttactgattt tgaattagca 3360
   cctcaatatc gttcaagcga cggaaaagat attaccgacc gttttcttga ctcaattgtt 3420
   caaaatggtt acggattgag cgatcgctat gacctgggat ttaagacacc cactaagtat 3480
   ggcacggacc aagacttgcg aaaagcaatt gaaagattac accaggctgg tatgtcagta 3540
   atggcagatt ttgtagccaa tcaaatttat ggcctacatg ctgataaaga agttgtgtcg 3600
   gctcagcatg tgaatattaa tggtgataca aagttagtag tagatccacg ctacggcaca 3660
   caaatgactg ttgttaattc cgttggtggt ggtgattatc aagctaaata tggtggtgag 3720
   tacttagata ctataagtaa gctttaccct gggttactct tagatagtaa tgggcaaaaa 3780
   atagatttgt ctacaaaaat taaagaatgg tcagcaaaat atctaaacgg gagcaatatt 3840
   cctcaagtgg gtatgggtta tgtcttaaaa gattggaaca atggccagta cttccacatt 3900
   cttgataaag aagggcaata tagcctacca acacaactcg tttctaatga tccagaaaca 3960
   caaataggtg agagtgtaaa ttataaatac tttattggta actctgatgc aacttataat 4020
   atgtatcata atctgcctaa taccgttagc cttattaatt ctcaagaagg tcagattaag 4080
   acacaacagt cgggtgtaac atctgattac gaagggcaac aagtgcaagt cacgcgccag 4140
   tacacagata gtaagggtgt gagttggaac ttaattacct ttgctggtgg tgatttacaa 4200
   ggacaaaagc tttgggtgga tagtcgtgcg ttaactatga cacc 4244
<210> 14
   <211> 1611
   <212> PRT
   <213> Leuconostoc citreum
<400> 14
   Met Glu Met Lys Glu Thr Ile Thr Arg Lys Lys Leu Tyr Lys Ser Gly
   1               5                   10                  15
   Lys Ser Trp Val Ala Ala Ala Thr Ala Phe Ala Val Met Gly Val Ser
               20                  25                  30
   Ala Val Thr Thr Val Ser Ala Asp Thr Gln Thr Pro Val Gly Thr Thr
           35                  40                  45
   Gln Ser Gln Gln Asn Leu Thr Gly Gln Thr Gly Gln Asp Lys Pro Thr
       50                  55                  60
   Thr Lys Glu Val Ile Asp Lys Lys Glu Pro Val Pro Gln Val Ser Ala
   65 70 75 80
   Gln Asn Ala Gly Asp Leu Ser Ala Asp Ala Lys Thr Pro Lys Ala Asp
   85 90 95
   Asp Lys Gln Asp Thr Gln Pro Thr Asn Ala Gln Leu Pro Asp Gln Gly
   100 105 110
   Asn Lys Gln Thr Asn Ser Asn Ser Asp Lys Gly Val Lys Glu Ser Thr
   115 120 125
   Thr Ala Pro Val Lys Thr Thr Asp Val Pro Ser Lys Ser Val Thr Pro
   130 135 140
   Glu Thr Asn Thr Ser Ile Asn Gly Gly Gln Tyr Val Glu Lys Asp Gly
   145 150 155 160
   Gln Phe Val Tyr Ile Asp Gln Ser Gly Lys Gln Val Ser Gly Leu Gln
   165 170 175
   Asn Ile Glu Gly His Thr Gln Tyr Phe Asp Pro Lys Thr Gly Tyr Gln
   180 185 190
   Thr Lys Gly Glu Leu Lys Asn Ile Asp Asp Asn Ala Tyr Tyr Phe Asp
   195 200 205
   Lys Asn Ser Gly Asn Gly Arg Thr Phe Thr Lys Ile Ser Asn Gly Ser
   210 215 220
   Tyr Ser Glu Lys Asp Gly Met Trp Gln Tyr Val Asp Ser His Asp Lys
   225 230 235 240
   Gln Pro Val Lys Gly Leu Tyr Asp Val Glu Gly Asn Leu Gln Tyr Phe
   245 250 255
   Asp Leu Ser Thr Gly Asn Gln Ala Lys His Gln Ile Arg Ser Val Asp
   260 265 270
   Gly Val Thr Tyr Tyr Phe Asp Ala Asp Ser Gly Asn Ala Thr Ala Phe
   275 280 285
   Lys Ala Val Thr Asn Gly Arg Tyr Ala Glu Gln Thr Thr Lys Asp Lys
   290 295 300
   Asp Gly Asn Glu Thr Ser Tyr Trp Ala Tyr Leu Asp Asn Gln Gly Asn
   305 310 315 320
   Ala Val Lys Gly Leu Asn Asp Val Asn Gly Glu Ile Gln Tyr Phe Asp
   325 330 335
   Glu Ile Ala Gly Ala Gln Leu Lys Gly His Thr Ala Thr Val Asp Gly
   340 345 350
   Val Thr Tyr Tyr Phe Glu Ser Asn Lys Gly Asn Leu Val Ser Val Val
   355 360 365
   Asn Ala Ala Pro Thr Gly Gln Tyr Lys Ile Asp Gly Asp Lys Val Tyr
   370 375 380
   Tyr Leu Asp Asn Gln Asn Gln Pro Leu Lys Gly Leu Tyr Ser Ile Asn
   385 390 395 400
   Gly Asn Leu Asn Tyr Phe Asp Leu Ala Thr Gly Ile Gln Val Lys Gly
   405 410 415
   Gln Ala Glu Asn Ile Asn Gly Ile Gly Tyr Tyr Phe Asp Gln Asn Asn
   420 425 430
   Gly Asn Gly Glu Tyr Arg Tyr Ser Leu Thr Gly Pro Val Val Lys Asp
   435 440 445
   Val Tyr Ser Gln His Asn Ala Val Asn Asn Leu Ser Ala Asn Asn Phe
   450 455 460
   Arg Asn Leu Val Asp Gly Phe Leu Thr Ala Glu Thr Trp Tyr Arg Pro
   465 470 475 480
   Ala Gln Ile Leu Ser Gln Gly Lys Asp Trp Val Ala Ser Thr Asp Lys
   485 490 495
   Asp Phe Arg Pro Leu Ile Thr Val Trp Trp Pro Asn Lys Asp Ile Gln
   500 505 510
   Val Asn Tyr Leu Lys Leu Met Gln Gln Ile Gly Ile Val Asp Asn Ser
   515 520 525
   Val Val Phe Asp Thr Asn Asn Asp Gln Leu Val Leu Asn Lys Gly Ala
   530 535 540
   Glu Ser Ala Gln Ile Gly Ile Glu Lys Lys Val Ser Glu Thr Gly Asn
   545 550 555 560
   Thr Asp Trp Leu Asn Glu Leu Leu Phe Ala Pro Asn Gly Asn Gln Pro
   565 570 575
   Ser Phe Ile Lys Gln Gln Tyr Leu Trp Asn Val Asp Ser Glu Tyr Pro
   580 585 590
   Gly Gly Trp Phe Gln Gly Gly Tyr Leu Ser Tyr Gln Asn Ser Asp Leu
   595 600 605
   Thr Pro Tyr Ala Asn Thr Asn Pro Asp Tyr Arg Thr His Asn Gly Leu
   610 615 620
   Glu Phe Leu Leu Ala Asn Asp Val Asp Asn Ser Asn Pro Val Val Gln
   625 630 635 640
   Ala Glu Gln Leu Asn Trp Leu Tyr Tyr Leu Met Asn Phe Gly Gln Ile
   645 650 655
   Thr Ala Asn Asp Ser Asn Ala Asn Phe Asp Ser Met Arg Ile Asp Ala
   660 665 670
   Ile Ser Phe Val Asp Pro Gln Ile Ala Lys Lys Ala Tyr Asp Leu Leu
   675 680 685
   Asp Lys Met Tyr Gly Leu Thr Asp Asn Glu Ala Val Ala Asn Gln His
   690 695 700
   Ile Ser Ile Val Glu Ala Pro Lys Gly Glu Thr Pro Ile Thr Val Glu
   705 710 715 720
   Lys Gln Ser Ala Leu Val Glu Ser Asn Trp Arg Asp Arg Met Lys Gln
   725 730 735
   Ser Leu Ser Lys Asn Ala Thr Leu Asp Lys Leu Asp Pro Asp Pro Ala
   740 745 750
   Ile Asn Ser Leu Glu Lys Leu Val Ala Asp Asp Leu Val Asn Arg Ser
   755 760 765
   Gln Ser Ser Asp Lys Asp Ser Ser Thr Ile Pro Asn Tyr Ser Ile Val
   770 775 780
   His Ala His Asp Lys Asp Ile Gln Asp Thr Val Ile His Ile Met Lys
   785 790 795 800
   Ile Val Asn Asn Asn Pro Asn Ile Ser Met Ser Asp Phe Thr Met Gln
   805 810 815
   Gln Leu Gln Asn Gly Leu Lys Ala Phe Tyr Glu Asp Gln His Gln Ser
   820 825 830
   Val Lys Lys Tyr Asn Gln Tyr Asn Ile Pro Ser Ala Tyr Ala Leu Leu
   835 840 845
   Leu Thr Asn Lys Asp Thr Val Pro Arg Val Phe Tyr Gly Asp Met Tyr
   850 855 860
   Gln Asp Tyr Gly Asp Asp Leu Asp Gly Gly Gln Tyr Met Ala Thr Lys
   865 870 875 880
   Ser Ile Tyr Tyr Asn Ala Ile Glu Gln Met Met Lys Ala Arg Leu Lys
   885 890 895
   Tyr Val Ala Gly Gly Gln Ile Met Ala Val Thr Lys Ile Lys Asn Asp
   900 905 910
   Gly Ile Asn Lys Asp Gly Thr Asn Lys Ser Gly Glu Val Leu Thr Ser
   915 920 925
   Val Arg Phe Gly Lys Asp Ile Met Asp Ala Gln Gly Gln Gly Thr Ala
   930 935 940
   Glu Ser Arg Asn Gln Gly Ile Gly Val Ile Val Ser Asn Ser Ser Gly
   945 950 955 960
   Leu Glu Leu Lys Asn Ser Asp Ser Ile Thr Leu His Met Gly Ile Ala
   965 970 975
   His Lys Asn Gln Ala Tyr Arg Ala Leu Met Leu Thr Asn Asp Lys Gly
   980 985 990
   Ile Val Asn Tyr Asp Gln Asp Asn Asn Ala Pro Ile Ala Trp Thr Asn
   995 1000 1005
   Asp His Gly Asp Leu Ile Phe Thr Asn Gln Met Ile Asn Gly Gln
   1010 1015 1020
   Ser Asp Thr Ala Val Lys Gly Tyr Leu Asn Pro Glu Val Ala Gly
   1025 1030 1035
   Tyr Leu Ala Val Trp Val Pro Val Gly Ala Asn Asp Asn Gln Asp
   1040 1045 1050
   Ala Arg Thr Val Thr Thr Asn Gln Lys Asn Thr Asp Gly Lys Val
   1055 1060 1065
   Leu His Thr Asn Ala Ala Leu Asp Ser Lys Leu Met Tyr Glu Gly
   1070 1075 1080
   Phe Ser Asn Phe Gln Lys Met Pro Thr Arg Gly Asn Gln Tyr Ala
   1085 1090 1095
   Asn Val Ile Ile Ala Lys Asn Ile Asp Leu Phe Lys Ser Trp Gly
   1100 1105 1110
   Ile Thr Asp Phe Glu Leu Ala Pro Gln Tyr Arg Ser Ser Asp Gly
   1115 1120 1125
   Lys Asp Ile Thr Asp Arg Phe Leu Asp Ser Ile Val Gln Asn Gly
   1130 1135 1140
   Tyr Gly Leu Ser Asp Arg Tyr Asp Leu Gly Phe Lys Thr Pro Thr
   1145 1150 1155
   Lys Tyr Gly Thr Asp Gln Asp Leu Arg Lys Ala Ile Glu Arg Leu
   1160 1165 1170
   His Gln Ala Gly Met Ser Val Met Ala Asp Phe Val Ala Asn Gln
   1175 1180 1185
   Ile Tyr Gly Leu His Ala Asp Lys Glu Val Val Ser Ala Gln His
   1190 1195 1200
   Val Asn Ile Asn Gly Asp Thr Lys Leu Val Val Asp Pro Arg Tyr
   1205 1210 1215
   Gly Thr Gln Met Thr Val Val Asn Ser Val Gly Gly Gly Asp Tyr
   1220 1225 1230
   Gln Ala Lys Tyr Gly Gly Glu Tyr Leu Asp Thr Ile Ser Lys Leu
   1235 1240 1245
   Tyr Pro Gly Leu Leu Leu Asp Ser Asn Gly Gln Lys Ile Asp Leu
   1250 1255 1260
   Ser Thr Lys Ile Lys Glu Trp Ser Ala Lys Tyr Leu Asn Gly Ser
   1265 1270 1275
   Asn Ile Pro Gln Val Gly Met Gly Tyr Val Leu Lys Asp Trp Asn
   1280 1285 1290
   Asn Gly Gln Tyr Phe His Ile Leu Asp Lys Glu Gly Gln Tyr Ser
   1295 1300 1305
   Leu Pro Thr Gln Leu Val Ser Asn Asp Pro Glu Thr Gln Ile Gly
   1310 1315 1320
   Glu Ser Val Asn Tyr Lys Tyr Phe Ile Gly Asn Ser Asp Ala Thr
   1325 1330 1335
   Tyr Asn Met Tyr His Asn Leu Pro Asn Thr Val Ser Leu Ile Asn
   1340 1345 1350
   Ser Gln Glu Gly Gln Ile Lys Thr Gln Gln Ser Gly Val Thr Ser
   1355 1360 1365
   Asp Tyr Glu Gly Gln Gln Val Gln Val Thr Arg Gln Tyr Thr Asp
   1370 1375 1380
   Ser Lys Gly Val Ser Trp Asn Leu Ile Thr Phe Ala Gly Gly Asp
   1385 1390 1395
   Leu Gln Gly Gln Lys Leu Trp Val Asp Ser Arg Ala Leu Thr Met
   1400 1405 1410
   Thr Pro Phe Lys Thr Met Asn Gln Ile Ser Phe Ile Ser Tyr Ala
   1415 1420 1425
   Asn Arg Asn Asp Gly Leu Phe Leu Asn Ala Pro Tyr Gln Val Lys
   1430 1435 1440
   Gly Tyr Gln Leu Ala Gly Met Ser Asn Gln Tyr Lys Gly Gln Gln
   1445 1450 1455
   Val Thr Ile Ala Gly Val Ala Asn Val Ser Gly Lys Asp Trp Ser
   1460 1465 1470
   Leu Ile Ser Phe Asn Gly Thr Gln Tyr Trp Ile Asp Ser Gln Ala
   1475 1480 1485
   Leu Asn Thr Asn Phe Thr His Asp Met Asn Gln Lys Val Phe Val
   1490 1495 1500
   Asn Thr Thr Ser Asn Leu Asp Gly Leu Phe Leu Asn Ala Pro Tyr
   1505 1510 1515
   Arg Gln Pro Gly Tyr Lys Leu Ala Gly Leu Ala Lys Asn Tyr Asn
   1520 1525 1530
   Asn Gln Thr Val Thr Val Ser Gln Gln Tyr Phe Asp Asp Gln Gly
   1535 1540 1545
   Thr Val Trp Ser Gln Val Val Leu Gly Gly Gln Thr Val Trp Val
   1550 1555 1560
   Asp Asn His Ala Leu Ala Gln Met Gln Val Ser Asp Thr Ser Gln
   1565 1570 1575
   Gln Leu Tyr Val Asn Ser Asn Gly Arg Asn Asp Gly Leu Phe Leu
   1580 1585 1590
   Asn Ala Pro Tyr Arg Gly Gln Gly Ser Gln Leu Ile Gly Met Thr
   1595 1600 1605
   Ala Asp Tyr
   1610
<210> 15
   <211> 5322
   <212> DNA
   <213> Artificial Sequence
<220>
   <223> optimized Leuconostoc fallax AN optimized sequence
<400> 15
   atgaagcagc aagagagcat cactcgtaag aagctgtaca aggcgggcaa aagctgggta 60
   gtcgcagcaa ctctgttcgc tgcaactctg tttgctgcaa tgggtgctgc tggtgcaact 120
   actgttgcat ctgcagacgt acaaaaggat actgtagtgg taaccgcaga taagaacacc 180
   accgataagg acaaggagcc aatcaagacc gcaggtgcta acgtagtcga taagggtgta 240
   gcacaaacta ccgataccaa caccaccgac aaaaagacca tcgaggtcgg taaaagcgtc 300
   gatatgagcg caactgacaa gaaggtgacc gagactgtca agagcgtaga cactagcgct 360
   actgacaaga aaacgacgga ggcagttaag cctgtcgata ctaacgctac cgataagaag 420
   gctaccgagg ctgttaagcc tgtagatact aacgcaaccg ataagaaaac caccgaggca 480
   gtgaagcctg tcgacactaa cactacggac aagaaggtca ctgaggcaat caaaccggtc 540
   aacactaacg cagacgataa aaccgccgag cctgttaaga ctatcagcgc aactaaagac 600
   acggtcaaaa ccatcgcgaa caaacagaaa ggtgccacgg aggagcaagc agtcatcact 660
   gagggtcatt acgaggcaca aggtgacggt tttgtctaca tcactaaaga cggcaaacag 720
   ctgaccggtc tgcaaaacat caacggtaac acccagtact tcgatccggc aactggtcaa 780
   caactgaaag gcgatatcaa agccgtggct ggtactgtct actacttcga caaaaacagc 840
   ggcaacgcac gtgtctacca aaaagtcgcc gatggtactt acagcgagaa caacgaacac 900
   tggcaataca tcagcaaagt cgacaacaaa ccagtggaag gtctgtacaa cgtgcagggt 960
   aacctgcagt acttcgacat gagcaccggt aaccaggtca aaaacgacat ccgtagcgtg 1020
   gacggtgtga cttactactt tgacaaagac agcggtaacg gctccgcttt caacgcactg 1080
   agcgcaggtg aatacgttga gaaaaaagaa accgacgcac agggtaacca aaacagctac 1140
   tggacgtaca gcggtctgga tggtaaccct gttaagggtc tgtacgatat caacggttcc 1200
   ctgcaatact tcgacgagaa aaacggcgca cagctgaaag gtggtactgc aactgtgaac 1260
   ggtgtgacgt actacttcga acaggataaa ggcaacctga tcagcgtggt caacagcgtg 1320
   gaaagcggtc aatacaaaat cgacaacgac aacgtgtact acatcgacaa ccagggcaac 1380
   accctgaaag gtctgtacgc tatcaacggt cagctgaact atttcgacat gtccacgggt 1440
   gtgcaactga aaggtgcaag cgaaaacgct aacggtgtgg gttactattt cgataaagac 1500
   aaaggcaacg gccagtacca gtacagcctg atcacgtcca ccctggcaaa cgctttcagc 1560
   aaacacaacg cagcaaacga ttacacgcag agcagcttca ctcataccgt ggatggtttc 1620
   ctgactgctg atacttggta ccgtccaact gaaatcctga aaaacggcac cacctgggtg 1680
   gcatctacta gccaagatct gcgtccaatg atcactgtgt ggtggccaaa caaaaacgtg 1740
   caactgaact acctgaaact gatgcagacc gaaggtctgc tggattctgg tcaagtgtac 1800
   gacctgaact ctgaccaagc actgctgaac caggctgctc agactgttca ggtaaacatc 1860
   gaaaaacgta tcaccaaagc cggtaactcc gactggctga acgatctgct gtacaactct 1920
   cacggtgaaa ctccatcttt cgtgaaacag caggctatct ggaacgctga ctctgaatac 1980
   cacggtggtt ggttccaggg tggttatctg gcttaccgta actctgacct gactccgtat 2040
   gctaactctt cttaccgtca ttacacgggt atggaatttc tgctggccaa cgacgtggac 2100
   aactctaacc cgatcgtgca ggctgaagat ctgaactggc tgtattacct gatgaacttc 2160
   ggcactgaaa cgggtaacga cccgcaagct aatttcgact ctatccgtat cgacgctatc 2220
   tctttcgtgg acaaacaggt ggctaaaaaa gcgtacgaac tgctgcacga catgtacggt 2280
   ctgtctgctt ctgacgctgt ggctaacaaa cacgtgtcta tcgtggaagc ttctgctgac 2340
   cagactccgg ttactactga aaaccacgac gctctgatcg aatcttactg gcgtgacact 2400
   atgaaaaact ccctgtccaa agacgcgtct atcgactcct ctgctggttc tctgtctgct 2460
   atgatcaacg acggtaacgt ggaccgtgct aatgactcta ctactgaatc ctccatcttc 2520
   ccgaactaca ccatcgtgca tgctcatgac aaagacatcc aggacgctgt gtctaacgtg 2580
   atgaaaatcg tgaacaacga cccgtccatc tccctggacg gtttcactat ggaacagctg 2640
   gaaaaaggcc tgtctgcttt ctacgcggat cagcgttctg ctgtaaaaca gtacaaccag 2700
   tacaacatcc cgtccgcgta tgcggttatg ctgactaaca aagacaccgt gccgcgtact 2760
   ttctacggcg atatgtacca ggatgacggt cagtatatgg cgaacaaatc cctgtactac 2820
   gacgcgatcg ataccatgat gaaagcccgt ctgaaatacg tttccggtgg tcagaccatg 2880
   tctgttacga aaatcaacaa tgccaactcc cagaaatccg gcgaagttct gacctccgtt 2940
   cgtttcggta aaggcgttat ggacgcgacc gatgccggtt ctgcggaatc tcgtacccag 3000
   ggtattggtg ttgttgtatc taactcttct ggtctgcagc tgaatgacaa cgacaaaatc 3060
   gttctgcaca tgggtgccgc gcataaaaac caggaatacc gtgcgctgat gctgaccacg 3120
   aatgatggta ttaagtcttt caacaacgac gaagcgccga tcaactacac cgacgacaac 3180
   ggcgatctga ttttcgacgg tcataacatc gacggtcagg aaaacaccgc gattcgtggt 3240
   tacctgaacc cgcaggttgc cggttatctg gcggtttggg ttccgacggg tgccaaagat 3300
   gatcaggatg cgcgtaccca gccgtctaat gaaaaatcta ccgatggtaa agttctgcat 3360
   accaatgcgg ccctggattc tgaactgatc tatgaaggtt tttctaattt ccagccgatg 3420
   ccgaccacca aagatgaata taccaacgtt atgatcgcga aaaacattga cctgttcaaa 3480
   tcctggggca ttaccaactt cgaactggcg ccgcagtacc gttcttccga cggtaaaaac 3540
   attaacgacc gcttcattga ctccctggtg cagaacggtt acggtctgtc cgatcgttac 3600
   gacctgggtt ttgaaacccc gaccaaatac ggcaccgatc aggatctgcg taccgccatt 3660
   aagaccctgc accaggcggg catgaccgta atggccgatt atgttgcgaa tcagatctat 3720
   ggcctgaata cctctcagga agttgtagat gcccagcgtg taaattctga taataatgcg 3780
   gtagaagtac gttacggcca gcacctgaat gttgtaaact ctattggcgg tggcgaatat 3840
   cagaacctgt acggcggcaa atatctggaa attctgaaca aactgtaccc ggacctgctg 3900
   gtagacgaaa acggcaacaa gattgacatt gacaccaaaa tcaaacagtg gtccgcgaaa 3960
   tacctgaacg gctccaacgt gaccggcctg ggcatgggct atgttctgaa agattggtct 4020
   aacggccagt atttcaacat ctccaacacc gacggcaaag ttatgctgcc ggaacagctg 4080
   gtaaaacaca tgccggcggt tgaaatcggc acccagacca attataccgc gtatatttct      4140
   tccaccattc gtcgtgacgg cctgtataac aacatgccgt ggggcgttac ggcgaccggc      4200
   caggatggca atgaaattaa gtgggaacgt cagggctcta cctccgatta taatcaccag      4260
   aaagttcagg ttaatcgtca gtatgttgac aaacagggcg tagtttggaa cctgattaac      4320
   ttcgatgata aagatctgtg ggttgactcc aacgcgctgg tgacggtaaa cttcacctcc      4380
   cagaaaccga ccaaacactt cgtacagttc ggcatgcgtc agggcaaata cgatggcttt      4440
   tacctgagcg cgccgtacaa acagaccgaa tctaaatggg ttgcgtctac ccgtacccac      4500
   cagggccagc tgctggaagt tgttggccag tataccaccg gctccggcag ccgcaaagtt      4560
   acctggtatc tggttggcct ggatggcaaa caggtttggg ttgatagccg cgccgttggc      4620
   acgaatttta gccacaaaac caatattaat ctgctgatta attccgcgac ccgcaatgat      4680
   ggcatgtatc tgaatgcccc gtatggccag aaaggctaca aacgcgaaac cagctcccgc      4740
   ttttataatg aaaaactggt taccgtttcc cagcagtatt atgataacaa aggcgttatt      4800
   tggaatctga ttaccctgaa cggcaaaaaa ctgtgggttg attcccgcgc ctttgcgacg      4860
   gttattgata aaaaagttaa ccagtccctg tacattaaca gccgcaacga tggtatgtat      4920
   ctgaacgccc cgtatcgcgc gcagggcgcg aaacgctatg cgtccaccaa aacctatacc      4980
   ggccagcgcg tacaggtaac cctgcagcgc aaagataccc acggcgttac gtggtatctg      5040
   accaaagttg atagcaaaca gctgtgggta gattcccacg cgtttgcgcc gacgtttacc      5100
   cgcaacgtta gcctgaacgt taaagttaac tccagcaaac gcaacgatgg catctatctg      5160
   aacgcgccgt atggcaacaa aaaagcgaaa cgcattgcga gcaccaaagc gtataacggc      5220
   aaacgcgtta aagcctccaa agaatacaaa gatgcgaaag gcgtaacctg gtacctggtt      5280
   aacctgaaca acaaacaggt atggattgat aaacgtgcgt tt                         5322
<210> 16
   <211> 1774
   <212> PRT
   <213> Leuconostoc fallax
<400> 16
   Met Lys Gln Gln Glu Ser Ile Thr Arg Lys Lys Leu Tyr Lys Ala Gly
   1               5                   10                  15
   Lys Ser Trp Val Val Ala Ala Thr Leu Phe Ala Ala Thr Leu Phe Ala
               20                  25                  30
   Ala Met Gly Ala Ala Gly Ala Thr Thr Val Ala Ser Ala Asp Val Gln
           35                  40                  45
   Lys Asp Thr Val Val Val Thr Ala Asp Lys Asn Thr Thr Asp Lys Asp
       50                  55                  60
   Lys Glu Pro Ile Lys Thr Ala Gly Ala Asn Val Val Asp Lys Gly Val
   65                  70                  75                  80
   Ala Gln Thr Thr Asp Thr Asn Thr Thr Asp Lys Lys Thr Ile Glu Val
   85 90 95
   Gly Lys Ser Val Asp Met Ser Ala Thr Asp Lys Lys Val Thr Glu Thr
   100 105 110
   Val Lys Ser Val Asp Thr Ser Ala Thr Asp Lys Lys Thr Thr Glu Ala
   115 120 125
   Val Lys Pro Val Asp Thr Asn Ala Thr Asp Lys Lys Ala Thr Glu Ala
   130 135 140
   Val Lys Pro Val Asp Thr Asn Ala Thr Asp Lys Lys Thr Thr Glu Ala
   145 150 155 160
   Val Lys Pro Val Asp Thr Asn Thr Thr Asp Lys Lys Val Thr Glu Ala
   165 170 175
   Ile Lys Pro Val Asn Thr Asn Ala Asp Asp Lys Thr Ala Glu Pro Val
   180 185 190
   Lys Thr Ile Ser Ala Thr Lys Asp Thr Val Lys Thr Ile Ala Asn Lys
   195 200 205
   Gln Lys Gly Ala Thr Glu Glu Gln Ala Val Ile Thr Glu Gly His Tyr
   210 215 220
   Glu Ala Gln Gly Asp Gly Phe Val Tyr Ile Thr Lys Asp Gly Lys Gln
   225 230 235 240
   Leu Thr Gly Leu Gln Asn Ile Asn Gly Asn Thr Gln Tyr Phe Asp Pro
   245 250 255
   Ala Thr Gly Gln Gln Leu Lys Gly Asp Ile Lys Ala Val Ala Gly Thr
   260 265 270
   Val Tyr Tyr Phe Asp Lys Asn Ser Gly Asn Ala Arg Val Tyr Gln Lys
   275 280 285
   Val Ala Asp Gly Thr Tyr Ser Glu Asn Asn Glu His Trp Gln Tyr Ile
   290 295 300
   Ser Lys Val Asp Asn Lys Pro Val Glu Gly Leu Tyr Asn Val Gln Gly
   305 310 315 320
   Asn Leu Gln Tyr Phe Asp Met Ser Thr Gly Asn Gln Val Lys Asn Asp
   325 330 335
   Ile Arg Ser Val Asp Gly Val Thr Tyr Tyr Phe Asp Lys Asp Ser Gly
   340 345 350
   Asn Gly Ser Ala Phe Asn Ala Leu Ser Ala Gly Glu Tyr Val Glu Lys
           355                 360                 365
   Lys Glu Thr Asp Ala Gln Gly Asn Gln Asn Ser Tyr Trp Thr Tyr Ser
       370                 375                 380
   Gly Leu Asp Gly Asn Pro Val Lys Gly Leu Tyr Asp Ile Asn Gly Ser
   385                 390                 395                 400
   Leu Gln Tyr Phe Asp Glu Lys Asn Gly Ala Gln Leu Lys Gly Gly Thr
                   405                 410                 415
   Ala Thr Val Asn Gly Val Thr Tyr Tyr Phe Glu Gln Asp Lys Gly Asn
               420                 425                 430
   Leu Ile Ser Val Val Asn Ser Val Glu Ser Gly Gln Tyr Lys Ile Asp
           435                 440                 445
   Asn Asp Asn Val Tyr Tyr Ile Asp Asn Gln Gly Asn Thr Leu Lys Gly
       450                 455                 460
   Leu Tyr Ala Ile Asn Gly Gln Leu Asn Tyr Phe Asp Met Ser Thr Gly
   465                 470                 475                 480
   Val Gln Leu Lys Gly Ala Ser Glu Asn Ala Asn Gly Val Gly Tyr Tyr
                   485                 490                 495
   Phe Asp Lys Asp Lys Gly Asn Gly Gln Tyr Gln Tyr Ser Leu Ile Thr
               500                 505                 510
   Ser Thr Leu Ala Asn Ala Phe Ser Lys His Asn Ala Ala Asn Asp Tyr
           515                 520                 525
   Thr Gln Ser Ser Phe Thr His Thr Val Asp Gly Phe Leu Thr Ala Asp
       530                 535                 540
   Thr Trp Tyr Arg Pro Thr Glu Ile Leu Lys Asn Gly Thr Thr Trp Val
   545                 550                 555                 560
   Ala Ser Thr Ser Gln Asp Leu Arg Pro Met Ile Thr Val Trp Trp Pro
                   565                 570                 575
   Asn Lys Asn Val Gln Leu Asn Tyr Leu Lys Leu Met Gln Thr Glu Gly
               580                 585                 590
   Leu Leu Asp Ser Gly Gln Val Tyr Asp Leu Asn Ser Asp Gln Ala Leu
           595                 600                 605
   Leu Asn Gln Ala Ala Gln Thr Val Gln Val Asn Ile Glu Lys Arg Ile
       610                 615                 620
   Thr Lys Ala Gly Asn Ser Asp Trp Leu Asn Asp Leu Leu Tyr Asn Ser
   625                 630                 635                 640
   His Gly Glu Thr Pro Ser Phe Val Lys Gln Gln Ala Ile Trp Asn Ala
                   645                 650                 655
   Asp Ser Glu Tyr His Gly Gly Trp Phe Gln Gly Gly Tyr Leu Ala Tyr
               660                 665                 670
   Arg Asn Ser Asp Leu Thr Pro Tyr Ala Asn Ser Ser Tyr Arg His Tyr
           675                 680                 685
   Thr Gly Met Glu Phe Leu Leu Ala Asn Asp Val Asp Asn Ser Asn Pro
       690                 695                 700
   Ile Val Gln Ala Glu Asp Leu Asn Trp Leu Tyr Tyr Leu Met Asn Phe
   705                 710                 715                 720
   Gly Thr Glu Thr Gly Asn Asp Pro Gln Ala Asn Phe Asp Ser Ile Arg
                   725                 730                 735
   Ile Asp Ala Ile Ser Phe Val Asp Lys Gln Val Ala Lys Lys Ala Tyr
               740                 745                 750
   Glu Leu Leu His Asp Met Tyr Gly Leu Ser Ala Ser Asp Ala Val Ala
           755                 760                 765
   Asn Lys His Val Ser Ile Val Glu Ala Ser Ala Asp Gln Thr Pro Val
       770                 775                 780
   Thr Thr Glu Asn His Asp Ala Leu Ile Glu Ser Tyr Trp Arg Asp Thr
   785                 790                 795                 800
   Met Lys Asn Ser Leu Ser Lys Asp Ala Ser Ile Asp Ser Ser Ala Gly
                   805                 810                 815
   Ser Leu Ser Ala Met Ile Asn Asp Gly Asn Val Asp Arg Ala Asn Asp
               820                 825                 830
   Ser Thr Thr Glu Ser Ser Ile Phe Pro Asn Tyr Thr Ile Val His Ala
           835                 840                 845
   His Asp Lys Asp Ile Gln Asp Ala Val Ser Asn Val Met Lys Ile Val
       850                 855                 860
   Asn Asn Asp Pro Ser Ile Ser Leu Asp Gly Phe Thr Met Glu Gln Leu
   865                 870                 875                 880
   Glu Lys Gly Leu Ser Ala Phe Tyr Ala Asp Gln Arg Ser Ala Val Lys
                   885                 890                 895
   Gln Tyr Asn Gln Tyr Asn Ile Pro Ser Ala Tyr Ala Val Met Leu Thr
   900 905 910
   Asn Lys Asp Thr Val Pro Arg Thr Phe Tyr Gly Asp Met Tyr Gln Asp
   915 920 925
   Asp Gly Gln Tyr Met Ala Asn Lys Ser Leu Tyr Tyr Asp Ala Ile Asp
   930 935 940
   Thr Met Met Lys Ala Arg Leu Lys Tyr Val Ser Gly Gly Gln Thr Met
   945 950 955 960
   Ser Val Thr Lys Ile Asn Asn Ala Asn Ser Gln Lys Ser Gly Glu Val
   965 970 975
   Leu Thr Ser Val Arg Phe Gly Lys Gly Val Met Asp Ala Thr Asp Ala
   980 985 990
   Gly Ser Ala Glu Ser Arg Thr Gln Gly Ile Gly Val Val Val Ser Asn
   995 1000 1005
   Ser Ser Gly Leu Gln Leu Asn Asp Asn Asp Lys Ile Val Leu His
   1010 1015 1020
   Met Gly Ala Ala His Lys Asn Gln Glu Tyr Arg Ala Leu Met Leu
   1025 1030 1035
   Thr Thr Asn Asp Gly Ile Lys Ser Phe Asn Asn Asp Glu Ala Pro
   1040 1045 1050
   Ile Asn Tyr Thr Asp Asp Asn Gly Asp Leu Ile Phe Asp Gly His
   1055 1060 1065
   Asn Ile Asp Gly Gln Glu Asn Thr Ala Ile Arg Gly Tyr Leu Asn
   1070 1075 1080
   Pro Gln Val Ala Gly Tyr Leu Ala Val Trp Val Pro Thr Gly Ala
   1085 1090 1095
   Lys Asp Asp Gln Asp Ala Arg Thr Gln Pro Ser Asn Glu Lys Ser
   1100 1105 1110
   Thr Asp Gly Lys Val Leu His Thr Asn Ala Ala Leu Asp Ser Glu
   1115 1120 1125
   Leu Ile Tyr Glu Gly Phe Ser Asn Phe Gln Pro Met Pro Thr Thr
   1130 1135 1140
   Lys Asp Glu Tyr Thr Asn Val Met Ile Ala Lys Asn Ile Asp Leu
   1145 1150 1155
   Phe Lys Ser Trp Gly Ile Thr Asn Phe Glu Leu Ala Pro Gln Tyr
   1160 1165 1170
   Arg Ser Ser Asp Gly Lys Asn Ile Asn Asp Arg Phe Ile Asp Ser
   1175 1180 1185
   Leu Val Gln Asn Gly Tyr Gly Leu Ser Asp Arg Tyr Asp Leu Gly
   1190 1195 1200
   Phe Glu Thr Pro Thr Lys Tyr Gly Thr Asp Gln Asp Leu Arg Thr
   1205 1210 1215
   Ala Ile Lys Thr Leu His Gln Ala Gly Met Thr Val Met Ala Asp
   1220 1225 1230
   Tyr Val Ala Asn Gln Ile Tyr Gly Leu Asn Thr Ser Gln Glu Val
   1235 1240 1245
   Val Asp Ala Gln Arg Val Asn Ser Asp Asn Asn Ala Val Glu Val
   1250 1255 1260
   Arg Tyr Gly Gln His Leu Asn Val Val Asn Ser Ile Gly Gly Gly
   1265 1270 1275
   Glu Tyr Gln Asn Leu Tyr Gly Gly Lys Tyr Leu Glu Ile Leu Asn
   1280 1285 1290
   Lys Leu Tyr Pro Asp Leu Leu Val Asp Glu Asn Gly Asn Lys Ile
   1295 1300 1305
   Asp Ile Asp Thr Lys Ile Lys Gln Trp Ser Ala Lys Tyr Leu Asn
   1310 1315 1320
   Gly Ser Asn Val Thr Gly Leu Gly Met Gly Tyr Val Leu Lys Asp
   1325 1330 1335
   Trp Ser Asn Gly Gln Tyr Phe Asn Ile Ser Asn Thr Asp Gly Lys
   1340 1345 1350
   Val Met Leu Pro Glu Gln Leu Val Lys His Met Pro Ala Val Glu
   1355 1360 1365
   Ile Gly Thr Gln Thr Asn Tyr Thr Ala Tyr Ile Ser Ser Thr Ile
   1370 1375 1380
   Arg Arg Asp Gly Leu Tyr Asn Asn Met Pro Trp Gly Val Thr Ala
   1385 1390 1395
   Thr Gly Gln Asp Gly Asn Glu Ile Lys Trp Glu Arg Gln Gly Ser
   1400 1405 1410
   Thr Ser Asp Tyr Asn His Gln Lys Val Gln Val Asn Arg Gln Tyr
   1415 1420 1425
   Val Asp Lys Gln Gly Val Val Trp Asn Leu Ile Asn Phe Asp Asp
   1430 1435 1440
   Lys Asp Leu Trp Val Asp Ser Asn Ala Leu Val Thr Val Asn Phe
   1445 1450 1455
   Thr Ser Gln Lys Pro Thr Lys His Phe Val Gln Phe Gly Met Arg
   1460 1465 1470
   Gln Gly Lys Tyr Asp Gly Phe Tyr Leu Ser Ala Pro Tyr Lys Gln
   1475 1480 1485
   Thr Glu Ser Lys Trp Val Ala Ser Thr Arg Thr His Gln Gly Gln
   1490 1495 1500
   Leu Leu Glu Val Val Gly Gln Tyr Thr Thr Gly Ser Gly Ser Arg
   1505 1510 1515
   Lys Val Thr Trp Tyr Leu Val Gly Leu Asp Gly Lys Gln Val Trp
   1520 1525 1530
   Val Asp Ser Arg Ala Val Gly Thr Asn Phe Ser His Lys Thr Asn
   1535 1540 1545
   Ile Asn Leu Leu Ile Asn Ser Ala Thr Arg Asn Asp Gly Met Tyr
   1550 1555 1560
   Leu Asn Ala Pro Tyr Gly Gln Lys Gly Tyr Lys Arg Glu Thr Ser
   1565 1570 1575
   Ser Arg Phe Tyr Asn Glu Lys Leu Val Thr Val Ser Gln Gln Tyr
   1580 1585 1590
   Tyr Asp Asn Lys Gly Val Ile Trp Asn Leu Ile Thr Leu Asn Gly
   1595 1600 1605
   Lys Lys Leu Trp Val Asp Ser Arg Ala Phe Ala Thr Val Ile Asp
   1610 1615 1620
   Lys Lys Val Asn Gln Ser Leu Tyr Ile Asn Ser Arg Asn Asp Gly
   1625 1630 1635
   Met Tyr Leu Asn Ala Pro Tyr Arg Ala Gln Gly Ala Lys Arg Tyr
   1640 1645 1650
   Ala Ser Thr Lys Thr Tyr Thr Gly Gln Arg Val Gln Val Thr Leu
   1655 1660 1665
   Gln Arg Lys Asp Thr His Gly Val Thr Trp Tyr Leu Thr Lys Val
   1670 1675 1680
   Asp Ser Lys Gln Leu Trp Val Asp Ser His Ala Phe Ala Pro Thr
   1685 1690 1695
   Phe Thr Arg Asn Val Ser Leu Asn Val Lys Val Asn Ser Ser Lys
   1700 1705 1710
   Arg Asn Asp Gly Ile Tyr Leu Asn Ala Pro Tyr Gly Asn Lys Lys
   1715 1720 1725
   Ala Lys Arg Ile Ala Ser Thr Lys Ala Tyr Asn Gly Lys Arg Val
   1730 1735 1740
   Lys Ala Ser Lys Glu Tyr Lys Asp Ala Lys Gly Val Thr Trp Tyr
   1745 1750 1755
   Leu Val Asn Leu Asn Asn Lys Gln Val Trp Ile Asp Lys Arg Ala
   1760 1765 1770
   Phe
<210> 17
   <211> 7
   <212> PRT
   <213> Artificial sequence
<220>
   <223> enzymatic domain
<400> 17
   Ala Asp Phe Val Ala Asn Gln
   1 5
<210> 18
   <211> 11
   <212> PRT
   <213> Artificial Sequence
<220>
   <223> Enzymatic domain
<400> 18
   Ser Met Arg Ile Asp Ala Ile Ser Phe Val Asp
   1 5 10
<210> 19
   <211> 11
   <212> PRT
   <213> Artificial Sequence
<220>
   <223> Enzymatic domain
<400> 19
   His Ile Ser Ile Val Glu Ala Pro Lys Gly Glu
   1 5 10
<210> 20
   <211> 15
   <212> PRT
   <213> Artificial sequence
<220>
   <223> Enzymatic domain
<400> 20
   Ile Val His Ala His Asp Lys Asp Ile Gln Asp Thr Val Ile His
   1 5 10 15
<210> 21
   <211> 7
   <212> PRT
   <213> Artificial Sequence
<220>
   <223> Enzymatic domain
<400> 21
   Ala Asp Tyr Val Ala Asn Gln
   1 5
<210> 22
   <211> 11
   <212> PRT
   <213> Artificial sequence
<220>
   <223> Enzymatic domain
<400> 22
   Ser Ile Arg Ile Asp Ala Ile Ser Phe Val Asp
   1 5 10
<210> 23
   <211> 11
   <212> PRT
   <213> Artificial sequence
<220>
   <223> Enzymatic domain
<400> 23
   His Val Ser Ile Val Glu Ala Ser Ala Asp Gln
   1 5 10
<210> 24
   <211> 15
   <212> PRT
   <213> Artificial sequence
<220>
   <223> Enzymatic domain
<400> 24
   Ile Val His Ala His Asp Lys Asp Ile Gln Asp Ala Val Ser Asn
   1               5                   10                  15

## Description

### DOMAINE DE L'INVENTION

La présente invention concerne un polypeptide isolé ayant la capacité de former uniquement des branchements d'unités glucosyle en alpha-1,3 sur un accepteur, un polynucléotide codant ledit polypeptide et son utilisation dans un procédé de production d'accepteurs branchés d'unités glucosyle en alpha-1,3.

### ART ANTERIEUR

Les glucosyltransférases sont des enzymes capables de catalyser la synthèse de polymères de glucose à partir d'un substrat peu coûteux, notamment le saccharose, seul ou en présence d'un accepteur d'unités glucosyle comprenant au moins un groupement hydroxyle. Au sein de ces molécules acceptrices, les unités glucosyle sont couplées par des liaisons osidiques de nature variable (α-1,6, α-1,4, α-1,2 ou α-1,3).

Les transglucosylases (ou glucane-saccharases) appartenant à la famille 70 des Glycoside-Hydrolases (base de données : Carbohydrate Active Enzymes database (http://www.cazy.org/) et CANTAREL et al., Nucleic Acids Res, vol. 37, p:D233-238 2009) et constituent des enzymes naturellement produites par les bactéries lactiques des genres *Leuconostoc, Lactobacillus, Streptococcus* ou *Weissela.* A partir de leur substrat, notamment de saccharose, substrat renouvelable et bon marché, ces enzymes catalysent la synthèse d'homopolymères d'unités glucosyle (glucanes) généralement de très haute masse molaire et présentant des structures variées (liaisons osidiques α-1,6/ α-1,4/ α-1,2 et/ou α-1,3). De plus, si des molécules hydroxylées sont rajoutées au milieu réactionnel en plus du donneur d'unités glucosyle, ces enzymes peuvent également glucosyler ces molécules au détriment de la synthèse de polymère, donnant lieu à une large gamme d'oligosaccharides et/ou de gluco-conjugués. La structure tridimensionnelle des glucane-saccharases, leur mécanisme catalytique et leur spécificité de substrat ont été décrites par HANS LEEHMUIS et al. (Journal of Biotechnology, vol. 163, p : 250-272, 2013).

Des travaux décrits dans JEANES *et al.* (1954), décrivant la purification et la caractérisation de glucanes produits par 96 souches de *Leuconostoc* sp., on connait ceux produit par la souche *Leuconostoc mesenteroides* NRRL B-742 (également retrouvée sous *L. mesenteroides* ATCC 13146, et depuis reclassée en *L. citreum* NRRL B-742). En effet, celle-ci produit deux types de glucanes : la fraction S et la fraction L.

Le premier glucane est composé à 50% de liaisons α-1,6 dans sa chaine principale et à 50% de branchements en α-1,3 (fraction S). Les études successives de ce dernier glucane décrites dans JEANES & SEYMOUR (Carbohydrate Research, vol.74, p :31-40), COTE & ROBYT (Carbohydrate Research, vol.119, p:141-156, 1983) et REMAUD et al. (J. Carbohydrate Chemistry, vol.11(3), 1992) montrèrent que celui-ci présentait une structure originale en forme de peigne. Plus spécifiquement, chaque unité glucosyle de la chaine linéaire au sein de cette structure se trouvait ramifiée en a -1,3 par une seule unité glucosyle.

Le second glucane (fraction L) produit par cette même souche se trouvait lui composé à 73% de liaisons α-1,6 et à 14% de liaisons α-1,4 aux points de ramifications (SEYMOUR et al., Carbohydrate Research, vol. 74, p :41-62, 1979).

Ces différents travaux ont permis de montrer que la souche de *L. citreum* NRRL B-742 possède plusieurs gènes codant pour des transglucosylases responsables de la synthèse de glucanes ; lesquelles présentent des spécificités différentes. En présence d'extraits de cette souche, il a été ainsi possible de produire des gluco-oligosaccharides qui se sont révélés avoir de fortes propriétés prébiotiques stimulant la croissance de *Bifidobacterium* sp. et de *Lactobacillus* sp. par exemple (CHUNG & DAY, Journal of Industrial Microbiology & Biotechnology, vol.29, p:196-199, 2002; CHUNG & DAY, Poultry Science, vol.83, p : 1302-1306, 2004 ; brevet US 7,772,212).

Maintenant, et bien que cette souche soit connue depuis plus de cinquante ans, les enzymes responsables de la synthèse de ces glucanes ne sont toujours pas connues. Il faut en effet comprendre que si certaines de ces enzymes sont extracellulaires ; d'autres, au contraire, restent fortement associées aux cellules, notamment l'enzyme responsable des glucanes à forte teneur en liaisons α-1,3 (REMAUD et al., 1992). De cette forte association aux cellules bactériennes, il en résulte que sa purification et sa caractérisation fine n'ont pu être effectuées.

En 2000, KIM et al. (J. Microbiol. Biotechnol., vol. 10(4), p: 559-563, 2000) décrivirent le clonage chez *E. coli* d'une transglucosylase issue de cette souche, appelée DsrB-742. La caractérisation du gène en question montrait que celui-ci présentait 95% de similarité avec celui de la DSR-B déjà caractérisée chez *L citreum* NRRL B-1299 et présentait une activité de polymerisation des unités glucosyle en α-1,6, mais aucune activité spécifique de branchement en α-1,3. Des variants de cette transglucosylase ont été obtenus par KHANG HEE-KYOUNG et al. (Journal of Molecular Catalysis B: Enzymatic, vol. 26, p: 167-176, 2003).

Finalement, l'identification et la caractérisation biochimique de l'enzyme responsable de la synthèse des glucanes (S) de type peigne composés à 50% de branchements en α-1,3 restait donc totalement infructueuse jusqu'à présent.

### DESCRIPTION DES DESSINS

La figure 1 montre la structure primaire de la protéine de référence α-1,3 BrS issue de la souche *L. citreum* NRRL B-742.
La figure 2 montre des profils d'analyses chromatographiques par HPAEC-PAD.
La figure 3 montre un profil RMN.
La figure 4 montre des profils d'analyses chromatographiques par HPAEC-PAD.
Les figures 5 et 6 montrent l'évolution du taux de liaisons α-1,3 en fonction du rapport saccharose/accepteur hydroxylé pour l'enzyme entière et l'enzyme tronquée respectivement.
Les figures 7 et 8 montrent un spectre RMN.

### SOMMAIRE DE L'INVENTION

Les inventeurs ont maintenant mis en évidence que la synthèse des dextranes en peigne par la souche *L. citreum* NRRL B-742 était due à l'action non d'une mais de deux transglucosylases distinctes : une responsable de la synthèse d'un dextrane linéaire, puis l'autre responsable des branchements en α-1,3 sur ces chaines linéaires qui jouent le rôle de molécules acceptrices. Les inventeurs ont donc mis en évidence un polypeptide présentant une activité enzymatique encore jamais décrite, laquelle est responsable de branchements spécifiques d'unités glucosyle en α-1,3 sur des molécules acceptrices, par exemple de type dextranes. A noter en outre que les inventeurs ont été capables de contrôler le taux de branchement de ces unités glycosyles. Enfin, les inventeurs ont été capables d'identifier deux séquences orthologues de cette protéine dans deux autres souches de *Leuconostoc.*

Il s'agit donc de la première enzyme de branchement naturelle décrite pour une transglucosylase qui par analyse de séquence se classe dans la famille GH-70. Par ailleurs, la synthèse de polysaccharides branchés avec des taux contrôlés d'unités glucosyle liées en alpha-1,3 n'a jamais été décrite, et aucun produit branché de ce type n'existait sur le marché jusqu'alors.

Ce type de liaisons en alpha-1,3 confère une résistance à l'action d'enzymes de dégradation telles que des glycoside-hydrolases comme par exemple les dextranases, glucoamylases, amylases et en particulier les enzymes digestives de l'appareil intestinal humain augmentant ainsi la durée de vie de la molécule acceptrice à laquelle elles sont associées, et attribuant aux gluco-oligosaccharides de nouvelles propriétés physicochimiques et/ou prébiotiques intéressantes sur le plan des applications industrielles.

Les prébiotiques sont des ingrédients alimentaires non digestibles qui arrivent intacts dans le colon où ils sont alors spécifiquement métabolisés par une certaine catégorie du microbiote intestinal (humain ou animal) dite « bénéfique ». Comparativement aux probiotiques, les prébiotiques prennent l'avantage sur les probiotiques sur le marché des alicaments, notamment grâce à une meilleure résistance à la barrière digestive, des coûts de production potentiellement moins chers, et une plus grande facilité à être incorporés aux préparations alimentaires.

Outre leur action sur les flores intestinales, les molécules dites prébiotiques peuvent également être métabolisées par d'autres flores commensales, telles que les flores cutanées ou vaginales, et participer au développement d'une flore dite « bénéfique » selon les mêmes principes que ceux cités ci-dessus.

Parmi ces prébiotiques, on classe -d'un point de vue commercial- sous le nom d'isomaltooligosaccharides tous les oligosaccharides de glucose composés majoritairement de liaisons a -1,6 dans la chaine principale et de taux variables en liaisons α-1,4 ; α-1,3 et/ou α-1,2. On les trouve naturellement dans divers produits fermentés tels que le miso, sake, la sauce soja ou le miel. Ils peuvent être produits industriellement par des hydrolysats d'amidon à l'aide d'a-transglucosylases. Dans ce cas, les IMOS contiennent exclusivement des liaisons α-1,6 et α-1,4. Or, ces produits peuvent également être synthétisés par réaction d'accepteur à l'aide des transglucosylases de la famille GH-70. Dans ce cas, les produits peuvent aussi contenir des liaisons α-1,3 ou α-1,2 en plus des α-1,6 et α-1,4, en fonction de la spécificité de liaison de la glucane-saccharase employée. Ces liaisons (α-1,3 ou α-1,2) sont plus rares dans la nature, et confèrent aux molécules des propriétés prébiotiques particulièrement intéressantes, car encore plus difficilement digestibles que les autres IMOS (notamment par certains pathogènes qui peuvent partiellement reconnaitre les α-1,6/α-1,4 IMOS).

La découverte faite par les inventeurs permet d'envisager la synthèse d'une large variété de nouveaux polysaccharides présentant des structures et des propriétés nouvelles et contrôlées.

Ainsi, les inventeurs ont démontré que le produit obtenu suivant la réaction de leur enzyme en présence de saccharose et d'un dextrane linéaire d'un poids moléculaire de 1500 Da permet d'obtenir un polysaccharide présentant une résistance améliorée à l'action d'enzymes digestives. Les propriétés de ce nouveau polysaccharide en tant que prébiotique sont donc susceptibles d'être comparables à celles des isomaltooligosaccharides ou des glucooligosaccharides branchés en α-1,2.

Finalement, de tels nouveaux polysaccharides pourraient trouver une utilisation en tant que prébiotiques ou en tant que biopolymères, dans la préparation de formulations industrielles. Parmi les biopolymères, les polysaccharides (surtout d'origine végétale, mais également de plus en plus d'origine microbienne) peuvent être utilisés comme agents texturants ou stabilisants pour divers types de produits industriels. Un exemple d'utilisation de ces biopolymères comprend la préparation de bioplastiques facilement dégradables. Ils pourraient alors remplacer l'usage de polymères d'origine synthétique.

### DESCRIPTION DETAILLEE DE L'INVENTION

Un premier objet de l'invention concerne un polypeptide isolé ayant la capacité de former uniquement des branchements d'unités glucosyle en alpha-1,3 sur un accepteur comprenant au moins un groupement hydroxyle et caractérisé en ce que ledit polypeptide comprend :
1) Le motif I de séquence SEQ ID NO: 1
2) Le motif II de séquence SEQ ID NO: 2
3) Le motif III de séquence SEQ ID NO: 3
4) Le motif IV de séquence SEQ ID NO: 4
ou des dérivés d'un ou plusieurs desdits domaines présentant au moins 80% d'identité avec ceux-ci ; lequel polypeptide présente en outre le résidu aspartique (D) en position 5 du motif II (SEQ ID NO: 2), le résidu glutamique (E) en position 6 du motif III (SEQ ID NO: 3) et le résidu aspartique (D) en position 6 du motif IV (SEQ ID NO: 4).

Ces trois derniers acides aminés peuvent être aisément identifiés par l'homme du métier, au regard des homologies de séquences entre les enzymes présentant des activités analogues, comme représentant la « triade catalytique » essentielle pour l'activité transglucosylase des enzymes de la famille GH 70 (LEEMHUIS et al,, Journal of Biotechnology, vol.163(2), p: 250-72, 2013).

Dans le polypeptide décrit précédemment, la séquence SEQ ID NO: 1 est elle que ADX₁VANQ avec X₁ correspond à F ou Y ; la séquence SEQ ID NO: 2 est elle que SX₂RIDAISFVD avec X₂ correspond à M ou I ; la séquence SEQ ID NO: 3 est elle que HX₃SIVEAX₄X₅X₆X₇ avec X₃ correspond à V ou I, X₄ correspond à P ou S, X₅ correspond à K ou A, X₆ correspond à G ou D, X₇ correspond à E ou Q ; la séquence SEQ ID NO: 4 est elle que IVHAHDKDIQDX₈VX₉X₁₀ avec X₈ correspond à T ou A, X₉ correspond à S ou I et X₁₀ correspond à H ou N.

De préférence, le motif I présente la séquence SEQ ID NO: 17 (ADFVANQ), le motif II présente la séquence SEQ ID NO: 18 (SMRIDAISFVD), le motif III présente la séquence SEQ ID NO: 19 (HISIVEAPKGE) et le motif IV présente la séquence SEQ ID NO: 20 (IVHAHDKDIQDTVIH).

De manière préférée, un polypeptide selon l'invention est un polypeptide comprenant la séquence SEQ ID NO: 5 (positions 636 à 1270 de la séquence SEQ ID NO: 9, domaines A et C + une partie du domaine B) un orthologue, un dérivé, ou un fragment de celle-ci, de préférence comprenant la séquence SEQ ID NO: 6 (positions 586 à 1284 de la séquence SEQ ID NO: 9, comprenant l'intégralité des domaines A, B et C), un polypeptide dont la séquence présente un pourcentage d'identité d'au moins 80% avec la SEQ ID NO : 6, ou un fragment de celle-ci, ledit fragment présentant une séquence d'au moins 150 acides aminés.

Avantageusement encore, un polypeptide selon l'invention est un polypeptide comprenant la séquence SEQ ID NO: 7 (positions 446 à 1356 de la séquence SEQ ID NO: 9, domaines A, B, C et IV) un orthologue, un dérivé, ou un fragment de celle-ci, de préférence comprenant la séquence SEQ ID NO: 8 (positions 403 à 1606 de la séquence SEQ ID NO: 9, domaines A, B, C, IV et V selon homologie avec la transglucosylase GTF-180 de *L. reuteri* 180), un orthologue, un dérivé, ou un fragment de celle-ci.

Un tel polypeptide peut notamment être le polypeptide tronqué à son extrémité C-terminale (position 1313) de séquence SEQ ID NO: 12 (domaines A, B et C complets).

Finalement, un polypeptide selon l'invention est un polypeptide comprenant ou consistant en la séquence SEQ ID NO: 9 (séquence entière de l'enzyme), un polypeptide dont la séquence présente un pourcentage d'identité d'au moins 80% avec la séquence SEQ ID NO: 9, ou un fragment de celle-ci, ledit fragment présentant une séquence d'au moins 150 acides aminés.

La séquence SEQ ID NO: 9 correspond à un polypeptide selon l'invention isolé de la souche *Leuconostoc citreum* NRRL B-742 (ATCC 13146), antérieurement appelé *Leuconostoc mesenteroides* NRRL B-742.

Par « orthologue », on entend un polypeptide présentant la même activité que celle du polypeptide de séquence SEQ ID NO: 9 de la souche *Leuconostoc citreum* NRRL742 ; lequel polypeptide présente une séquence en acides aminés qui diffère d'au moins un résidu avec la séquence SEQ ID NO: 9 et a été isolé à partir d'une souche autre que celle énoncée précédemment. Plus généralement, on entend par orthologue, un polypeptide présentant la même activité que celle du polypeptide de séquence SEQ ID NO: 9 isolé à partir d'une souche bactérienne donnée qui descend de la même séquence unique que le polypeptide de séquence SEQ ID NO: 9 isolé à partir de la souche *Leuconostoc citreum* NRRL B-742, laquelle séquence unique est issue du dernier ancêtre commun à ces deux souches.

A titre de tels orthologues, on peut citer les séquences SEQ ID NO: 14 et SEQ ID NO: 16.

Avantageusement, cet orthologue a été isolé à partir d'une souche bactérienne appartenant à la famille Leuconostocaceae, laquelle comprend les genres *Leuconostoc, Oenococcus* et *Weissela.* Maintenant, cet orthologue est préférentiellement isolé à partir d'une souche bactérienne appartenant au genre *Leuconostoc,* voire appartenant au groupe consistant en les espèces *Leuconostoc argentinum, Leuconostoc carnosum, Leuconostoc citreum, Leuconostoc cremoris, Leuconostoc dextranicum, Leuconostoc fallax, Leuconostoc fructosum, Leuconostoc gasicomitatum, Leuconostoc gelidum, Leuconostoc inhae, Leuconostoc kimchii, Leuconostoc lactis, Leuconostoc mesenteroides, Leuconostoc paramesenteroides* et *Leuconostoc pseudomesenteroides.*

Plus simplement, un tel orthologue devra présenter une identité de séquence d'au moins 50 ou 60% avec la séquence de référence notamment SEQ ID NO: 9, de préférence d'au moins 65%, 70%, 75%, 80% ou 85%, et de manière encore plus préférée d'au moins 90%, 95%, 97%, 98% ou 99% avec la séquence de référence.

Par « dérivé », on fera référence à un motif ou un polypeptide dont la séquence présente un pourcentage d'identité d'au moins 80 %, par exemple d'au moins 85%, de préférence d'au moins 90%, et de manière particulièrement préférée d'au moins 95% avec la séquence de référence, à savoir un motif spécifique (I, II, III ou IV) ou un polypeptide selon l'invention, de préférence avec un polypeptide de séquence SEQ ID NO: 9.

Naturellement, un tel dérivé du polypeptide selon l'invention présentera l'activité enzymatique décrite précédemment.

Par "pourcentage d'identité entre deux séquences polypeptidiques", on entend le pourcentage d'acides aminés identiques, entre deux séquences devant être comparées, obtenu avec le meilleur alignement possible desdites séquences. Ce pourcentage est purement statistique et les différences entre les deux séquences sont réparties aléatoirement sur toute la longueur des séquences d'acides aminés.

Par "meilleur alignement possible ou alignement optimal", on entend l'alignement permettant d'obtenir le pourcentage d'identité le plus élevé. Les comparaisons de séquences entre deux séquences d'acides aminés sont habituellement réalisées en comparant lesdites séquences après que celles-ci aient été alignées selon le meilleur alignement possible ; la comparaison est alors réalisée sur des segments de comparaison de façon à identifier et comparer des régions de similarité. Le meilleur alignement possible pour effectuer une comparaison peut être réalisé en utilisant l'algorithme d'alignement local développé par SMITH & WATERMAN (Ad. App. Math., vol.2, p:482, 1981), en utilisant l'algorithme d'alignement global développé par NEDDLEMAN & WUNSCH (J. Mol. Biol., vol.48, p:443, 1970), en utilisant la méthode de similarité développé par PEARSON & LIPMAN (Proc. Natl. Acd. Sci. USA, vol.85, p:2444, 1988), en utilisant des programmes d'ordinateur basés sur de tels algorithmes (GAP, BESTFIT, BLAST P, BLAST N, FASTA, TFASTA, Genetics Computer Group, 575 Science Dr., Madison, WI USA), en utilisant les algorithmes d'alignements multiples MUSCLE (Edgar, Robert C., Nucleic Acids Research, vol. 32, p: 1792, 2004) ou CLUSTAL (Goujon M, McWilliam H, Li W, Valentin F, Squizzato S, Paern J, Lopez R. Nucleic acids research 2010 Jul, 38 Suppl: W695-9). Pour obtenir le meilleur alignement possible, on utilisera de préférence le programme BLAST avec la matrice BLOSUM 62 ou la matrice PAM 30. Le pourcentage d'identité est déterminé en comparant les deux séquences alignées de façon optimale, lesdites séquences pourront comprendre des additions ou des délétions au regard de la séquence de référence de sorte d'obtenir le meilleur alignement possible entre ces deux séquences. Le pourcentage d'identité est calculé en déterminant le nombre de positions identiques entre les deux séquences, en divisant le nombre obtenu par le nombre total de positions comparées et en multipliant le résultat obtenu par 100 pour obtenir le pourcentage d'identité entre ces deux séquences.

Par « fragment », on entend un polypeptide comprenant les 4 motifs tels que décrits précédemment et présentant une séquence d'au moins 150 acides aminés, à titre d'exemple d'au moins 450 acides aminés, à titre d'exemple d'au moins 700 acides aminés, et de manière particulièrement préférée un polypeptide d'au moins 1 000 acides aminés.

De préférence, on entend par fragment un polypeptide comprenant les domaines A, B et C comme la séquence SEQ ID NO: 12.

Les termes "acide aminé" et "acides aminés" au sens de la présente invention correspondent à tout acide aminé présent naturellement ou à leurs résidus. Les acides aminés peuvent être identifiés soit par leur abréviation à une seule lettre, soit par leur abréviation à trois lettres. (Asp D acide aspartique ; Ile I isoleucine ; Thr T threonine ; Leu L leucine ; Ser S serine ; Tyr Y tyrosine ; Glu E acide glutamique ; Phe F phenylalanine ; Pro P proline ; His H histidine ; Gly G glycine ; Lys K lysine ; Ala A alanine ; Arg R arginine ; Cys C cysteine ; Trp W tryptophane ; Val V valine ; Gln Q glutamine ; Met M methionine ; Asn N asparagine). Selon la présente invention, les acides aminés naturels peuvent être remplacés par des acides aminés chimiquement modifiés.

La détermination de l'activité enzymatique du polypeptide selon l'invention peut être déterminée par des méthodes connues de l'Homme de l'art, telle que par utilisation de la technique de Chromatographie Liquide à Haute performance sur des produits de la réaction avec le polypeptide selon l'invention (MOULIS et al., J. Biol.Chem, 2006) ou dosage des sucres réducteurs par la méthode à l'acide dinitrosalycilique (SUMNER & HOWELL, 1935). Plus spécifiquement, cette activité enzymatique est exprimée en unité glucane-saccharase qui représente la quantité d'enzyme qui libère une µmole de fructose par minute, à 30 °C, avec une concentration de 100 g.L-1 de saccharose et dans un tampon à pH 5,2 comprenant 50mM acétate de sodium. Cette activité est déterminée préférentiellement par la mesure de la vitesse initiale de production des sucres réducteurs (fructose) à l'aide de la méthode du DNS (SUMNER & HOWELL). Pour se faire, une gamme étalon de 0 à 2 g.L-1 de fructose est réalisée. Au cours d'une cinétique, 100 µl de milieu réactionnel sont ensuite prélevés et la réaction est stoppée par ajout d'un volume équivalent de réactif. Les échantillons sont ensuite chauffés 5 min à 95°C, refroidis dans la glace, dilués au demi dans l'eau et l'absorbance est lue à 540 nm.

Le polypeptide est "isolé" au sens de la présente invention en ce sens qu'il a été soustrait à son environnement originel (l'environnement dans lequel il est localisé naturellement). Par exemple, un polypeptide présent à l'état naturel dans une cellule n'est pas isolé. Le même polypeptide séparé des autres polypeptides adjacents au sein de la cellule dans laquelle il est naturellement présent, le plus couramment par un procédé de purification, est isolé.

Selon un mode de réalisation préféré, le substrat du polypeptide selon l'invention est choisi dans le groupe comprenant l'a-D-glucopyranosyl fluoride, le *p-*nitrophenyl α-D-glucopyranoside, le α-D-glucopyranosyl α-L-sorofuranoside, le lactulosucrose et le saccharose, de préférence le saccharose qui est le substrat naturel.

Le « saccharose » est constitué d'une unité α-D-glucopyranosyle et d'une unité β-D-fructofuranosyle associées par une liaison (alphal-béta2). L'hydrolyse du saccharose conduit à un mélange de glucose et de fructose.

On entend par « unité glucosyle » le résidu provenant du clivage du saccharose qui se trouve associé temporairement à l'enzyme sous la forme d'un β-glucosyl-enzyme et qui est transféré sur un accepteur comprenant un groupement hydroxyle par formation d'une liaison osidique avec ce groupement hydroxyle.

On entend par « branchement en alpha-1,3 » selon l'invention une liaison glucosidique de condensation entre la fonction -OH du carbone située en position 1 d'un premier sucre et une fonction -OH du carbone située en position 3 d'un autre sucre, ladite liaison glucosidique étant formée dans une configuration alpha.

L'expression « branchement d'unités glucosyle en alpha-1,3 » fait référence à une liaison glucosidique en alpha-1,3 entre un accepteur selon l'invention comprenant au moins un groupement hydroxyle et une unité glucosyle issue de l'hydrolyse du saccharose par le polypeptide selon l'invention.

Le terme « accepteur » selon l'invention fait référence à toute molécule organique comprenant au moins un groupement hydroxyle (-OH) libre, lequel accepteur est ajouté dans le milieu réactionnel en complément du substrat donneur d'unités glucosyle.

Un tel accepteur peut être choisi parmi les accepteurs glucidiques et non glucidiques.

Des exemples d'accepteurs non glucidiques comprennent de manière non limitative les alcools, les polyols, les composés phénoliques ou encore les acides aminés.

Des exemples d'accepteurs glucidiques sont avantageusement des polysaccharides ou plus généralement des accepteurs comprenant des unités glycosyle.

Selon un mode de réalisation préférée, un accepteur selon l'invention est un accepteur glucidique, de préférence celui-ci comprendra des unités glucosyle.

Toujours selon un mode de réalisation préférée, un accepteur selon l'invention comprend les polysaccharides. Par polysaccharide, on entend un polymère de sucres comprenant n unités de sucres où n est entier supérieur ou égal à 3.

Avantageusement, ces polysaccharides sont composés exclusivement de monomères de glucose (glucanes), et ils peuvent être linéaires ou bien ramifiés. Ces polysaccharides peuvent correspondre soit à des α-glucanes, soit à des β-glucanes.

Les α-glucanes sont des polymères de glucose liés entre eux en position a. A titre d'exemple d'a-glucanes, on peut citer le dextrane (plus de 50% de liaisons α-1,6 dans la chaine principale), le dextrane ramifié en α-1,2 (par action d'une α-1,2 « branching sucrase »), l'alternane (liaisons α-1,6 et α-1,3 alternées dans la chaine principale), le mutane (plus de 50% de liaisons α-1,3), le reuterane (liaisons α-1,4 et α-1,6 dans la chaine principale), l'amidon (a-1,4- et α-1,6-glucane), l'amylopectine (α-1,4- et α-1,6-glucane), l'amylose (α-1,4-glucane), le glycogène (a-1,4- et α-1,6-glucane) et le pullulane (a-1,4- et α-1,6-glucane).

Les β-glucanes sont des polymères de glucose liés entre eux en position β. A titre d'exemple de β-glucanes, on peut citer la cellulose (β-1,4-glucane), le curdlan (β-1,3-glucane), le laminarin (β-1,3- et β-1,6-glucane), le lentinane (β-1,6:β-1,3-glucane), le pamylon, le pleurane (β-1,3- et β-1,6-glucane) et le zymosan (β-1,3-glucane).

Maintenant, un accepteur préféré serait un α-glucane comme l'amylose, l'amidon, l'amylopectine, le dextrane, le glycogène ou le pullulane, les dextranes ramifiés en α-1,2, l'alternane, le mutane et le reuterane.

Selon un mode de réalisation particulièrement préféré, un accepteur selon l'invention est un dextrane, polymère dont les unités glucosyle sont reliées entre elles par des liaisons alpha 1-6. Ce polymère peut également comprendre des ramifications constituées de liaisons alpha-1,2 ou -1,3 ou -1,4.

Les dextranes utilisés en tant qu'accepteur selon l'invention présentent un poids moléculaire (PM) compris entre 300 et 10⁹ Dalton (Da), de préférence entre 10³ et 10⁹ Da et de manière encore plus préférée entre 1000 et 2.10⁶ Da.

L'avantage présenté par l'invention consiste en ce que le polypeptide tel que décrit précédemment est responsable de la formation de branchement d'unités glucosyle en position alpha-1,3 sur un accepteur.

De manière préférée, le polypeptide selon l'invention a la capacité de former uniquement des branchements d'unités glucosyle en alpha-1,3 sur un accepteur à un taux compris entre 1 et 50%, de préférence entre 5% et 40%, et manière plus préférée encore entre 10 et 40%.

De manière préférée encore, le polypeptide selon l'invention a la capacité de former uniquement des branchements d'unités glucosyle en alpha-1,3 sur un accepteur à un taux maximal de 50%.

Un autre objet de l'invention concerne un polynucléotide isolé codant pour un polypeptide tel que défini précédemment ou un fragment de celui-ci.

Selon l'invention, ledit polynucléotide est une molécule d'ADN ou d'ARN.

Le terme "polynucléotide" se réfère de façon large à une molécule d'ADN comme par exemple un ADNc (ADN complémentaire) ou de l'ADN génomique ou synthétique, ou à une molécule d'ARN, comme par exemple un ARN messager ou encore de l'ARN synthétique, aussi bien qu'à des analogues d'ADN ou ARN contenant des analogues de nucléotides non naturels, des liens internucléotidiques non naturels ou encore les deux. De manière préférée, ledit polynucléotide est une molécule d'ADN. Les polynucléotides peuvent présenter toute conformation topologique, telle que linéaire ou circulaire.

Dans un mode de réalisation préférée de l'invention, ledit polynucléotide est défini par la séquence SEQ ID NO: 10.

Un autre objet de l'invention concerne un vecteur d'expression comprenant un polynucléotide tel que décrit précédemment.

On entend par « vecteur » tout véhicule capable de faciliter le transfert d'un polynucléotide dans une cellule. De manière générale, les vecteurs selon l'invention incluent, sans limitation, les plasmides, cosmides, phagemides ou tout autre véhicule dérivé de sources virales ou bactériennes qui ont été manipulées pour l'insertion ou incorporation d'une séquence nucléotidique.

Le choix des vecteurs utilisables dans le cadre de la présente invention est vaste. Il peut s'agir de vecteurs de clonage et/ou d'expression. D'une manière générale, ils sont connus de l'homme de l'art et nombre d'entre eux sont disponibles commercialement mais il est également possible de les construire ou les modifier par les techniques de manipulation génétique.

De manière préférée, les vecteurs selon l'invention sont des vecteurs plasmidiques, également appelés plasmides. Les plasmides ont été largement décrits dans l'art antérieur et sont bien connus de l'Homme du métier (voir par exemple SANBROOK et al., "Molecular Cloning: A Laboratory Manual," Second Edition, Cold Spring Harbor Laboratory Press, 1989). A titre d'exemples, on peut citer les plasmides les plus communément utilisés tels que pBR322, pUC18, pUC19, pRC/CMV, SV40, et pBlueScript, pET-53-DEST, pET-55-DEST, pBAD49-DEST, pET-60-DEST. Les plasmides peuvent être conçus par l'utilisation d'enzymes de restriction et de réactions de ligation ou de systèmes de recombinaison pour éliminer ou ajouter des fragments d'ADN spécifiques. Les plasmides dans lesquels sont insérées les séquences nucléotidiques sont sous forme d'un ADN simple ou double brin, linéaire ou circulaire.

De préférence, un vecteur mis en oeuvre dans le cadre de la présente invention contient une origine de réplication assurant l'initiation de la réplication dans une cellule productrice et/ou une cellule hôte. Il comprend également les éléments nécessaires à l'expression d'un polynucléotide de l'invention, tels qu'un promoteur et un terminateur. Des exemples de promoteurs utilisables selon l'invention comprennent, mais ne sont pas limités aux promoteurs T7, araBAD, pLac, POX2, AOX *(alcohol oxidase).*

Par ailleurs, il peut en outre comprendre un ou plusieurs gène(s) de sélection permettant de sélectionner ou d'identifier les cellules transformées ou transfectées par ledit vecteur (complémentation d'une mutation d'auxotrophie, gène codant pour la résistance à un antibiotique...). Il peut aussi comprendre des éléments supplémentaires améliorant son maintien et/ou sa stabilité dans une cellule donnée (séquence *cer* qui favorise le maintien monomérique d'un plasmide, séquences d'intégration dans le génome cellulaire).

Le vecteur de l'invention peut éventuellement être associé à une ou plusieurs substances améliorant l'efficacité de transformation ou de transfection et/ou la stabilité du vecteur. Ces substances sont largement documentées dans la littérature accessible à l'homme de l'art. A titre illustratif mais non limitatif, il peut s'agir de polymères, de lipides notamment cationiques, de liposomes, de protéines nucléaires ou encore de lipides neutres. Ces substances peuvent être utilisées seules ou en combinaison. Une combinaison envisageable est un vecteur recombinant plasmidique associé à des lipides cationiques (DOGS, DC-CHOL, spermine-chol, spermidine-chol etc.) et des lipides neutres (DOPE).

Le polynucléotide, préférentiellement la molécule d'ADN, dans le vecteur d'expression est lié de manière opérationnelle à un promoteur pour diriger la synthèse d'ARN. A titre d'exemple, les promoteurs peuvent être des promoteurs eucaryotes ou procaryotes tels que CMV immediate early, HSV thymidine kinase, early and late SV40, LTRs de retrovirus et métallothionéine-I de souris. Le vecteur d'expression comporte également un site de fixation de ribosome pour l'initiation de la traduction et un vecteur de transcription. Le vecteur devrait également comporter des séquences d'amplification de l'expression.

On entend par « liée de manière opérationnelle à un promoteur» le lien par lequel un promoteur est situé de manière contigüe au polynucléotide selon l'invention pour contrôler l'expression de ladite séquence.

Le terme « promoteur » est bien connu de l'homme de l'art et se réfère à une région de l'ADN située à proximité d'un gène sur laquelle se fixe l'ARN polymérase, afin de démarrer la transcription.

Un autre objet de l'invention concerne également une cellule hôte transformée comprenant un vecteur selon l'invention.

Aux fins de la présente invention, une telle cellule est constituée par toute cellule qui peut être transformée ou transfectée par un vecteur de l'invention tels que décrits ci-avant.

La cellule est appelée « cellule hôte » et peut être une cellule procaryote ou une cellule eucaryote.

De manière préférée, la cellule hôte transformée selon l'invention est une cellule procaryote choisie dans le groupe consistant en les eubactéries, les archaebactéries et les cyanobactéries.

Les systèmes d'expression bactériens peuvent être utilisés dans le cadre de la présente invention. Des exemples de cellules hôtes bactériennes comprennent notamment des bactéries des genres *Escherichia* (par exemple *Escherichia coli*), *Pseudomonas* (par exemple *Pseudomonas fluorescens* ou *Pseudomonas stutzerei*), *Proteus* (par exemple *Proteus mirabilis), Ralstonia* (par exemple *Ralstonia eutropha), Streptomyces, Staphylococcus* (par exemple *Streptomyces carnosus*), *Lactococcus* (par exemple *Lactoccocus lactis), Bacillus* (par exemple *Bacillus subtilis, Bacillus megaterium* ou *Bacillus licheniformis*), *Lactobacillus* ou *Leuconostoc* etc.

De manière encore préférée, la cellule hôte transformée selon l'invention est une cellule eucaryote choisie dans le groupe consistant en les cellules animales, fongiques, de levure et de plantes.

Les cellules de levures sont des cellules hôtes pouvant convenir dans le cadre de l'invention. Des exemples de cellules hôtes de levure pouvant utilisées comprennent, mais ne sont pas limités à, *Saccharomyces cerevisiae, Schizosaccharomyces pombe, Klyveromyces lactis, Yarrowia lipolytica, Hansenula polymorpha* ou *Pichia pastoris.*

Les systèmes d'expression fongiques sont aussi envisageables dans le cadre de la présente invention, tels que *Aspergillus niger, Chrysosporium lucknowense, Aspergillus* (par exemple *Aspergillus oryzae, Aspergillus niger, Aspergillus nidulans*, etc.), *Podospora anserina* ou *Trichoderma reesei.*

D'autres systèmes d'expression tels que les systèmes d'expression mammifères peuvent encore être utilisés dans le cadre de l'invention, par exemple les lignées cellulaires NSO, CHO, BHK, les systèmes transgéniques d'origine mammifère, mais aussi les cellules d'insectes ou les systèmes d'expression viraux tels que les bactériophages M13, T7 ou λ ou encore les systèmes d'expression *Baculovirus.*

Préférentiellement, la cellule hôte selon la présente invention est une cellule procaryote.

Les termes « cellule hôte transformée », « transformé » et « transformation » au sens de la présente invention font référence à l'introduction d'ADN dans une cellule. L'introduction d'un polynucléotide ou d'un vecteur tel que décrit dans la présente invention dans la cellule hôte peut être effectuée par des méthodes bien connues de l'homme du métier telles l'électroporation, le choc thermique sur cellules compétentes, la recombinaison la conjugaison, la transfection par PEI, par le phosphate de calcium, la transfection par DEAE-Dextran ou encore l'électroporation.

Selon un autre objet, l'invention concerne une composition comprenant au moins un polypeptide, un polynucléotide, un vecteur ou une cellule hôte tels que décrits précédemment.

Un autre objet de l'invention concerne un procédé de production d'un polypeptide tel que décrit précédemment, ledit procédé comprend les étapes de :
a) insertion d'un polynucléotide ou d'un vecteur tel que décrit précédemment dans une cellule hôte;
b) la mise en culture de ladite cellule obtenue à l'étape a) ; et
c) l'extraction du polypeptide selon l'invention à partir de la culture obtenue à l'étape b).

L'étape (a) d'introduction d'un polynucléotide ou d'un vecteur tel que décrit précédemment dans la cellule hôte est réalisée par des techniques de transformation bien connues de l'homme du métier, telles que la transfection, la lipofection, la transformation par l'acétate de lithium, la transformation par biolistique, la transformation par PEI, la fusion de protoplastes, la transformation par liposome, la transformation par *Agrobacterium tumefaciens*, ou encore les infections virales ou adénovirales.

L'extraction du polypeptide de l'invention est faite à partir de la culture de l'étape (b) et réalisée par des techniques bien connues de l'homme du métier. Si l'organisme hôte produit ce polypeptide de manière extracellulaire, le surnageant de culture est récupéré par centrifugation et peut directement être utilisé pour la mise en oeuvre des synthèses de produits. Si l'expression est intracellulaire, les cellules sont centrifugées, concentrées puis lysées au moyen de lysozymes et de détergents ou broyées aux ultrasons ou traitées par cassage mécanique à l'aide de billes de verre ou presse de FRENCH.

Au besoin, l'extraction peut consister en une purification du polypeptide peut être conduite par chromatographie d'affinité pour des métaux chélants tels que nickel ou cobalt et utilisation d'un tag (étiquette) de type « Histidine » (6 histidines successives) fusionné à la séquence du polypeptide tel que décrit précédemment.

Un autre objet de l'invention concerne un procédé de production d'accepteurs branchés d'unités glucosyle en alpha-1,3 comprenant un taux de branchements de telles unités glucosyle en alpha-1,3 compris entre 1 et 50 %, ledit procédé comprenant les étapes de:
i) mélange dans un milieu réactionnel d'un polypeptide selon l'invention, d'un substrat dudit polypeptide et d'un accepteur comprenant au moins un groupement hydroxyle; et
ii) incubation dudit mélange obtenu à l'étape i) de sorte d'obtenir le branchement d'unités glucosyle en alpha -1,3 sur ledit accepteur.

On entend par « accepteur branché d'unités glucosyle en alpha-1,3 » selon l'invention un accepteur tel que défini précédemment sur lequel sont fixées par l'action d'un polypeptide selon l'invention des unités glucosyle issues de l'hydrolyse du substrat.

Selon un mode de réalisation préféré, un accepteur branché d'unités glucosyle en alpha-1,3 selon l'invention est sélectionné parmi le groupe comprenant les polysaccharides, de préférence les glucanes, notamment les α-glucanes comme les α-1,6 glucanes (e.g. dextrane), les dextranes ramifiés en α-1,2, les alternanes, les mutanes, les reuteranes, l'amidon, l'amylopectine, l'amylose, le glycogène et le pullulane.

Selon un mode de réalisation préféré et tel que décrit ci-dessus, le substrat du polypeptide selon l'invention est choisi dans le groupe comprenant l'a-D-glucopyranosyl fluoride, le p-nitrophenyl α-D-glucopyranoside, le α-D-glucopyranosyl α-L-sorofuranoside, le lactulosucrose et le saccharose, de préférence le saccharose.

Selon un mode de réalisation préféré, le procédé selon l'invention permet de contrôler le taux de branchement alpha-1,3 de l'accepteur en faisant varier directement le rapport entre la concentration en substrat et celle en accepteur.

Selon un mode de réalisation préféré de l'invention, ledit procédé est caractérisé en ce qu'il est destiné à l'obtention d'un accepteur branché d'unités glucosyle en alpha-1,3 à un taux compris entre 1% et 50 %, de préférence entre 5 et 40% et de manière plus préférée encore entre 10 et 40%. Cette variation est possible en fonction du rapport entre la concentration du substrat sur celle des groupements hydroxyles libre de la molécule acceptrice, ou du rapport entre la concentration massique du substrat sur celle de l'accepteur lorsqu'il s'agit de dextrane et de saccharose.

Avantageusement, les concentrations d'accepteur et de substrat sont ajustées de sorte d'obtenir un taux de branchement compris entre 35 et 50%, de préférence entre 35 et 40%. Typiquement, ce taux de branchement est obtenu avec un rapport supérieur ou égal à 1.

Avantageusement encore, les concentrations d'accepteur et de substrat sont ajustées de sorte d'obtenir un taux de branchement compris entre 20 et 35%. Typiquement, ce taux de branchement est obtenu avec un rapport compris entre 0,5 et 1.

Avantageusement toujours, les concentrations d'accepteur et de substrat sont ajustées de sorte d'obtenir un taux de branchement inférieur à 20%. Typiquement, ce taux de branchement est obtenu avec un rapport inférieur à 0,5.

Selon l'invention, le taux de branchements en alpha-1,3 obtenus dans le cadre du présent procédé est considéré par rapport à la totalité des sites disponibles sur ledit accepteur.

Selon un mode de réalisation particulier, ledit procédé selon l'invention comprend en outre une étape c) de purification des accepteurs branchés d'unités glucosyle en alpha-1,3.

Toujours selon un mode de réalisation particulier, ledit procédé selon l'invention comprend une étape d) de caractérisation des accepteurs branchés d'unités glucosyle en alpha-1,3 selon l'invention. Une telle étape de caractérisation peut être réalisée par diverses méthodes bien connues de l'Homme de l'art.

A titre d'exemple, seront utilisées la technique de chromatographie liquide sous haute pression (HPLC), la spectrométrie de masse, la spectrométrie par résonance magnétique nucléaire (RMN) les techniques chimiques comme la méthylation et l'acétolyse ou encore l'ELISA avec des anticorps monoclonaux spécifiques des liaisons alpha-1,3.

La présente demande divulgue également un accepteur branché d'unités glucosyle en alpha-1,3 susceptible d'être obtenu par le procédé tel que décrit précédemment.

Ledit accepteur peut ne pas être un dextrane.

Avantageusement encore, le taux de branchement dudit accepteur est inférieur à 50%, de préférence inférieur à 40%.

La présente demande de brevet vise également à couvrir les diverses utilisations possibles d'un polypeptide, d'un polynucléotide, d'un vecteur, d'une cellule hôte et/ou d'une composition de l'invention tels que décrits précédemment.

Ainsi, un autre objet de l'invention concerne l'utilisation d'un polypeptide, d'un polynucléotide, d'un vecteur, d'une cellule hôte et/ou d'une composition selon l'invention pour la production d'accepteurs branchés d'unités glucosyle en alpha-1,3.

De manière préférée, lesdits accepteurs sont branchés d'unité glucosyle en alpha-1,3 à un taux compris entre 1 et 50%, de préférence entre 5 et 40%, et de manière préférée encore entre 10 et 40%.

La présente demande de brevet vise également à couvrir les diverses utilisations possibles d'un accepteur branché d'unités glucosyle en alpha-1,3 selon l'invention.

La présente demande divulgue également l'utilisation d'un accepteur branché d'unités glucosyle en alpha-1,3 produits à partir d'un polypeptide selon l'invention en tant qu'agent de charge, épaississant, émulsifiant, texturant et/ou stabilisant dans la préparation de formulations industrielles alimentaires, cosmétiques, agrochimiques, pétrochimiques et pharmaceutiques.

Ces utilisations consistent en l'utilisation desdits accepteurs branchés d'unités glucosyle en alpha-1,3 en tant que biopolymères.

Des exemples de formulations industrielles selon l'invention incluent de manière non limitative les bioplastiques mais aussi les formulations agroalimentaires, par exemple des produits de boulangerie, ainsi que des formulations dans le secteur pharmaceutique.

D'autres exemples de formulations industrielles incluent également des formulations destinées aux industries du bâtiment, de la peinture, du papier, du textile, des phytosanitaires, du traitement des eaux, industrie pétrolière.

La présente demande divulgue également l'utilisation non thérapeutique d'un accepteur branché d'unités glucosyle en alpha-1,3 en tant qu'agent prébiotique.

Les accepteurs branchés d'unités glucosyle en alpha-1,3 produits selon l'invention présente les avantages d'avoir une meilleure résistance à la barrière digestive, une meilleure stabilité, des coûts de production potentiellement moins chers, et une plus grande facilité à être incorporés aux préparations alimentaires.

Les prébiotiques sont des ingrédients alimentaires non digestibles qui arrivent intacts dans le colon où ils sont alors spécifiquement métabolisés par une certaine catégorie du microbiote intestinal (humain ou animal) dite « bénéfique ».

A titre d'exemples non limitatifs d'effets prébiotiques, on peut citer l'amélioration du transit intestinal chez les animaux et chez l'homme, l'amélioration de l'assimilation des minéraux tels que le calcium, le magnésium, le zinc ou encore le fer, la réduction de l'inflammation intestinale ou encore la réduction de la croissance de pathogènes.

### EXEMPLES

### Exemple 1 : Criblage de nouvelles enzymes chez L. citreum NRRL B-742

Après séquençage du génome de la souche *L. citreum* NRRL B-742, un gène s'est avéré particulièrement original En effet, la protéine putative correspondante s'est avérée posséder une séquence présentant un maximum d'identité de seulement 54% avec la Glycoside-Hydrolase putative de *Leuconostoc fallax* KCTC 3537 dont la séquence est disponible dans les bases de données, et référencée par le NCBI sous le numéro ZP_08312597. Maintenant, aucune autre séquence protéique présentant une identité significative n'a pu être identifiée.

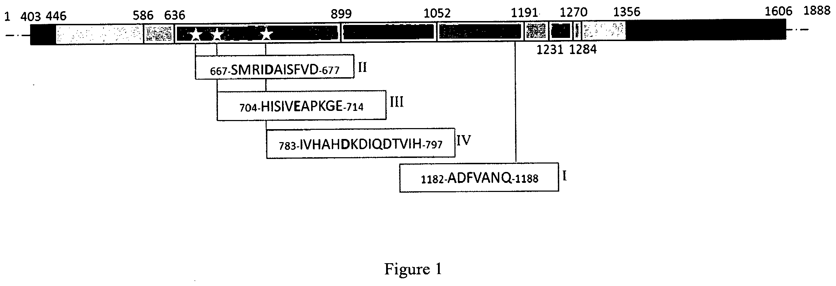

Ce gène code pour une transglucosylase putative de 1888 acides aminés, possédant la triade catalytique caractéristique DED et les 4 zones conservées décrites habituellement chez les transglucosylases de la famille 70. La représentation schématique de la protéine est montrée à la figure 1 (basée sur l'alignement de séquences protéiques avec la GTF180) avec 5 domaines : i) domaine V (403-446 et 1356-1800), ii) domaine IV (446-586 et 1284-1356), iii) domaine A (catalytique) (636-899, 1052-1191 et 1231-1270), iv) domaine B (586-636, 1191-1231 et 1270-1284) et v) domaine C (899-1052). Les acides aminés catalytiques (DED) sont indiqués à l'aide d'une étoile dans la structure primaire et sont représenté en gras et rouge dans les différents motifs II, III et IV.

Maintenant, des motifs protéiques particulièrement originaux ont pu être identifiés en amont et en aval des acides aminés de la triade catalytique, dans des zones habituellement fortement conservées (cf. tableau 1).

Du fait de l'originalité de cette transglucosylase putative, il a été décidé d'en initier le clonage en vue de débuter sa caractérisation biochimique.

**Tableau 1 : Alignement de séquences des zones conservées du coeur catalytique de la nouvelle transglucosylase (nommée α-1,3BrS dans le tableau) et des orthologues identifiés (issus de Leuconostoc fallax KCTC3537 et de Leuconostoc citreum LBAE E16) avec des enzymes caractérisées et de spécificité de liaisons connue**

| GenBank | | | **Motif II** | | **Motif III** | | **Motif IV** | | **Motif** I | Spécificité |
|---|---|---|---|---|---|---|---|---|---|---|
| AAC63063.1 | GtfI [Sd] | 449 | SIRVDAVDNVD | 486 | HVSIVEAWSDN | 559 | FARAHDSEVQDLIRD | 931 | ADWVPDQ | α-1,3 |
| AAA88588.1 | GtfB [Sm] | 1011 | SIRVDAVDNVD | 1048 | HLSILEAWSDN | 1120 | FIRAHDSEVQDLIAD | 1488 | ADWVPDQ | |
| BAA26114.1 | GtfSI [Sm] | 473 | SIRVDAVDNVD | 510 | HLSILEAWSDN | 583 | FIRAHDSEVQDLIRD | 954 | ADWVPDQ | |
| AAU08015.1 | GtfA [Lr] | 1020 | SVRVDAPDNID | 1056 | HINILEDWNHA | 1128 | FVRAHDNNSQDQIQN | 1508 | ADWVPDQ | α-1,4/α-1,6 |
| AAY86923.1 | GtfO [Lr] | 1020 | SVRVDAPDNID | 1056 | HINILEDWNSS | 1128 | FIRAHDNNSQDQIQN | 1508 | ADWVPDQ | |
| CAB65910.2 | Asr [Lm] | 631 | GIRVDAVDNVD | 668 | HLSILEDWNGK | 762 | FVRAHDYDAQDPIRK | 1168 | ADWVPDQ | α-1,6/α-1,3 |
| ABQ83597.1 | GtfW [Lr] | 748 | GFRVDAADNID | 785 | HLVYNEGYHSG | 568 | FVTNHDQR-KNVINQ | 1216 | EDLVMNQ | α-4,6 |
| AAU08003.2 | GtfML4 [Lr] | 1012 | GFRVDAADNID | 1049 | HLSYNEGYHSG | 1121 | FVTNHDQR-KNLINR | 1479 | EDIVMNQ | |
| ABF85832.1 | DsrCB4 [Lc] | 526 | GIRVDAVDNVD | 563 | HLSILEDWSHN | 636 | FVRAHDSEVQTVIAQ | 1001 | ADWVPDQ | α-1,6 |
| CAB76565.1 | DsrC [Lm] | 498 | GIRVDAVDNVD | 535 | HLSILEDWSHN | 608 | FVRAHDSEVQTVIAQ | 973 | ADWVPDQ | |
| AAD10952.1 | DsrS [Lm] | 547 | GIRVDAVDNVD | 584 | HLSILEDWSHN | 657 | FVRAHDSEVQTVIAQ | 1023 | ADWVPDQ | |
| AAU08001.1 | GTF180 [Lr] | 1021 | GIRVDAVDNVD | 1058 | HINILEDWGWD | 1131 | FVRAHDSNAQDQIRQ | 1503 | ADWVPDQ | |
| CAD22883.1 | GBD-CD₂ [Lc] | 2206 | SIRIDAVDFIH | 2243 | HISLVEAGLDA | 2317 | IIHAHDKGVQEKVGA | 2688 | ADVVDNQ | α-1,2 |
| | α-1,3 BrS [Lc] | 667 | SMRIDAISFVD | 704 | HISIVEAPKGE | 783 | IVHAHDKDIQDTVIH | 1182 | ADFVANQ | |
| | α-1,3 BrS [L. fallax] | 734 | SIRIDAISFVD | 771 | HVSIVEASADQ | 845 | IVHAHDKDIQDAVSN | 1232 | ADYVANQ | |
| | α-1,3 BrS [L. citreum E16] | 667 | SMRIDAISFVD | 704 | HISIVEAPKGE | 783 | IVHAHDKDIQDTVIH | 1182 | ADFVANQ | |

### Exemple 2 : Production d'une nouvelle enzyme chez E. coli

Le gène codant pour cette enzyme a été cloné dans plusieurs vecteurs (pET 53, 55, 49 et 60) commercialisés par NOVAGEN ou INVITROGEN, et exprimé dans différentes souches d'*E. coli* (TOP10, BL21AI, BL21 Star DE3, Arctic Express DE3).

Ce clonage a permis une production conséquente de la protéine. Cette production a donc permis d'initier les expériences de caractérisation biochimique en vue de préciser les propriétés catalytiques de l'enzyme identifiée.

Simultanément, une forme tronquée du peptide signal et en C-terminal, ΔPS ΔC-1313, SEQ ID NO: 11 et SEQ ID NO: 12 pour les séquences nucléique et protéique respectivement) de la protéine a été clonée et exprimée dans la souche *d'E.coli* BL21 star DE3, ce qui a permis là encore une expression conséquente de la protéine ; laquelle expression s'est révélée près de deux fois supérieure à celle de la protéine sauvage.

### Exemple 3 : Réactions d'un polypeptide selon l'invention avec du saccharose, un accepteur de type dextrane et l'enzyme objet de l'invention et analyse des produits de cette réaction

Pour la caractérisation des propriétés fonctionnelles de cette transglucosylase putative, l'enzyme a d'abord été mise en oeuvre sur saccharose seul, substrat naturel des enzymes de la famille GH70.

De façon inattendue, les analyses chromatographiques (HPAEC-PAD, HPSEC) ont montré que l'enzyme seule n'est capable que d'hydrolyser ce substrat en quantité équimolaires de glucose + fructose. Maintenant, et à partir de saccharose seul, cette enzyme n'a montré aucune capacité à produire des polymères d'unités glucosyle, de même que sa forme tronquée.

Il est intéressant de noter qu'à ce jour, les analyses bioinformatiques sur la structure primaire des GH de la famille 70 n'auraient pas permis de prédire cette particularité (les déterminants structuraux régissant la capacité -ou non- d'une transglucosylase à polymériser ne sont pas encore connus).

Alors que rien ne présageait que cette protéine présentait malgré tout une activité, cette dernière (tout comme sa forme tronquée) a aussi été incubée en présence de saccharose et d'un dextrane linéaire (glucane composé exclusivement de liaisons α-1,6) d'un poids moléculaire de 1500 Da.

Ont été incubés pendant 16h à 30°C, de l'enzyme dans du saccharose (de 25g/L à 170g/L) en présence de dextranes de masse molaire variable (de 1500 Da à 2.10⁶Da) et de concentration variant de 30g/L à 100g/L. ; le rapport Saccharose/Accepteur (M/M) variant selon le taux de branchement en α-1,3 souhaité. Le milieu réactionnel a été tamponné avec une solution d'acétate de sodium à 50mM final, pH 5.2. Un prélèvement au temps initial et final de la réaction a été effectué, chauffé à 95°C pendant 5 minutes afin de stopper la réaction puis analysé par différentes techniques chromatographies (HPAEC-PAD, HPSEC) et structurales (RMN du proton).

Plus spécifiquement, les monosaccharides, disaccharides et petits oligosaccharides (degré de polymérisation inférieur à 20) ont été séparés et quantifiés par HPAEC-PAD (High Performance Anion Exchange Chromatography with Pulsed Amperometric Detection), sur colonne Dionex CarboPac PA-100. Un gradient d'acétate de sodium de 6 à 300 mM en 36 min, contenant 150 mM de soude permet de séparer glucose, fructose, saccharose, leucrose, isomaltooligosaccharides, etc. Des gammes étalons de 5, 10, 15 et 20 mg.kg-1 de ces sucres ont été réalisées pour permettre leur quantification. Ces échantillons ont été dilués pour être à une concentration en sucres totaux de 25 mg.kg-1.

Des analyses par HPSEC (High-Performance Size Exclusion Chromatography) ont permis d'estimer la masse moléculaire des populations d'oligosaccharides ou des polymères synthétisés au cours des réactions. La séparation s'est faite à l'aide de deux colonnes SHODEX (OH-Pack SB-805 et 802.5) placées en série. Des solutions de 1, 2,5, 5, et 10 g.L-1 de saccharose, fructose, maltoheptaose, Dextrane 11,3 kDa, 68,4 kDa, 503 kDa, 2000 kDa ont servis d'étalons. Les échantillons ont été dilués au 10e pour être à une concentration maximale de 10 g.kg-1. Une solution de 0,45 M NaNO3 + 1% (v/v) d'éthylène glycol à 0,3 mL.min-1 sert d'éluant, et les échantillons doivent être dilués dans la phase mobile. Colonnes et précolonnes sont maintenues à 70°C, la détection se fait par réfractométrie.

Pour les analyses RMN, les polymères synthétisés ont été conservés à -80°C pendant la nuit pour être ensuite lyophilisé (appareil CHRIST ALPHA 2-4). 10 mg de la poudre obtenue sont ensuite dissous dans 0.5mL d'eau deutérée et analysée par RMN proton. Les spectres RMN 1H ont été réalisés sur un spectromètre BRUKER AVANCE (500 MHz). Les données ont ensuite été traitées avec le logiciel TOPSPIN 3.0.

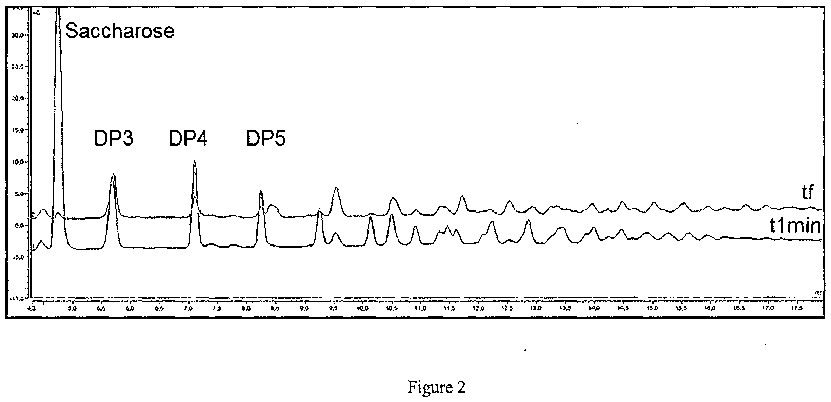

La figure 2 montre les Profils HPAEC-PAD d'une réaction utilisant comme accepteur du dextrane 1500Da.

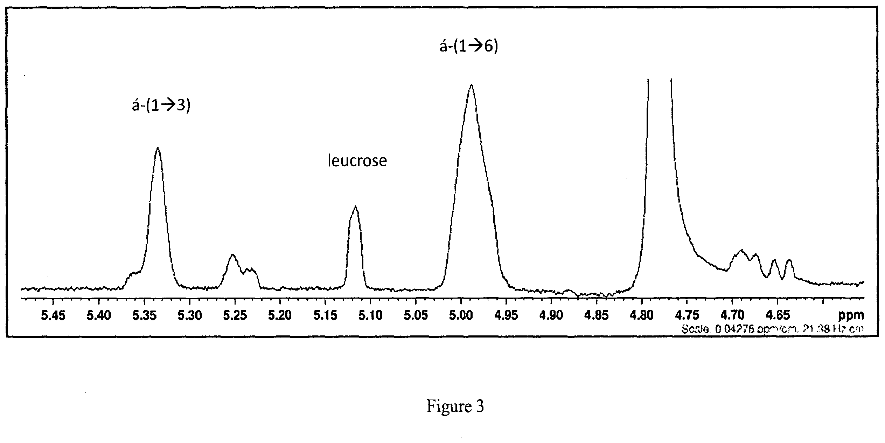

La figure 3 montre le profil RMN du dextrane 1500Da obtenu en fin d'incubation.

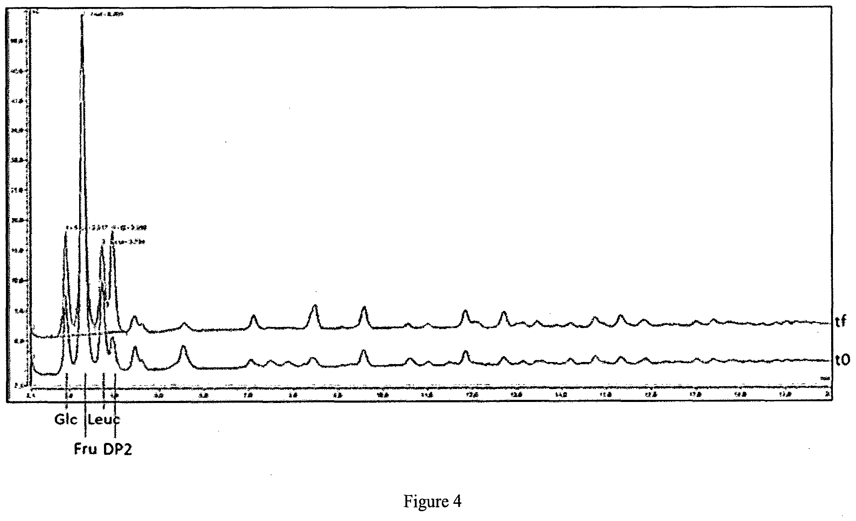

La figure 4 montre les profils HPAEC-PAD d'une digestion à l'endodextranase des produits de la réaction d'accepteur Saccharose + dextrane 1500 Da (to= dextrane 1500Da branché en α-1,3).

Alors qu'aucune polymérisation n'était observée en présence de saccharose seul, les résultats ont révélé une modification des caractéristiques du dextrane. Une analyse plus fine des résultats de chromatographie et d'analyses structurales par RMN du proton mettent en évidence la synthèse de branchements en α-1,3 sur la molécule acceptrice (figures 2 et 3). Notamment, le produit de la réaction résiste à l'action d'une endodextranase, enzyme spécifique de l'hydrolyse des liaisons α-1,6 (Figure 4). Plus globalement, ces résultats laissent présumer d'une résistance à l'action des enzymes digestives, et donc de l'existence de propriétés prébiotiques comparables à celles des isomaltooligosaccharides ou des gluco-oligosaccharides branchés en α-1,2 (GOFFIN et al., Crit. Rev. Food Sci. Nutr., vol.51(5), p:394-409, 2011 ; SARBINI et al., Appl. Environ. Microbiol., vol.77(15), p:5307-15, 2011).

L'enzyme, tout comme sa forme tronquée, s'est par ailleurs montrée particulièrement efficace pour catalyser le transfert de résidus glucosyle sur des isomaltooligosaccharides puisque l'ajout de dextrane 1500Da en tant que molécule acceptrice dans le milieu réactionnel a pour effet de multiplier l'activité de l'enzyme d'un facteur 26 (par rapport à l'activité mesurée sur saccharose seul).

Finalement, ces résultats mettent en évidence que la protéine identifiée est responsable des branchements en α-1,3 du glucane correspondant à la fraction S et qu'une enzyme seule comme on le croyait jusqu'alors n'est pas à la base de la synthèse de ce glucane spécifique. Du fait de cette activité, le gène correspondant, codant pour une «enzyme responsable de glucosylations spécifiques par branchement en α-1,3» a été baptisé *α-1,3BrS*.

### Exemple 4 : Influence de la variation de concentration de substrat/accepteur sur le nombre de branchements obtenus en alpha-1,3

Des expériences complémentaires ont montré en outre qu'en jouant sur la concentration en saccharose par rapport à la concentration en dextrane 1500 Da (rapport donneur/accepteur), **il est possible de contrôler le taux de branchement en α-1,3** de ce petit dextrane.

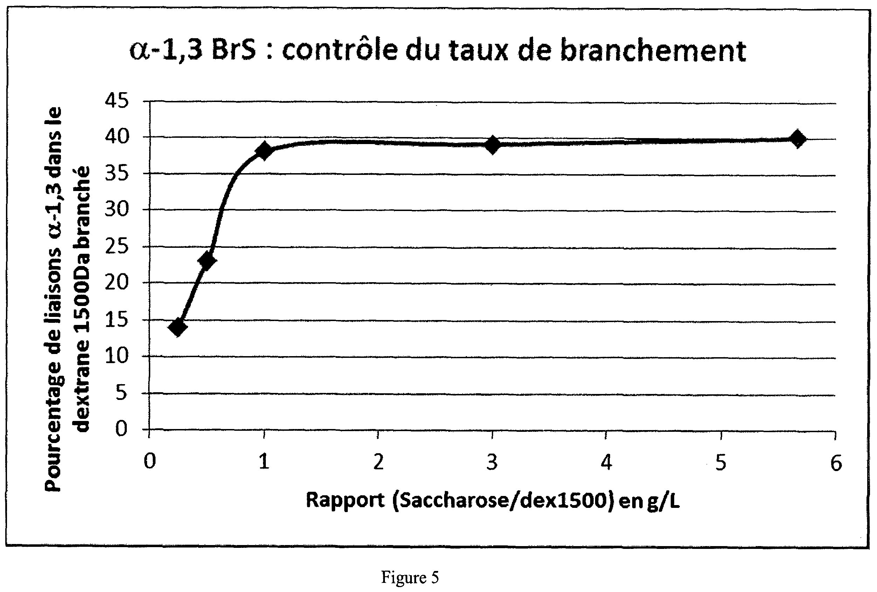

La figure 5 montre le contrôle du taux de liaisons α-1,3 en fonction du rapport saccharose/dextrane 1500Da (utilisation de concentrations massiques).

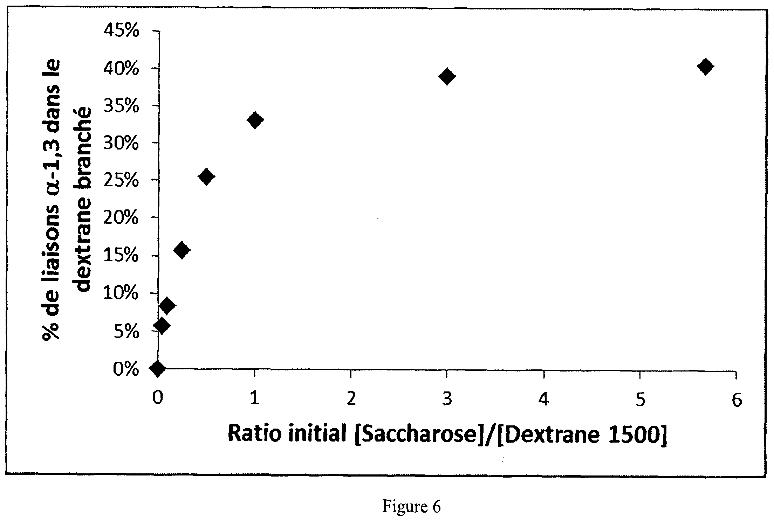

La figure **6** montre également le contrôle du taux de liaisons α-1,3 en fonction du rapport saccharose/dextrane 1500Da mais avec l'enzyme tronquée.

Les résultats montrent qu'à un rapport saccharose sur dextrane 1500 (équivalent à un rapport substrat sur groupement hydroxyle) supérieur ou égal à 1, on arrive à un taux de substitution de l'ordre de 40% et qui tend vers 50% en augmentant la concentration de substrat. Maintenant, en modulant ce rapport, on arrive à diminuer le taux de substitution. Ainsi pour un rapport de 1/2, le taux de substitution est légèrement inférieur à 25%, et pour un rapport de 1/3, le taux de substitution s'élève est légèrement inférieur à 15%.

A noter que les inventeurs sur le même substrat et avec la forme tronquée sont arrivés aux mêmes résultats (figure 6), confirmant le fait que l'enzyme tronquée conserve la même spécificité que la forme entière.

### Exemple 5 : Accepteurs Dextrane

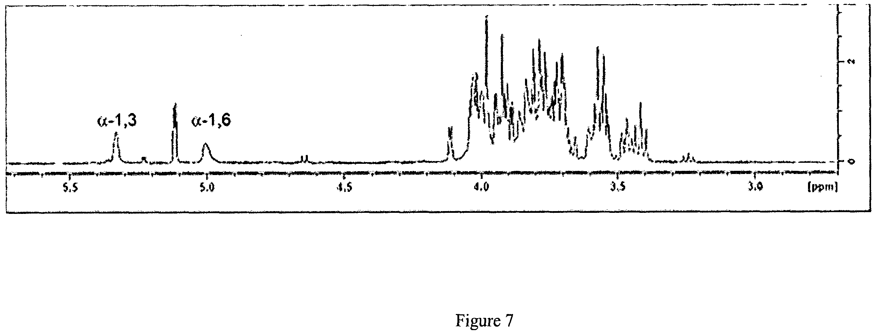

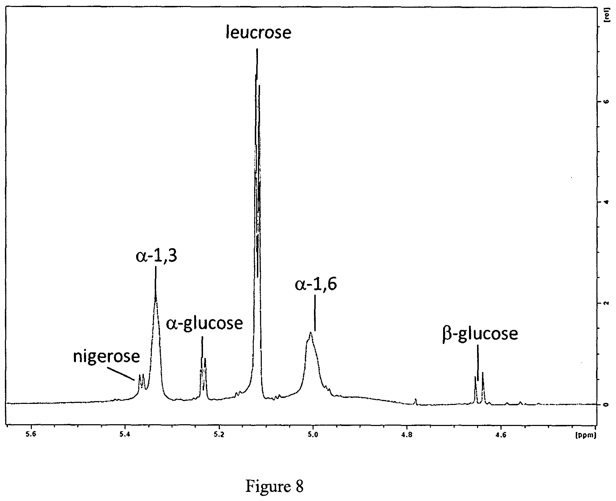

De plus, l'enzyme et sa forme tronquée se sont montrées capable de faire ces réactions de branchements en α-1,3 sur une large gamme de dextranes de masse molaire supérieure. Des essais ont été notamment réalisés sur du dextrane 68.4 kDa, 503 kDa et 2 x 10⁶ Da. D'après des analyses de RMN du proton, les dextranes de haut poids moléculaire présentent 50% de liaisons α-1,3 (figures 7 et 8). On retrouve ici très certainement la structure en peigne décrite dans les années 80 lors des travaux sur le glucane natif produit par la souche.

Les figures 7 et 8 montrent le spectre RMN du dextrane 68.4kDa branché en α-1,3 obtenu avec l'enzyme entière et l'enzyme tronquée respectivement.

Ces résultats montrent que la α-1,3BrS est donc capable de reconnaitre et de brancher de nombreux dextranes dont la masse molaire varie de 1,5 à 2 x 10⁶ kDa. Nous pouvons donc proposer un panel de produits allant des petits gluco-oligosaccharides prébiotiques aux polymères de haute masse molaire, avec des taux contrôlés -à façon- de branchements en α-1,3.

### Exemple 6 : Identification d'un orthologue chez L. citreum.

Fort de cette caractérisation, les inventeurs ont cherché des orthologues à cette enzyme, ce qui a permis une telle identification d'un orthologue de cette enzyme dans le génome de la souche *L. citreum* LBAE E16 (SEQ ID NO: 13 et SEQ ID NO: 14 pour les séquences nucléique et protéique de cet orthologue respectivement).

Une analyse comparative de la séquence nouvellement identifiée avec la précédente a révélée qu'elles partagent 98% d'identité pour la séquence complète avec une identité de 100% en ce qui concerne les motifs I à IV du coeur catalytique.

### Exemples 7 : Identification d'un orthologue chez L. fallax.

L'identification d'un orthologue a ouvert la voie à la recherche d'autres orthologues dans d'autres espèces, ce qui a permis aux inventeurs d'identifier chez la souche KCTC 3537 de *Leuconostoc fallax* une protéine (SEQ ID NO: 16) présentant une identité d'ensemble de près de 54% avec la séquence protéique d'a-1,3 BrS, laquelle monte à 68% lorsque l'on se focalise sur les domaines catalytiques A, B et C.

Une analyse des motifs catalytiques I, II, III et IV montre une légère divergence en ce qui concerne le motif III.

| | Motif II (SEQ ID NO:) | Motif III (SEQ ID NO:) | Motif IV (SEQ ID NO:) | Motif I (SEQ ID NO:) |
|---|---|---|---|---|
| α-1,3 BrS | | HISIVEAPKGE (19) | | ADFVANQ (17) |
| GH *L.fallax* | SIRIDAISFVD (22) | | | ADYVANQ (21) |
| Identité (%) | 90 | 54,5 | 80 | 85 |

Comme précédemment, les inventeurs ont effectué un clonage de cette glycoside hydrolase putative et son expression recombinante chez *E.coli.*

Pour se faire, les inventeurs ont préalablement réalisé un gène synthétique (SEQ ID NO: 15), par optimisation de codon du gène sauvage, en vue de faciliter l'expression recombinante de la protéine chez *E. coli* BL21 star DE3 comme précédemment. La production de la protéine a permis l'obtention d'une quantité de protéine permettant sa caractérisation (production 2,5 fois supérieure à celle obtenue pour α-1,3 BrS).

Mise en présence de saccharose seul, l'enzyme a révélée, comme pour l'a-1,3 BrS, son incapacité à polymériser les unités glucosyle. Maintenant, et comme pour l'a-1,3 BrS, elle a montré sa capacité à effectuer des branchements en α-1,3 sur un dextrane de 1500 Da avec un taux de branchement de 37%.

### Exemple 8 : Développement d'un procédé en 1 seule étape pour la production d'oligosaccharides avec des branchements contrôlés en α-1,3.

Ce procédé consiste en la mise en oeuvre d'une polymérase de la famille GH-70 couplée à l'action de l'enzyme α-1,3 BrS sur saccharose seul.

Dans sa mise en oeuvre, le procédé est testé avec une proportion relative variable des deux enzymes.

Les produits formés issus de la réaction enzymatique sont analysés par chromatographies (HPAEC-PAD, HPSEC afin de déterminer la taille des oligosaccharides produits) et par RMN du proton (détermination de la proportion de liaisons a -1,3).

### Exemple 9 : Evaluation des propriétés physico-chimiques des glucanes de très haute masse molaire à teneur en liaisons α-1,3 contrôlée.

Différents glucanes de très haute masse molaire sont incubés en présence de l'enzyme α-1,3 BrS. Les propriétés physico-chimiques des glucanes obtenus sont investiguées, notamment par analyse thermogravimétrique, par détermination de la température de transition vitreuse, et par analyse rhéologique (cf. IRAGUE et al, Biomacromolecules, 2012).

### Exemple 10 : Evaluation des propriétés physico-chimiques des glucanes de très haute masse molaire à teneur contrôlée en liaisons α-1,2 et α-1,3.

Différents glucanes de très haute masse molaire sont incubés en présence de l'enzyme α-1,3 BrS et de l'enzyme GBD-CD2 (BRISON et *al.,* 2009) qui montre une activité de branchement en α-1,2. Les propriétés physico-chimiques des glucanes obtenus sont investiguées, notamment par analyse thermogravimétrique, par détermination de la température de transition vitreuse, et par analyse rhéologique (cf. IRAGUE et al, Biomacromolecules, 2012).

### Exemple 11 : Evaluation des propriétés physico-chimiques et prébiotiques d'oligosaccharides à teneur contrôlée en liaisons α-1,2 et α-1,3.

Des isomaltooligosaccharides sont incubés en présence de l'enzyme α-1,3 BrS et de l'enzyme GBD-CD2 qui montre une activité de branchement en α-1,2. Les propriétés prébiotiques et physico-chimiques des glucanes obtenus sont investiguées.

### Exemple 12 : Evaluation des propriétés prébiotiques, nutritionnelles et des effets métaboliques des oligosaccharides branchés en α-1,3

Ces propriétés sont testées pour différents oligosaccharides branchés avec l'enzyme α-1,3 BrS.

### Exemple 13 : Criblage d'une librairie d'accepteur

L'enzyme α-1,3 BrS est mise en présence de son substrat naturel (le saccharose) et d'un panel de différents accepteurs, dont des dextranes branchés en α-1,2 (BRISON *et al.,* 2009), des mutanes, des alternanes, des reuteranes, des fructanes et fructooligosaccharides, des polyphénols, des flavonoïdes, des acides aminés. Les produits de la réaction sont analysés par différentes techniques de chromatographie (spectrométrie de masse, HPAEC-PAD) et par RMN.

Ces expériences ont d'ores et déjà montré que cet enzyme permet la glucosylation d'oligosaccharides tes que les fructooligosaccharides (FOS) et xylooligosaccharides (XOS)

### Exemple 14 : Criblage d'une librairie de donneur

L'enzyme α-1,3 BrS est mise en oeuvre sur différents analogues du saccharose (DAUDE *et al,* 2012) pouvant jouer le rôle de donneur d'unités glucosyle.

## Revendications

1. Un polypeptide isolé ayant la capacité de former uniquement des branchements d'unités glucosyle en alpha-1,3 sur un accepteur comprenant au moins un groupement hydroxyle et **caractérisé en ce que** ledit polypeptide comprend :
i) le motif I de séquence SEQ ID NO: 1
ii) le motif II de séquence SEQ ID NO: 2
iii) le motif III de séquence SEQ ID NO: 3
iv) le motif IV de séquence SEQ ID NO: 4
ou des dérivés d'un ou plusieurs desdits domaines présentant au moins 80% d'identité avec ceux-ci ; lequel polypeptide présente en outre le résidu aspartique (D) en position 5 du motif II (SEQ ID NO: 2), le résidu glutamique (E) en position 6 du motif III (SEQ ID NO: 3) et le résidu aspartique (D) en position 6 du motif IV (SEQ ID NO: 4).

2. Le polypeptide isolé selon la revendication 1, **caractérisé en ce que** ledit polypeptide comprend la séquence SEQ ID NO: 6, un polypeptide dont la séquence présente un pourcentage d'identité d'au moins 80% avec la séquence SEQ ID NO: 6, ou un fragment de celle-ci, ledit fragment présentant une séquence d'au moins 150 acides aminés.

3. Le polypeptide selon l'une quelconque des revendications 1 à 3, **caractérisé en ce que** ledit polypeptide comprend ou consiste en la séquence SEQ ID NO: 9, un polypeptide dont la séquence présente un pourcentage d'identité d'au moins 80% avec la séquence SEQ ID NO: 9, ou un fragment de celle-ci, ledit fragment présentant une séquence d'au moins 150 acides aminés.

4. Le polypeptide selon l'une quelconque des revendications 1 à 3, **caractérisé en ce que** ledit polypeptide est choisi dans le groupe comprenant les séquences SEQ ID NO: 14 et SEQ ID NO: 16.

5. Un polynucléotide isolé codant pour un polypeptide selon l'une quelconque des revendications 1 à 4, de préférence ledit polynucléotide est définie par la séquence SEQ ID NO: 10.

6. Un vecteur d'expression comprenant un polynucléotide tel que défini à la revendication 5.

7. Une cellule hôte transformée comprenant un vecteur d'expression tel que défini à la revendication 6.

8. Une composition comprenant au moins un polypeptide tel que défini à l'une quelconque des revendications 1 à 4, un polynucléotide tel que défini à la revendication 5, un vecteur tel que défini à la revendication 6 ou une cellule hôte transformée telle que définie à la revendication 7.

9. Un procédé de production d'un polypeptide tel que défini à l'une quelconque des revendications 1 à 4, ledit procédé comprenant les étapes de :
a) insertion d'un polynucléotide tel que défini à la revendication 5 ou d'un vecteur tel que défini à la revendication 6 dans une cellule hôte;
b) la mise en culture de ladite cellule obtenue à l'étape a) ; et
c) l'extraction du polypeptide à partir de la culture obtenue à l'étape b).

10. Un procédé de production d'accepteurs branchés d'unités glucosyle en alpha-1,3 comprenant un taux de branchement de telles unités glucosyle en alpha-1,3 compris entre 1 et 50 %, ledit procédé comprenant les étapes de :
i) mélange dans un milieu réactionnel d'un polypeptide tel que défini à l'une quelconque des revendications 1 à 4, d'un substrat dudit polypeptide et d'un accepteur comprenant au moins un groupement hydroxyle; et
ii) incubation dudit mélange obtenu à l'étape i) de sorte d'obtenir le branchement d'unités glucosyle en alpha-1,3 sur ledit accepteur.

11. Le procédé selon la revendication 10, **caractérisé en ce que** le rapport entre la concentration de substrat sur la concentration de l'accepteur est supérieur ou égal à 1 de sorte d'obtenir un taux de branchement compris entre 35 et 50%.

12. Le procédé selon la revendication 10, **caractérisé en ce que** le rapport entre la concentration de substrat sur la concentration de l'accepteur est compris entre 0,5 et 1 de sorte d'obtenir un taux de branchement compris entre 20 et 35%.

13. Le procédé selon la revendication 10, **caractérisé en ce que** le rapport entre la concentration de substrat sur la concentration de l'accepteur est inférieur à 0,5 de sorte d'obtenir un taux de branchement inférieur à 20%.

14. Le procédé selon la revendication 10, **caractérisé en ce que** ledit accepteur branché d'unités glucosyle en alpha-1,3 est sélectionné parmi le groupe comprenant les polysaccharides, de préférence les glucanes, notamment les α-glucanes comme le dextrane, les dextranes ramifiés en α-1,2, les alternanes, les mutanes, les reuteranes, l'amidon, l'amylopectine, l'amylose, le glycogène et le pullulane.

15. Le procédé selon l'une quelconque des revendications 10 ou 14, **caractérisé en ce que** le substrat est choisi dans le groupe comprenant l'a-D-glucopyranosyl fluoride, le p-nitrophenyl α-D-glucopyranoside, le α-D-glucopyranosyl α-L-sorofuranoside le lactulosucrose et le saccharose.

16. Une utilisation d'un polypeptide tel que défini à l'une quelconque des revendications 1 à 4, d'un polynucléotide tel que défini à la revendication 5, d'un vecteur tel que défini à la revendication 6, d'une cellule hôte telle que définie à la revendication 7 et/ou d'une composition telle que définie à la revendication 8 pour la production d'accepteurs branchés d'unités glucosyle en alpha-1,3, de préférence lesdits accepteurs sont branchés d'unité glucosyle en alpha-1,3 à un taux compris entre 1 et 50%, de préférence entre 5 et 40% et de manière encore plus préférée entre 10 et 40%.

## Patentansprüche

1. Isoliertes Polypeptid mit der Fähigkeit, ausschließlich Alpha-1,3-Verknüpfungen von Glykosyl-Einheiten an einem Akzeptor zu bilden, der mindestens eine Hydroxyl-Gruppe aufweist, **dadurch gekennzeichnet, dass** das Polypeptid umfasst:
i) das Sequenzmuster I SEQ ID NO: 1
ii) das Sequenzmuster II SEQ ID NO: 2
iii) das Sequenzmuster III SEQ ID NO: 3
iv) das Sequenzmuster IV SEQ ID NO: 4
oder Derivate einer oder mehrerer der genannten Domänen, die mindestens 80% Identität mit diesen aufweisen, wobei dieses Polypeptid außerdem den Asparaginsäure-Rest (D) in Position 5 des Musters II (SEQ ID NO: 2) aufweist, den Glutaminsäure-Rest (E) in Position 6 des Musters III (SEQ ID NO: 3) und Asparaginsäure-Rest (D) in Position 6 des Musters IV (SEQ ID NO: 4).

2. Isoliertes Polypeptid nach Patentanspruch 1, **dadurch gekennzeichnet, dass** das genannte Polypeptid die Sequenz SEQ ID NO: 6 umfasst, ein Polypeptid, dessen Sequenz mindestens 80% Identität mit der Sequenz SEQ ID NO: 6 aufweist oder ein Fragment von dieser, wobei das genannte Fragment eine Sequenz von mindestens 150 Aminosäuren aufweist.

3. Polypeptid nach irgendeinem der Patentansprüche 1 bis 2, **dadurch gekennzeichnet, dass** das genannte Polypeptid die Sequenz SEQ ID NO: 9 umfasst oder daraus besteht, ein Polypeptid, dessen Sequenz einen Identitätsanteil von mindestens 80% mit der Sequenz SEQ ID NO: 9 aufweist, oder ein Fragment von dieser, wobei das genannte Fragment eine Sequenz von mindestens 150 Aminosäuren aufweist.

4. Polypeptid nach irgendeinem der Patentansprüche 1 bis 3, **dadurch gekennzeichnet, dass** das genannte Polypeptid aus der Gruppe gewählt wurde, bestehend aus den Sequenzen SEQ ID NO: 14 und SEQ ID NO: 16.

5. Isoliertes Polynukleotid, das ein Polypeptid nach irgendeinem der Patentansprüche 1 bis 4 codiert, wobei das genannte Polynukleotid vorzugsweise durch die Sequenz SEQ ID NO: 10 definiert ist.

6. Expressionsvektor, ein Polypeptid nach Patentanspruch 5 enthaltend.

7. Transformierte Wirtszelle, einen Expressionsvektor nach Patentanspruch 6 enthaltend.

8. Gemisch, mindestens ein Polypeptid nach irgendeinem der Patentansprüche 1 bis 4 enthaltend, ein Polynukleotid nach Patentanspruch 5, einen Vektor nach Patentanspruch 6 oder eine transformierte Wirtszelle nach Patentanspruch 7.

9. Verfahren zur Herstellung eines Polypeptids nach irgendeinem der Patentansprüche 1 bis 4, wobei das genannte Verfahren die folgenden Schritte umfasst:
a) Inserieren eines Polynukleotids nach Patentanspruch 5 oder eines Vektors nach Patentanspruch 6 in eine Wirtszelle
b) Kultivierung der genannten, im Schritt a) erhaltenen Zelle, und
c) Extraktion des Polypeptids aus der im Schritt b) erhaltenen Kultur.

10. Verfahren zur Herstellung von mit Glykosyl-Einheiten Alpha-1,3-verknüpften Akzeptoren, die einen Anteil an Alpha-1,3-Verknüpfungen derartiger Glykosyl-Einheiten zwischen 1 und 50% enthalten, wobei das genannte Verfahren die folgenden Schritte umfasst:
i) Mischung eines Polypeptids nach irgendeinem der Patentansprüche 1 bis 4, eines Substrates des genannten Polypeptids und eines Akzeptors, der mindestens eine Hydroxylgruppe aufweist, in einem Reaktionsmilieu und
ii) Inkubation des genannten, im Schritt i) erhaltenen Gemisches, derart, dass die Alpha-1,3-Verknüpfung von Glykosyl-Einheiten am genannten Akzeptor erhalten wird.

11. Verfahren nach Patentanspruch 10, **dadurch gekennzeichnet, dass** das Verhältnis der Konzentration des Substrates zur Konzentration des Akzeptors größer oder gleich 1 ist, derart, dass ein Verknüpfungsanteil zwischen 35 und 50% erzielt wird.

12. Verfahren nach Patentanspruch 10, **dadurch gekennzeichnet, dass** das Verhältnis der Konzentration des Substrates zur Konzentration des Akzeptors zwischen 0,5 und 1 beträgt, derart, dass ein Verknüpfungsanteil zwischen 20 und 35% erzielt wird.

13. Verfahren nach Patentanspruch 10, **dadurch gekennzeichnet, dass** das Verhältnis der Konzentration des Substrates zur Konzentration des Akzeptors kleiner als 0,5 ist, derart, dass ein Verknüpfungsanteil unter 20% erzielt wird.

14. Verfahren nach Patentanspruch 10, **dadurch gekennzeichnet, dass** der genannte, mit Glykosyl-Einheiten Alpha-1,3-verknüpfte Akzeptor aus der Gruppe gewählt wurde, die die Polysaccharide, vorzugsweise Glucane, insbesondere α-Glucane, wie etwa Dextran, α-1,2-verzweigte Dextrane, Alternane, Mutane, Reuterane, Stärke, Amylopektin, Amylose, Glykogen und Pullulan umfasst.

15. Verfahren nach irgendeinem der Patentansprüche 10 oder 14, **dadurch gekennzeichnet, dass** das Substrat aus der Gruppe gewählt wurde, die α-D-Glucopyranosylfluorid, p-Nitrophenyl-α-D-Glucopyranosid, α-D-Glucopyranosyl-α-L-sorofuranosid, Lactulosaccharose und Saccharose umfasst.

16. Gebrauch eines Polypeptids nach irgendeinem der Patentansprüche 1 bis 4, eines Polynukleotids nach Patentanspruch 5, eines Vektors nach Patentanspruch 6 oder einer transformierten Wirtszelle nach Patentanspruch 7 und/oder eines Gemisches nach Patentanspruch 8 zur Herstellung von mit Glykosyl-Einheiten Alpha-1,3-verknüpften Akzeptoren, wobei die genannten Akzeptoren vorzugsweise einen Anteil an Alpha-1,3-Verknüpfungen von Glykosyl-Einheiten zwischen 1 und 50% enthalten, vorzugsweise zwischen 5 und 40% und noch stärker bevorzugt zwischen 10 und 40%.

## Claims

1. An isolated polypeptide having the ability to specifically form connections of glucosyl units in alpha-1,3 on an acceptor comprising at least one hydroxyl group and **characterized in that** said polypeptide comprises :
i) pattern I of sequence SEQ ID NO: 1
ii) pattern II of sequence SEQ ID NO: 2
iii) pattern III of sequence SEQ ID NO: 3
iv) pattern IV of sequence SEQ ID NO: 4
or derivatives of one or more of said domains having at least 80% identity therewith; which polypeptide further has the aspartic residue (D) in position 5 of pattern II (SEQ ID NO: 2), the glutamic residue (E) in position 6 of pattern III (SEQ ID NO: 3) and the aspartic residue (D) in position 6 of pattern IV (SEQ ID NO: 4).

2. The polypeptide isolated according to claim 1, **characterized in that** said polypeptide comprises the sequence SEQ ID NO: 6, a polypeptide whose sequence has an identity percentage of at least 80% with the sequence SEQ ID NO: 6, or a fragment thereof, said fragment having a sequence of at least 150 amino acids.

3. The polypeptide according to any of claims 1 to 2, **characterized in that** said polypeptide comprises or consists of the sequence SEQ ID NO: 9, a polypeptide whose sequence has an identity percentage of at least 80% with the sequence SEQ ID NO: 9, or a fragment thereof, said fragment having a sequence of at least 150 amino acids.

4. The polypeptide according to any of claims 1 to 3, **characterized in that** said polypeptide is selected from the group comprising the sequences SEQ ID NO: 14 and SEQ ID NO: 16.

5. An isolated polynucleotide encoding a polypeptide according to any of claims 1 to 4, preferably said polynucleotide is defined by the sequence SEQ ID NO: 10.

6. An expression vector comprising a polynucleotide as defined in claim 5.

7. A transformed host cell comprising an expression vector as defined in claim 6.

8. A composition comprising at least one polypeptide as defined in any of claims 1 to 4, a polynucleotide as defined in Claim 5, a vector as defined in Claim 6 or a transformed host cell as defined in Claim 7.

9. A process for producing a polypeptide as defined in any of claims 1 to 4, said process comprising the steps of:
a) inserting a polynucleotide as defined in claim 5 or a vector as defined in claim 6 into a host cell;
b) culturing the said cell obtained in step a); and
(c) extracting the polypeptide from the culture obtained in step (b).

10. A method for producing acceptors connected to glycosyl units in alpha-1,3 comprising a connection rate of said glucosyl units in alpha-1,3 between 1 and 50%, said method comprising the steps of:
i) mixing in a reaction medium a polypeptide as defined in any of claims 1 to 4, a substrate of said polypeptide and an acceptor comprising at least one hydroxyl moiety; and
ii) incubating said mixture obtained in step i) so as to obtain the connection of glucosyl units in alpha-1,3 to said acceptor.

11. The process according to claim 10, **characterized in that** the ratio between the substrate concentration and the acceptor concentration is greater than or equal to 1 so as to obtain a connection rate between 35 and 50%.

12. The process according to claim 10, **characterized in that** the ratio between the substrate concentration and the acceptor concentration is between 0.5 and 1 so as to obtain a connection rate between 20 and 35%.

13. The process according to claim 10, **characterized in that** the ratio between the substrate concentration and the acceptor concentration is less than 0.5 so as to obtain a connection rate of less than 20%.

14. The process according to claim 10, **characterized in that** said acceptor connected to glucosyl units in alpha-1,3 is selected from the group comprising polysaccharides, preferably glucans, including α-glucans such as dextran, dextrans branched to α-1,2, alternanes, mutans, reuteranes, starch, amylopectin, amylose, glycogen and pullulan.

15. The process according to any of claims 10 or 14, **characterized in that** the substrate is selected from the group comprising α-D-glucopyranosyl fluoride, p-nitrophenyl α-D-glucopyranoside, α-D-glucopyranosyl, α-L-sorofuranoside, lactulosucrose and sucrose.

16. A use of a polypeptide as defined in any of claims 1 to 4, a polynucleotide as defined in claim 5, a vector as defined in claim 6, of a host cell as defined in claim 7 and/or a composition as defined in claim 8 for the production of acceptors connected to glucosyl units in alpha-1,3, preferably said acceptors are connected to glucosyl units in alpha-1,3 at a connection rate between 1 and 50%, preferably between 5 and 40% and even more preferably between 10 and 40%.
